(19) Europäisches Patentamt / European Patent Office / Office européen des brevets

(11) **EP 4 507 612 B1**

(12) **EUROPEAN PATENT SPECIFICATION**

(45) Date of publication and mention of the grant of the patent:
**17.06.2026 Bulletin 2026/25**

(21) Application number: **23719347.9**

(22) Date of filing: **11.04.2023**

(51) International Patent Classification (IPC):
*A61B 34/00* (2016.01)   *A61B 34/30* (2016.01)
*A61F 2/915* (2013.01)   *A61B 90/00* (2016.01)

(52) Cooperative Patent Classification (CPC):
**A61B 34/73;** A61B 34/32; A61B 34/35; A61B 34/72;
A61B 2017/00871; A61B 2034/102; A61B 2034/303;
A61B 2090/376; A61B 2090/378; A61F 2/915;
A61F 2002/91575; A61M 25/0116

(86) International application number:
**PCT/EP2023/059438**

(87) International publication number:
**WO 2023/198706 (19.10.2023 Gazette 2023/42)**

(54) **FOLDABLE STRUCTURE**

FALTBARE STRUKTUR

STRUCTURE PLIABLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **11.04.2022 LU 501826**

(43) Date of publication of application:
**19.02.2025 Bulletin 2025/08**

(73) Proprietor: **Max-Planck-Gesellschaft zur Förderung der Wissenschaften e.V.**
**80539 München (DE)**

(72) Inventors:
• **WANG, Tianlu**
  **80539 München (DE)**
• **SITTI, Metin**
  **80539 München (DE)**
• **HU, Wenqi**
  **80539 München (DE)**

(74) Representative: **Richardt Patentanwälte PartG mbB**
**Wilhelmstraße 7**
**65185 Wiesbaden (DE)**

(56) References cited:
EP-A1- 1 591 057    WO-A2-2008/126087
CN-A- 108 553 300    JP-A- 2005 052 502
US-A1- 2007 083 258    US-A1- 2012 035 440
US-A1- 2020 046 633

• IKEUCHI K ET AL: "Locomotion of medical micro robot with spiral ribs using mucus", MICRO MACHINE AND HUMAN SCIENCE, 1996., PROCEEDINGS OF THE SEVENTH INTERNATIONAL SYMPOSIUM NAGOYA, JAPAN 2-4 OCT. 1996, NEW YORK, NY, USA,IEEE, US, 2 October 1996 (1996-10-02), pages 217 - 222, XP010208880, ISBN: 978-0-7803-3596-7, DOI: 10.1109/MHS.1996.563427
• BHARGAVA AARUSHI ET AL: "Ultrasound actuated shape-memory polymer based drug delivery containers", PROCEEDINGS OF SPIE; [PROCEEDINGS OF SPIE ISSN 0277-786X VOLUME 10524], SPIE, US, vol. 10595, 15 March 2018 (2018-03-15), pages 105952H - 105952H, XP060104124, ISBN: 978-1-5106-1533-5, DOI: 10.1117/12.2296739
• BRIAN P. TIMKO ET AL: "Remotely Triggerable Drug Delivery Systems", ADVANCED MATERIALS, vol. 22, no. 44, 3 September 2010 (2010-09-03), pages 4925 - 4943, XP055163288, ISSN: 0935-9648, DOI: 10.1002/adma.201002072

- YUANFENG HAN ET AL: "Fiberbot: A miniature crawling robot using a directional fibrillar pad", 2015 IEEE INTERNATIONAL CONFERENCE ON ROBOTICS AND AUTOMATION (ICRA), IEEE, 26 May 2015 (2015-05-26), pages 3122 - 3127, XP033168842, DOI: 10.1109/ICRA.2015.7139628
- MCGIRR M E A ET AL: "The use of the InteliSite^ (R) Companion device to deliver mucoadhesive polymers to the dog colon", EUROPEAN JOURNAL OF PHARMACEUTICAL SCIENCES, ELSEVIER AMSTERDAM, NL, vol. 36, no. 4-5, 2 March 2009 (2009-03-02), pages 386 - 391, XP025914676, ISSN: 0928-0987, [retrieved on 20081121], DOI: 10.1016/J.EJPS.2008.11.007
- RICHERT H ET AL: "Development of a magnetic capsule as a drug release system for future applications in the human GI tract", JOURNAL OF MAGNETISM AND MAGNETIC MATERIALS, ELSEVIER, AMSTERDAM, NL, vol. 293, no. 1, 1 May 2005 (2005-05-01), pages 497 - 500, XP027843066, ISSN: 0304-8853, [retrieved on 20050501]

**Description**

The invention relates to the field of small-scale robotic devices. More particularly, the invention relates to a robotic device for insertion into a blood vessel of a vascular system with a foldable structure, an actuation and control device configured for actuating and controlling the foldable structure and a system comprising the robotic device and the actuation and control device.

Multiple approaches for administration of drugs are known, like, e.g., injections, infusions, tablets, etc. However, these approaches have in common that the administration is rather unfocused. Such a systemic administrations of drugs may have the drawback that they may lead to complications and/or to a reduced effectiveness due to the lacking focus. Thus, there is a need for effective medical tools usable for an improved delivery of drugs.

The article "Ultrasound actuated shape-memory polymer based drug delivery containers" by Aarushi Bhargava et al. in PROCEEDINGS OF SPIE, vol. 10595, 2018, describes employing biodegradable shape memory polymer (SMP) based drug-delivery containers. Focused ultrasound (FU) is used as a trigger for noninvasively stimulating SMP-based drug capsules. A mathematical Multiphysics model is used which performs an acoustic-thermoelastic analysis, to optimize the design of SMP containers. The proposed designs exploit various parameters such as nonlinearity, absorption and diffraction effects, as well as input power and frequency of the propagating acoustic wave to attain the desired shape recovery, as required by the application or location of drug release. The acoustic-thermoelastic effects on the SMP containers are studied with the help of finite-element methods. Multilayer simulations are performed at millimeter scale to mimic the in vivo conditions of a drug delivery container travelling inside an artery. By manipulating the design and the shape recovery rate of the SMP containers, velocity of the drug particles is controlled and directed towards a specific location.

CN 108 553 300 A describes a drug releasing method based on a 4D printing shape memory polymer structure. Shape memory polymers and a drug are mixed at the indoor temperature, a shape memory polymer carrying the drug is obtained, an initial constructed drug releasing structure is prepared through a 4D printing technology, the structure is heated to the glass transition temperature, the initial constructed drug releasing structure is converted to a temporary constructed drug releasing structure with a smaller superficial area, the temporary constructed drug releasing structure is placed in the environment which is prepared for simulating human body fluid, external motivators are given, the initial constructed drug releasing structure is recovered, and the whole process is completed.

US 2020/046633 A1 describes a system and a method to control a dispensing of swallow able medicine at a desired flow rate and in a desired location of the gastro-intestinal tract.

EP 1 591 057 A1 describes a capsule medical apparatus inserted in a body cavity including a spiral projected portion on the outer peripheral surface of a body cavity inserting portion. Pitch, height, and cross section of the projected portion and the like are set to have proper values and shapes suitable to the advance thereof. The body cavity inserting portion has a magnet. An external magnetic guiding device applies a rotating magnetic field to the magnet and the magnetic torque acts on a magnet for rotation. Thus, the medical apparatus stably advances.

The article "Remotely Triggerable Drug Delivery Systems" by Brian P. Timko et al. in ADVANCED MATERIALS, vol. 22, no. 44, 2010, provides a review of triggerable materials that range in scale from nano to macro, and are activated by a range of stimuli.

US2012/035440 refers to a further prior art document relevant for the present invention.

The present invention is defined by appended claim 1. Embodiments of the invention are disclosed in the dependent claims. Methods are not claimed.

It is an objective to provide for a robotic device for insertion into a blood vessel of a vascular system with a foldable structure, an actuation and control device configured for actuating and controlling the foldable structure, and a system comprising the robotic device and the actuation and control device.

The invention relates to a robotic device for insertion into a blood vessel of a vascular system with a base structure and a foldable structure. The foldable structure comprises a closed retaining section configured for retaining an agent for medical use. The foldable structure further is configured for opening the closed retaining section and releasing the agent upon reaching a predefined internal energy level.

The robotic device with the foldable structure enables a local on-demand delivery of the agent for medical use within the vascular system. The foldable structure is used to transport the agent for medical use to a target destination within the vascular system. Upon reaching the target destination by the robotic device, a release of the agent may be triggered by purposefully increasing the internal energy of the foldable structure up to the predefined internal energy level. Upon reaching a predefined internal energy level, the closed retaining section locally releases the agent. Thus, the agent may unfold its effect locally at a target destination, where it is required.

A local on-demand delivery of the agent may have the beneficial effect that complications resulting from a systemic administration may be avoided. Furthermore, an effect of the agent may be precisely controllable based on time and location of the administration. In addition, a local concentration of the agent due to the administration may be precisely controllable. The local delivery using the robotic device may reduce the risk of an inhibition of the effect of the agent due to bio-chemical interactions of the agent on its way to its target destination, e.g., with inhibitors. Finally, a local delivery may also reduce the risk for the agent to be cleared from the body, e.g., via absorption by the liver, before reaching its target destination.

For example, the foldable structure may be a foldable shape-memory structure, e.g., made from a shape-memory material, like a shape-memory polymer. The foldable shape-memory structure provides a closed retaining section configured for retaining an agent for medical use. The foldable shape-memory structure further is configured for opening the closed retaining section and releasing the agent upon reaching a shape-memory transition temperature.

For example, the foldable structure may be made from a photo-responsive material. A photo-responsive material is a material with the ability to alter its physical properties, e.g., its shape, in response to external photonic stimuli. For example, the internal energy of the foldable structure is increased using photons.

The foldable structure is made from one of the following materials: a photo-responsive material, an electro-responsive material, a magneto-responsive material, an ultrasound-responsive material. An electro-responsive material is a material with the ability to alter its physical properties, e.g., its shape, in response to external electric stimuli. For example, the internal energy of the foldable structure is increased using an electric field and/or current. A magneto-responsive material is a material with the ability to alter its physical properties, e.g., its shape, in response to external magnetic stimuli. For example, the internal energy of the foldable structure is increased using a magnetic field.

An ultrasound-responsive material is a material with the ability to alter its physical properties, e.g., its shape, in response to external ultrasound stimuli. For example, the internal energy of the foldable structure is increased using ultrasound.

The robotic device with the foldable structure retaining the agent for medical use within the retaining section propagates through the blood vessel of the vascular.

When the target destination is reached, the closed retaining section may be opened releasing the agent. The opening may, e.g., be activated using an external stimulus, e.g., a photo-, electro-, magnetic- and/or ultrasound-stimulus. The opening may be activated upon reaching a predefined internal energy level by increasing the internal energy of the foldable structure to the predefined internal energy level. For example, a temperature of the foldable structure made from a shape-memory material is raised to a predefined shape-memory transition temperature. The internal energy may, e.g., be increased using a photo-, electro-, magnetic- and/or ultrasound-source. The internal energy may, e.g., be raised using external radiofrequency induced heating of the foldable structure.

The foldable structure may have any shape of a container, such as a cubic container, a box-shaped container, a flower-shaped container, or a pouch-shaped container. In case of a box-shaped container, an exemplary size of the container may, e.g., be 450 $\mu$m * 300 $\mu$m * 120 $\mu$m. The foldable structure may be configured to deliver any type of agent for medical use, e.g., medicine, RNA, stem cells etc.

For example, the foldable structure is made from a shape-memory material, e.g., a shape-memory polymer, configured for opening the closed retaining section and releasing the agent upon reaching a predefined shape-memory transition temperature. Examples may have the beneficial effect, that the shape-memory material may enable a transition of the shape-memory structure from a first shape, in which the retaining section is closed, to a second shape, in which the retaining section is open, upon reaching a shape-memory transition temperature.

Shape-memory polymers (SMPs) are polymeric smart materials that have the ability to return from a temporary shape to their original permanent shape when induced by an external stimulus, i.e., trigger, such as temperature change. For example, the permanent shape of the retaining section at room temperature may be the closed shape, while the temporary shape of the retaining section at a higher temperature may be the open shape. An SMP may, e.g., retain two shapes with the transition between those being induced by temperature.

For example, the closed retaining section forms a sealed space. The agent to be delivered may thus be retained in the sealed space until energy-controlled release is triggered, e.g., using radiofrequency induced heating. Examples may have the beneficial effect, that while the agent is contained within the retaining section, it may be prevented from interactions with components of the blood.

For example, the foldable structure further comprises metal particles for enhancing an efficiency of an external energy input into the foldable structure. The external energy input may, e.g., be an external radiofrequency induced heating of the foldable structure. Examples may have the beneficial effect, that the metal particles may enhancing the efficiency of the external energy input, e. g., a radiofrequency induced heating of the foldable structure. For example, a radiofrequency coil may be used to heat the metal particles comprised by the foldable structure resulting in a heating of the foldable structure itself and a transition from a first closed shape of the retaining section to a second open shape of the retaining section. For example, the foldable structure has a size in the submillimeter range. Examples may have the beneficial effect, that a small-scale robotic device with the foldable structure may be provided, which enables an on-demand delivery of the agent even into small blood vessels of distal vascular regions, like, e.g., the M4 segment of the middle cerebral artery.

The base structure is a hollow cylindrical elastic base structure. Thus, the robotics device may be a tube-shaped robotic device. Examples may have the beneficial effect, that a blood flow through the robotic device may be enabled.

Furthermore, the elasticity of the base structure enables an adaption of a diameter of the robotic device to different diameters of the blood vessel, e.g., for upholding a contact with an inner surface of the blood vessel for a surface propulsion. Furthermore, the elastic base structure may enable a bending of the robotic device around curvatures of the blood vessel and/or into branches branching form the blood vessel.

For example, the foldable structure is configured to open the closed retaining section in radial direction towards a central longitudinal body axis of the robotic device. Examples may have the beneficial effect, that for a hollow cylindrical base structure an opening towards the central longitudinal body axis may be avoided that the opening is blocked, e.g., by the wall of the blood vessel.

For example, a catheter, e.g., a micro-catheter, may be used for inserting the robotic device into the vascular system and positing the robotic device in a target section of the blood vessel. When the catheter, e.g., a micro-catheter, has reached the target section, the robotic device may be released and advance forward on its own independently of the catheter. For this purpose, the robotic device moves its own, using a magnetically induced surface propulsion.

The elastic base structure is configured for providing a radial elastic force configured for establishing a contact between an outer anisotropic surface structure of the robotic device and an inner surface of the blood vessel. The anisotropic surface structure comprises a geometrical anisotropy between a direction circumferential to the robotic device and a direction longitudinal to the robotic device. The anisotropic surface structure is configured for establishing by the contact an anisotropic friction between the anisotropic surface structure and the inner surface of the blood vessel. The anisotropic friction results in a surface propulsion of the robotic device when being rotated around the central longitudinal axis of the robotic device. The robotic device further comprising a magnetic material distributed circumferentially around the robotic device and configured for establishing within an external magnetic field a rotation of the robotic device around the central longitudinal body axis of the robotic device.

The anisotropic surface structure in combination with the magnetic material provides a propulsion system for the robotic device based on a surface propulsion. This propulsion system enables the robotic device to actively move through the blood vessel of the vascular system. The robotic device may be usable as a robotic device, which may move after insertion into the blood vessel through the same. For example, the robotic device may move through the vessel to a target destination within the vascular system. The robotic device may, e.g., be enabled to move downstream as well as upstream regarding the direction of flow of blood through the vessel.

The term anisotropic refers to a geometric anisotropy. The anisotropic surface structure of the robotic device is therefore a surface structure, which comprises a geometrical anisotropy. In case of the anisotropic surface structure, the geometrical anisotropy of the anisotropic surface structure is a geometric between the direction circumferential to the robotic device and the direction longitudinal to the robotic device.

When an external magnetic field is applied, the robotic device is rotated around its central longitudinal body axis due the magnetic forces caused by the external magnetic field and acting on the magnetic material distributed circumferentially around the robotic device. The rotation of the robotic device causes an anisotropic friction due to the contact between the anisotropic surface structure of the robotic device with the geometrical anisotropy and the inner surface of the blood vessel. This anisotropic friction results in a surface propulsion of the robotic device. Since the geometrical anisotropy is an anisotropy between the direction circumferential to the robotic device and the direction longitudinal to the robotic device, the resulting friction is anisotropic with respect to the direction circumferential to the robotic device and the direction longitudinal to the robotic device. The friction in the direction circumferential to the robotic device may be larger than the friction in the direction longitudinal to the robotic device. The difference in friction in the two directions may result in a propulsion in the direction longitudinal to the robotic device, when the robotic device is rotated. Depending on the direction of rotation, the robotic device may, e.g., move forward or backward through the blood vessel.

A magnet generating the external magnetic field may be moved along the blood vessel magnetically forcing movement of the robotic device through the blood vessel. In case the magnet is moved in front of the robotic device along the blood vessel, an additional magnetic dragging force may act on the robotic device dragging the robotic device behind the magnet along the blood vessel. This magnetic dragging force may contribute to the propulsion of the robotic device, e.g., in addition to the surface propulsion due to the magnetically induced rotation of the robotic device.

Furthermore, the contact between the anisotropic surface structure of the robotic device and the inner surface of the blood vessel due to the radial elastic force of the robotic device may, e.g., further result in an anchoring of the robotic device at its current destination within the blood vessel, when the rotation is turned off. The rotation is turned on by applying an external magnetic field configured for rotating the robotic device. For example, a rotating external magnetic field may be applied. When no external magnetic field configured for rotating the robotic device is applied, the rotation of the robotic device is turned off. Thus, the propulsion system for the robotic device may not only implement a propulsion mechanism for the robotic device, but at the same time also an anchoring mechanism for anchoring the robotic device in place within the blood vessel. The anchoring ability of the robotic device may, e.g., ensure that the robotic device holds its position at the target destination, while releasing the agent from the foldable structure. Thus, the focusing of the administration of the agent may be improved.

Examples may have the beneficial effect that a robotic device may be provided, which is configured to be deployed, actively navigated, used for medical functions, and retrieved, e.g., in M4 segment of the middle cerebral artery. The efficiency of the shape-adaptively controlled locomotion of the robotic device is illustrated in phantoms emulating the physiological conditions. For example, the robotic device may be configured to adapt to lumen diameter shrinking, e.g., from 1.5 mm to 1 mm, to radius of curvature of a tortuous lumen, e.g., getting as small as 3 mm, to a lumen bifurcation angle, e.g., going up to 120°, and to a pulsatile flow speed, e.g., reaching up to 26 cm/s. The robotic device may, e.g., withstand the flow when the magnetic actuation is turned off. These locomotion capabilities are confirmed in porcine arteries ex vivo. Furthermore, the robotic device may release an agent for medical use on-demand locally, e.g., a tissue plasminogen activator for thrombolysis. These features may, e.g., be used for therapies towards acute ischemic stroke, aneurysm, arteriovenous malformation, dural arteriovenous fistulas, and brain tumors. Furthermore. these functions may, e.g., facilitate the robotic device's usage in new distal endovascular operations.

Using a robotic device configured for moving through a blood vessel may allow to avoid disadvantageous from using a catheter. A longer access route from the arterial puncture sites, e.g., radial artery or femoral artery, the vessel tortuosity, and the smaller vessels with thinner vessel walls may increase challenges to safely navigate and push a catheter into regions, like the M4 segment of the middle cerebral artery, without causing injuries, by either manual or robotic insertion. Furthermore, some vascular lesions, such as thrombotic diseases and arteriosclerosis, decrease or even block the blood flow in arteries, making it impractical to utilize the flow to advance the catheter to the target position.

For example, distally cortical arteries are still limited to safely and effectively access by catheters to treat various diseases and lesions around this region, such as acute ischemic stroke (AIS), aneurysm, cerebral arteriovenous malformation (CAVM), dural arteriovenous fistulas (dAVFs), and brain tumors. Due to the challenge in safe reachability to the proximity of targeted lesions by catheterization, the therapeutic elements/agents, such as stents, coils, and medications, may not be delivered using a catheter accurately and efficiently. Thus, the efficacy of therapies using a catheter may be decreased. Meanwhile, the distal lesions could also be devastating or fatal for patients. For example, the hemispheric distal occlusions commonly lead to partial aphasia, fractionated hemiparesis or hemianesthesia, as well as partial or complete hemivisual field defects. Moreover, the most malignant primary brain tumors occur in the cerebral cortex. Similar limitations also apply to other distal vascular routes. Therefore, there are unmet needs for more effective medical tools for minimally invasive therapies in these distal arterial regions. Such an effective medical tool enabling minimally invasive therapies even in distal arterial regions may, e.g., be provided by a robotic device as disclosed herein.

Burgeoning efforts have been made to develop wireless medical devices at the milli/microscale, which could enter these hard-to-reach sites because of their tetherless nature and smaller dimensions. In tortuous distal vascular regions, these devices may be required to achieve shape adaption for changing lumen diameters, withstand the flow even if the external actuation is disrupted or turned off (i.e., self-anchoring capability) for safe local operations, traverse among curved routes and bifurcations, and be movable against the blood flow for retrieval or mistaken navigation. However, all the previous works have shown limitations in concurrently fulfilling all of these requirements. Notably, most of the previous designs lack the self-anchoring capability with the surrounding lumen and could be drifted away by the pulsatile flow. The distribution of such drifting devices may be unpredictable and may accumulate in non-target vessels and organs, which might lead to prolonged health risks.

The robotic device as described herein may be able to achieve all of the above requirements. It may in particular be able to operate in the distal M4 segment of MCA. The robotic device may achieve retrievably adaptive locomotion, e.g., with the lumen diameter of blood vessels down to 1 mm, small lumen radius of curvatures down to 3 mm, branch bifurcation angles up to 120°, and pulsatile blood flow speeds up to 26 cm/s at 80 beats per minute (bpm). Moreover, the robotic device may provide a safe self-anchoring capability configured to withstand the flow of blood even when the external magnetic actuation input is turned off.

A retrievable adaptive locomotion may, e.g., be achieved with the lumen diameter of blood vessels even down to 0.3 mm, e.g., from 1.5 mm to 0.3 mm. Also a self-anchoring capability may, e.g., be maintained with the lumen diameter of blood vessels down to 0.3 mm, e.g., from 0.7 mm to 0.3 mm.

In addition to controlled and precise navigation, it is demonstrated that variants of the robotic device may be able to provide several essential medical functions. For example, the robotic device may be configured for a delivery of tissue plasminogen activator (tPA) on-demand to achieve thrombolysis at a target location. These functions may enable new minimally invasive targeted therapies for AIS, aneurysm, CAVM, dAVFs, and brain tumors in the distal and tortuous vascular regions.

For example, the elastic base structure is configured for passively adapting a diameter of the robotic device to altering diameters of the blood vessel for diameters of the blood vessel altering within a predefined range of diameters. Examples may have the beneficial effect, that the robotic device is enabled to adapt to altering diameters of the blood vessel, when moving through the respective blood vessel. Due to the adaption, e.g., the contact between the between the outer anisotropic surface structure of the robotic device and the inner surface of the blood vessel may be upheld even for changing diameters of the blood vessel. By upholding the contact, the surface propulsion may be ensured even for sections of a blood vessel with changing diameters. Thus, the robotic device may be enabled to advance into sections of a blood vessel, in which the diameter of the blood vessel is decreasing, as well as into sections of the blood vessel, in which the diameter of the blood vessel is increasing.

The robotic device is configured for providing the radial elastic force configured for establishing the contact between the outer anisotropic surface structure of the robotic device and the inner surface of the blood vessel for the diameters of the blood vessel within the pre-defined range of diameters.

For example, the anisotropic surface structure comprises one or more protrusions added onto the elastic base structure. Examples may have the beneficial effect, that the protrusions may come into contact with inner surface of the blood vessel due to the radial elastic force of the robotic device. Thus, the friction between the protrusions and the inner surface of the blood vessel may be larger than the friction between other sections of the outer surface of the robotic device and the inner surface of the blood vessel. The friction between the protrusions and the inner surface of the blood vessel may in particular be larger in comparison to those sections of the outer surface of the robotic device not in contact with the inner surface of the blood vessel at all. With the protrusions being, e.g., distributed anisotropically, the friction caused by the protrusions may be anisotropic.

For example, the anisotropic surface structure comprises one or more recesses inserted into the elastic base structure. Examples may have the beneficial effect, that the recesses may not come into contact with inner surface of the blood vessel due to the radial elastic force of the robotic device. Thus, no friction may occur between the recesses and the inner surface of the blood vessel, while there may occur friction between other sections of the outer surface of the robotic device and the inner surface of the blood vessel. With the recesses being, e.g., distributed anisotropically, the friction caused by the other sections of the outer surface of the robotic device in contact with the inner surface of the blood vessel may be anisotropic.

For example, the anisotropic surface structure may comprise one or more protrusions added onto the elastic base structure as well as one or more recesses inserted into the elastic base structure. For example, the one or more protrusions as well as the one or more recesses may be distributed anisotropically.

For example, the magnetic material is comprised by the elastic base structure. Examples may have the beneficial effect, that the magnetic material may be integrated into the elastic base structure. The magnetic material may be integrated in form of magnetic particles distributed, e.g., evenly distrusted, within the elastic base structure. The external magnetic field may cause a rotation of the magnetic material around the central longitudinal body axis of the robotic device, thereby rotating the robotic device around the central longitudinal body axis.

For example, the magnetic material is distributed on an outer surface of the elastic base structure. For example, the magnetic material is distributed on an inner surface of the elastic base structure. The magnetic material distributed on the outer and/or inner surface of the hollow cylindrical elastic base structure of the robotic device may be distributed circumferentially around the central longitudinal body axis of the robotic device. When the magnetic material is rotated around the central longitudinal body axis of the robotic device by the external magnetic field, the robotic device may be rotated around the central longitudinal body axis.

The magnetic material may, e.g., be distributed on the outer and/or inner surface of the elastic base structure in form of a coating film comprising the magnetic material. The magnetic material may, e.g., be distributed on the outer and/or inner surface of the elastic base structure in form of magnetic particles. The magnetic particles may, e.g., be deposited on the outer and/or inner surface of the elastic base structure.

For example, the magnetic material is comprised by the anisotropic surface structure. Examples may have the beneficial effect, that the magnetic material may be integrated into the anisotropic surface structure. The magnetic material may be integrated in form of magnetic particles distributed, e.g., evenly distrusted, within the anisotropic surface structure. The external magnetic field may cause a rotation of the magnetic material around the central longitudinal body axis of the robotic device, thereby rotating the anisotropic surface structure and with the anisotropic surface structure the robotic device around the central longitudinal body axis.

For example, the anisotropic surface structure comprises one or more helical structures extending in the longitudinal direction around the elastic base structure. Examples may have the beneficial effect, that by the one or more helical structures a geometrical anisotropy between the direction circumferential to the robotic device and the direction longitudinal to the robotic device may be unimplemented. The one or more helical structures may, e.g., be right-handed helical structures. The one or more helical structures may, e.g., be left-handed helical structures.

For example, the robotic device further comprises a continuous inner layer configured for channeling a blood flow through the robotic device. Examples may have the beneficial effect, that the robotic device may be used for a flow diversion by channeling the blood flow through the robotic device blocking a lesion of the blood vessel, when being arranged at a lesion site within the blood vessel. For example, a porosity of the continuous inner layer is less than 70%. For example, the porosity of the continuous inner layer is preferably less than 50%. For example, the porosity of the continuous inner layer is more preferably less than 1%, e.g., equal 0%.

For example, the foldable structure is arranged on an inner surface of the hollow cylindrical elastic base structure. For example, the foldable structure is integrated into the base structure. For example, the foldable structure is arranged on an external surface of the base structure.

For example, the base structure comprises a mesh structure. The mesh structure may comprise plurality of cells.

Examples may have the beneficial effect, that using a mesh structure rather than a solid base structure may increase the elasticity of the base structure. In particular, a radial elastic force configured for establishing the contact between the outer anisotropic surface structure of the robotic device and the inner surface of the blood vessel may be implemented.

For example, the mesh structure is formed by a plurality of identical cells. Using identical cells may have the beneficial effect that elastic properties of the base structure may be distributed evenly, e.g., rotational symmetric around the central longitudinal body axis. In particular, the resulting radial elastic force may be rotational symmetric with respect to the central longitudinal body axis.

For example, the cells are diamond-shaped cells. The diamond shape refers to a rhombic shape. The diamond-shaped cells may be provided by rhombi, i.e., equilateral quadrilaterals. For example, the cells may have a hexagonal shape. The cells may, e.g., be provided by regular hexagons flattened or stretched along one symmetry direction. The symmetry direction may be parallel to the central longitudinal body axis. These regular hexagons stretched along the symmetry direction may be seen as elongated rhombi.

For example, a porosity of the mesh structure defined by a ratio between an accumulated area of voids of the cells of the mesh structure within a section of the mesh structure and a total area the respective section of mesh structure is configured for channeling a blood flow through the robotic device. Examples may have the beneficial effect, that the porosity of the mesh structure per se may be configured for providing a sufficient channeling of blood flow through the robotic device, e.g., for blocking a lesion of the blood vessel. Examples may have the beneficial effect, that the mesh structure per se may be used for the flow diversion by the channeling of the blood flow, when being arranged at a lesion site within the blood vessel.

For example, the porosity of the mesh structure is less than 70%. For example, the porosity of the mesh structure is preferably less than 50%.

For example, the predefined range of diameters is 0.002 mm to 25 mm. For example, the predefined range of diameters is preferably 0.01 mm to 10 mm. For example, the predefined range of diameters is more preferably 1 mm to 2 mm.

For example, the robotic device may be configured to be operated, i.e., advance using surface propulsion, in the proximal vasculature. For example, the robotic device may be configured to be operated in the distal vasculature.

Examples may have the beneficial effect, that the robotic device may be enabled to operate even in the distal M4 segment of the middle cerebral artery (MCA). For example, the robotic device may be configured to be delivered up to the end of the M3 segment of the MCA and then advance into the M4 section of the MCA. The M4 section of the MCA, i.e., the vessels exiting the Sylvian fissure and spread out over the convex surface of the cerebral hemispheres, may be for catheterization. However, with the robotic device described herein, the M4 section may be better reachable.

For example, the robotic device is made from an elastic material. For example, the robotic device is made from an elastomeric material. For example, the robotic device is made from polydimethylsiloxane, rubber, e.g., silicone rubber, hydrogel, liquid crystalline elastomer, polyurethane elastomer, protein, biomaterial, or any combination thereof.

Examples may have the beneficial effect, that using an elastic material the robotic device may be configured to adapt to different diameters of the blood vessel upholding a contact between the anisotropic surface structure and the inner surface of the blood vessel. Furthermore, the robotic device may be configured to bend around curvatures of the blood vessel and/or into branches branching form the blood vessel.

Rubber-like solids with elastic properties are referred to as elastomers. Polymer chains are held together in these materials by relatively weak intermolecular bonds, which permit the polymers to stretch in response to macroscopic stresses. An elastomer is a polymer with viscoelasticity (i.e., both viscosity and elasticity) and with weak intermolecular forces, generally low Young's modulus and high failure strain compared with other materials.

Polydimethylsiloxane (PDMS), also known as dimethylpolysiloxane or dimethicone, belongs to a group of polymeric organosilicon compounds that are commonly referred to as silicones. Silicone rubber refers to an elastomer composed of silicone polymers. The silicone polymers comprise silicon together with carbon, hydrogen, and oxygen. Hydrogel refers to a crosslinked hydrophilic polymer that does not dissolve in water.

A liquid crystalline elastomer (LCE) refers to a slightly crosslinked liquid crystalline polymer network. It combines the entropy elasticity of an elastomer with the self-organization of the liquid crystalline phase. In liquid crystalline elastomers, mesogens may either be part of the polymer chain, referred to as main-chain liquid crystalline elastomers, or attached via an alkyl spacer, referred to as side-chain liquid crystalline elastomers. A liquid crystalline phase (LC) refers to a state of matter that has properties between those of conventional liquids and those of solid crystals. For instance, a liquid crystal may flow like a liquid, but its molecules may be oriented in a crystal-like way.

Rubber may either be natural rubber or synthetic rubber. Natural rubber comprises polymers of the organic compound isoprene. It may further comprise minor impurities of other organic compounds. Synthetic rubber refers to any artificial elastomer. They may comprise polymers synthesized from petroleum byproducts.

Polyurethane refers to a class of polymers composed of organic units joined by carbamate links. Proteins are large biomolecules and macromolecules comprising one or more long chains of amino acid residues. A biomaterial refers to a substance configured to interact with a biological system for augmenting, repairing and/or replacing natural tissue of the body.

For example, the magnetic material comprises magnetic particles distributed circumferentially around the robotic device. For example, the magnetic material comprises ferromagnetic microparticles or nanoparticles.

Examples may have the beneficial effect, that the magnetic material distributed circumferentially around the robotic device may be rotated around the central longitudinal body axis of the robotic device due to a magnetic interaction between the magnetic material and the external magnetic field. The external magnetic field may be a rotational magnetic field causing a rotation of the magnetic material within the field. The rotation of the magnetic material distributed circumferentially around the robotic device may cause a rotation of the robotic device around the central longitudinal body axis of the robotic device.

For example, the magnetic material may be distributed circumferentially around the central longitudinal body axis of the robotic device. Magnetic material being distributed circumferentially around the robotic device may be distributed circumferentially around the central longitudinal body axis of the robotic device.

The magnetic material may be arranged on opposite sides of the robotic device. For a cross-section perpendicular to the central longitudinal body axis of the robotic device through the robotic device, the magnetic material may be arranged on opposite sides of the robotic device. For example, the magnetic martial may be distributed circumferentially around the robotic device following one or more helical patterns.

For example, the magnetic martial may be arranged at equal intervals, e.g., at angular distance, circumferentially around the robotic device. For example, the magnetic martial may be distributed continuously around the robotic device.

For example, the magnetic material comprises a magnetic coating film distributed circumferentially around the robotic device. For example, magnetic material may be arranged on the robotic device in form of a magnetic coating film being coated on the robotic device. Examples may have the beneficial effect, that a magnetic interaction with the external magnetic field may cause a rotation of the magnetic coating film. The rotation of the magnetic film may cause a rotation of the robotic device around the central longitudinal body axis of the robotic device.

For example, the robotic device further comprises a hemocompatible surface coating film. Examples may have the beneficial effect, that applying an additional hemocompatible surface coating film on the surfaces of the robotic device, e.g., on the outer and/or inner surface of the robotic device, may prevent adverse interactions of the robotic device with any blood components comprised by the blood in the blood vessel. Adverse interactions may, e.g., comprise an inappropriate activation or even destruction of the respective blood components. Adverse interactions may, e.g., comprise biochemical interactions with the respective blood components.

In another aspect, the invention relates to an actuation and control device configured for actuating and controlling the foldable structure of the robotic device of any of the aforementioned examples of a robotic device. The actuation and control device comprises an energy source configured for increasing the internal energy level of the foldable structure of the robotic device to a predefined internal energy level for an opening of a closed retaining section of the foldable structure upon reaching the predefined internal energy level.

For example, the energy source is a radiofrequency coil configured for radiofrequency induced heating of the foldable structure made from a shape-memory material to a predefined shape-memory transition temperature for an opening of a closed retaining section of the foldable structure upon reaching the predefined shape-memory transition temperature.

Examples may have the beneficial effect, that the energy source may enable an external energy input into the foldable structure increasing an internal energy level of the foldable structure. Thus, the internal energy level of the foldable structure may be controllable. The energy source may, e.g., be a photo-, electro-, magnetic- and/or ultrasound-source.

As long as the energy source not activated, i.e., without an external energy input, the internal energy level of the foldable structure may be below a predefined energy level. Using the energy input, the internal energy level of the foldable structure may be selectively increased. Upon reaching the predefined internal energy level of the foldable structure by the energy input, the retaining section may transit from a first closed shape to a second open shape. Thus, by activating the energy source a releasing of an agent for medical use may be induced.

For example, a radiofrequency coil may enable radiofrequency induced heating of a foldable shape-memory structure. Based on the radiofrequency induced heating an on-demand local release of the agent may be implemented. Thus, the temperature of the foldable shape-memory structure may be controllable. As long as the radiofrequency coil is not activated, i.e., without radiofrequency induced heating, the temperature of the foldable shape-memory structure may be equal to the surrounding temperature of the robotic device, i.e., body temperature, when the robotic device is inserted into a vascular system. Using the radiofrequency induced heating, the foldable shape-memory structure may be selectively heated. Upon reaching the predefined shape-memory transition temperature by the heating of the foldable shape-memory structure resulting, the retaining section transition from a first closed shape to a second open shape. Thus, by activating the radiofrequency coil a releasing of an agent for medical use may be induced.

The energy source, e.g., a radiofrequency coil, may, e.g., be mounted on a distal end of a robotic arm comprised by a moving unit of the actuation and control device. Using the robotic arm, the radiofrequency coil may be arranged in the vicinity of the robotic device for the radiofrequency induced heating.

For example, the actuation and control device further comprises a moving unit configured for moving a magnet configured for generating a magnetic field. The magnetic field is configured for inducing a rotation of the robotic device inserted in a blood vessel of a vascular system around a central longitudinal body axis of the robotic device. The moving unit is configured for moving the magnet along the blood vessel magnetically forcing movement of the robotic device through the blood vessel.

Examples may have the beneficial effect, that the actuation and control device may be configured for actuating and controlling the movement of the robotic device within the blood vessel. The movement of the robotic device may be actuated by generating the magnetic field using the magnet. The magnetic field, e.g., a rotating magnetic field, generated using the magnet is configured to rotate the robotic device around its central longitudinal body axis resulting in a movement of the robotic device along the blood vessel due to surface propulsion. The movement of the robotic device may be controlled by moving the magnet along the blood vessel resulting in a magnetically forced movement of the robotic device through the blood vessel. When the movement of the moving unit and/or the operation of the magnet is stopped, the movement of the robotic device may be stopped. For example, the robotic device may be anchored in place due to the frictional forces between the robotic device and the blood vessel.

For example, the moving unit comprises a robotic arm with the magnet mounted on a distal end of the robotic arm. Examples may have the beneficial effect, that a robotic arm may enable a precise and flexible movement of the magnet along the blood vessel.

The robotic arm with the magnet may be the same robotic arm, on which the radiofrequency coil is mounted. The robotic arm with the magnet may be a second robotic arm provided in addition to the robotic arm, on which the radiofrequency coil is mounted.

For example, the magnet may be moved along the blood vessel at a predefined distance or within a predefined range of distances. For example, the robotic arm may be configured for moving the magnet along the blood vessel at the predefined distance or within the predefined range of distances.

For example, the magnet is a permanent magnet, which is rotated for generating the magnetic field. The magnetic field being generated by rotating the permanent magnet may be a rotating magnetic field. Examples may have the beneficial effect, that by rotating the permanent magnet a rotating magnetic field may be generated for rotating the robotic device around its central longitudinal body axis.

For example, the magnet is an electromagnet, in which the magnetic field is produced by an electric current. Examples may have the beneficial effect, that the magnetic field may be switched don and of bay switching on and of the electric current. For example, the electromagnet may be rotated for generating a rotating magnetic field.

For example, the actuation and control device further comprises a computational unit. The computational unit comprises a processor and a memory storing program instructions executable by the processor. Execution of the program instructions by the processor may further cause the computational unit to activate an energy source configured for inputting energy into a foldable structure comprised by the robotic device increasing an internal energy level of the foldable structure to a predefined internal energy level. Upon reaching the predefined internal energy level, a closed retaining section of the foldable structure may be opened and an agent for medical use retained therein may be released. Examples may have the beneficial effect, that the computational unit of the actuation and control device may be configured for controlling a movement and/or actuation of the energy source. Examples may have the beneficial effect, that the computational unit of the actuation and control device may be configured for controlling the movement and/or actuation of the energy source and thus the internal energy level of the foldable structure.

For example, an execution of the program instructions by the processor causes the computational unit to activate a radiofrequency coil for a radiofrequency induced heating of a foldable shape-memory structure comprised by the robotic device to a predefined shape-memory transition temperature. Upon reaching the predefined shape-memory transition temperature, a closed retaining section of the foldable shape-memory structure may be opened and an agent for medical use retained therein may be released. Examples may have the beneficial effect, that the computational unit of the actuation and control device may be configured for controlling the movement and/or actuation of the radio frequency coil and thus the temperature of the foldable shape-memory structure.

Execution of the program instructions by the processor may further cause the computational unit to move the energy source, e.g., a radiofrequency coil, within a predefined vicinity of a target destination of the robotic device for the release of the agent.

Execution of the program instructions by the processor may further cause the computational unit to move the magnet generating the magnetic field along the blood vessel magnetically forcing movement of the robotic device through the blood vessel to a predefined destination within the vascular system.

Examples may have the beneficial effect, that the computational unit of the actuation and control device may be configured for controlling the movement and/or actuation of the magnet generating the magnetic field. By controlling the movement of the magnet, the movement of the robotic device along the blood vessel in the vascular system may be controlled. Thus, a movement of the robotic device through the blood vessel to a predefined destination within the vascular system may be forced.

In another aspect, the invention relates to a system comprising the robotic device of any of the aforementioned examples of a robotic device and the actuation and control device of any of the aforementioned examples of an actuation and control device.

In another aspect, the disclosure relates to a method for actuating and controlling the foldable structure of the robotic device of any of the aforementioned examples of a robotic device using the actuation and control device of any of the aforementioned examples of an actuation and control device. The method comprises actuating an energy source configured for increasing an internal energy level of the foldable structure of the robotic device with a closed retaining section configured for retaining an agent for medical use, The internal energy level of the foldable structure is increased to a predefined internal energy level at which the closed retaining section is opened.

Examples may have the beneficial effect, that by actuating the energy source for an energy input into a foldable structure increasing, the internal energy level of the foldable structure may be selectively increased. Upon reaching the predefined internal energy level, a closed retaining section of the foldable structure may be opened and an agent for medical use retained therein may be released. For example, the robotic device may be moved to a predefined destination within the vascular system for release of the agent. Upon reaching the predefined destination, the energy source may be actuated and the release of the agent at the predefined destination within the vascular system initiated.

For example, the energy soured is a radiofrequency coil for radiofrequency induced heating of the foldable structure made from a shape-memory material to a predefined shape-memory transition temperature with the closed retaining section opening upon reaching the predefined shape-memory transition temperature.

Examples may have the beneficial effect, that by actuating the radiofrequency coil for a radiofrequency induced heating of a foldable shape-memory structure, the temperature of the shape-memory transition temperature may be selectively increased. Upon reaching the predefined shape-memory transition temperature, a closed retaining section of the foldable shape-memory structure may be opened and an agent for medical use retained therein may be released. For example, the robotic device may be moved to a predefined destination within the vascular system for release of the agent. Upon reaching the predefined destination, the radiofrequency coil may be actuated and the release of the agent at the predefined destination within the vascular system initiated.

For example, the method further comprises moving a magnet generating a magnetic field along a blood vessel of a vascular system magnetically forcing movement of the robotic device inserted in the blood vessel through the blood vessel to a predefined destination within the vascular system.

Examples may have the beneficial effect, that by moving the magnet generating the magnetic field along the blood vessel, the robotic device may be magnetically forced to move through the blood vessel to a predefined destination within the vascular system by a surface propulsion resulting from a rotation of the robotic device due to the magnetic field.

The above-described examples and embodiments may be combined freely as long as the combinations are not mutually exclusive.

In the following, embodiments of the invention are described in greater detail in which

Figs. 1    show an exemplary robotic device with foldable structures;
Fig. 2    illustrates an exemplary radiofrequency-based heating and release of a composite cargo;
Fig. 3    shows a cross-sectional view of an exemplary robotic device with a foldable structure;
Fig. 4    shows a cross-sectional view of another exemplary robotic device with a foldable structure;
Fig. 5    shows an exemplary effect of thrombolysis using tPA;
Fig. 6    shows a comparison of exemplary quantitative results on the effect of thrombolysis of Fig. 5;
Fig. 7    shows a schematic drawing of an exemplary robotic device;
Fig. 8    shows an exemplary implementation of a robotic device;
Fig. 9    shows an exemplary 3D digital model of a robotic device;
Fig. 10    shows a cross-sectional view of an exemplary robotic device;
Fig. 11    a cross-sectional view of an exemplary robotic device;
Fig. 12    shows a cross-section of an exemplary implementation of a robotic device;
Fig. 13    shows a cross-section of an exemplary implementation of a robotic device;
Fig. 14    shows a cross-section of an exemplary implementation of a robotic device;
Fig. 15    shows a cross-section of an exemplary implementation of a robotic device;
Fig. 16    shows a cross-section of an exemplary implementation of a robotic device;
Fig. 17    shows a cross-section of an exemplary implementation of a robotic device;
Fig. 18    shows a cross-section of an exemplary implementation of a robotic device;
Fig. 19    shows a cross-section of an exemplary implementation of a robotic device;
Fig. 20    shows an exemplary actuation and control device;
Fig. 21    illustrate an exemplary actuation of a robotic device;
Fig. 22    shows an exemplary controlling of a movement of a robotic device;
Fig. 23    shows an exemplary concept of an application scenario of a robotic device;

Fig. 24 illustrates a relation between forces, when the robotic device moves along with the flow of blood;

Fig. 25 illustrates a relation between forces, when the robotic device moves against the flow of blood;

Fig. 26 illustrates a relation between forces, when the robotic device halts at a targeted location;

Fig. 27 shows an exemplary radial shape adaptation of a robotic device;

Fig. 28 shows a further exemplary radial shape adaptation of a robotic device;

Fig. 29 shows an exemplary diagram of the completeness of an anisotropic surface structure;

Fig. 30 shows an exemplary modeling of a maximum distance between the magnet and the robotic device;

Fig. 31 shows an exemplary comparison of a dependence of a maximum distance between the magnet and the robotic device on a lumen diameter;

Fig. 32 shows a comparison of a left-hand and a right-hand rotation for an anisotropic surface structure;

Fig. 33 illustrates the effect of a magnet translation speed on a robotic device displacement;

Fig. 34 illustrates a self-anchoring of a robotic device in a phantom;

Fig. 35 illustrates physiological requirements on curved lumens with flow speed visualization;

Fig. 36 illustrates an effect of lumen's curvature and lumen diameter on a minimum required torque to bend a robotic device;

Fig. 37 shows exemplary snapshots for retrievable curved routes traversing a curvature of a blood vessel;

Fig. 38 shows an exemplary illustration of a coordinate system and via-points discretization for a curved route traversing;

Fig. 39 shows exemplary strategies for a curved route traversing along the flow as well as against the flow;

Fig. 40 shows exemplary experimental results of robotic device locomotion speed among various radii of curvature in different phantoms;

Fig. 41 shows exemplary snapshots for a retrievable branch traversing among a bifurcation;

Fig. 42 shows an exemplary coordinate system and via-points discretization a branch traversing among a bifurcation;

Fig. 43 shows exemplary strategies for a branch traversing long the flow as well as against the flow;

Fig. 44 shows exemplary results of locomotion speed of the robotic device among various bifurcation angles in different phantoms;

Fig. 45 shows an exemplary locomotion along a tortuous route;

Fig. 46 shows an exemplary locomotion of a robotic device in a strongly curved route;

Fig. 47 shows an exemplary locomotion among branches;

Fig. 48 shows an exemplary locomotion among 3D branches in a cranium simulant;

Fig. 49 shows an exemplary locomotion among 3D branches in a cranium simulant;

Fig. 50 shows an exemplary delivery of a robotic device;

Figs. 51 show exemplary X-ray imaging parameters for detecting the robotic device;

Figs. 52 show exemplary properties of sonographic image acquisition;

Fig. 53 shows exemplary X-ray imaging tests;

Fig. 54 shows further exemplary X-ray imaging tests;

Fig. 55 shows an exemplary robotic device;

Fig. 56 shows an exemplary robotic device before and after being arranged in a microcatheter;

Fig. 57 shows an exemplary blood vessel with a saccular-shape aneurysm;

Fig. 58 shows the robotic device arranged at the saccular-shape aneurysm of Fig. 57;

Fig. 59 shows an exemplary flow diversion using a robotic device;

Fig. 60 shows an exemplary actuation and control device;

Fig. 61 shows an exemplary computational unit; and

Fig. 62 shows a flow diagram of another exemplary method for controlling a radiofrequency coil.

In the following similar features are denoted by the same reference numerals.

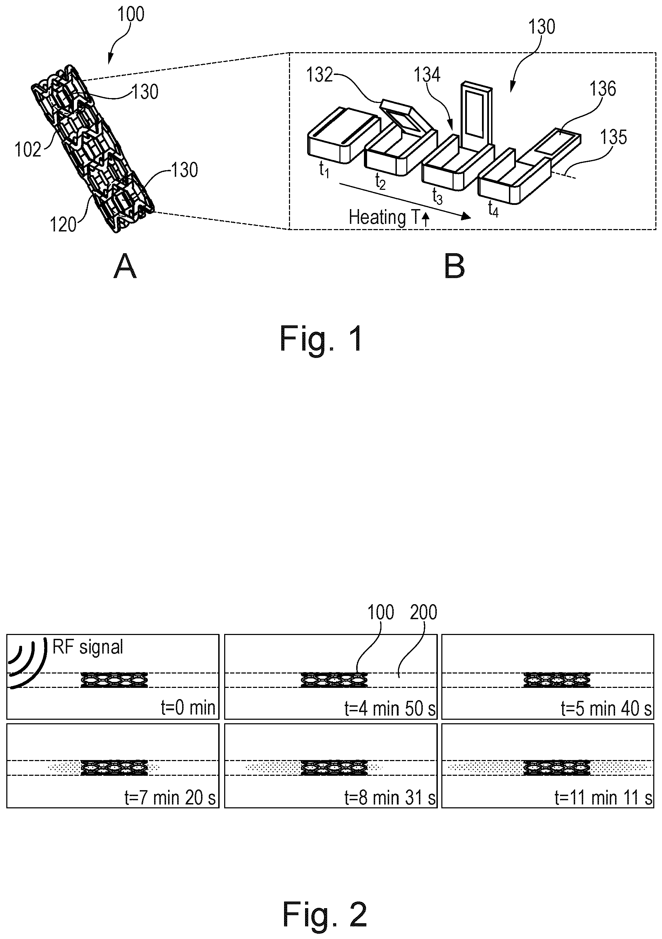

Fig. 1 show an exemplary robotic device 100 with foldable structures 130, e.g., SMP-based foldable structures, configured for a local on-demand delivery of an agent 140 for medical use. The agent may, e.g., be an endovascular tissue plasminogen activator (tPA), e.g., to achieve thrombolysis at a target location. Such a local on-demand delivery of tPA may, e.g., be used for a therapy of an acute ischemic stroke. Fig. 1A shows an exemplary robotic device 100 comprising a hollow cylindrical elastic base structure 102 provided in form of a mesh structure 120. Into the mesh structure SMP-based foldable structures 130 are integrated. In the example of Fig. 1A, two SMP-based foldable structures 130 are integrated. Fig. 1B shows the SMP-based foldable structures 130 of Fig. 1A in detail along a time line from a time $t_1$ over a time $t_2$ and $t_3$ to a time $t_4$. The exemplary SMP-based foldable structures 130 as illustrated in Fig. 1B comprises rectangular retaining section 132 providing a cuboidal cargo hold configured for receiving an agent 136. In order to close the retaining section 132 and prevent the agent 136 from being released, the retaining section 132 may be folded around a folding axis 135 into to a cuboidal reception 134 provided by the SMP-based foldable structures 130. As long as the retaining section 132 is folded in and received by the cuboidal reception 134, the retaining section 132 is covered by the reception 134 and the agent 136 arranged within the retaining section 132 is prevented from being released. When the retaining section 132 is expanded by being unfolded around the folding axis 135 out of the cuboidal reception 134 provided by the SMP-based foldable structures 130, the retaining section 132 is not covered any longer by the reception 134 and the agent 136 is released from the retaining section 132.

The time line illustrates different states of the SMP-based foldable structure 130 from a closed state, i.e., folded state, at time $t_1$ to an open state, i.e., unfolded state at time $t_4$. At temperatures below a predefined shape-memory transition temperature, the SMP-based foldable structure 130 is in the folded state, i.e., closed state. In the folded state, the retaining section 132 is folded around the folding axis 135 and received by the reception 134. Using heating, i.e., radiofrequency induced heating, the temperature T of the SMP-based foldable structure 130 may be raised. Upon reaching the predefined shape-memory transition temperature, SMP-based foldable structure 130 may open the closed retaining section 132 and releasing the agent 136. This opening is illustrated at times $t_2$ to $t_4$. The retaining section 132 is unfolded around the folding axis 135. For example, the retaining section 132 is unfolded by being rotated around the folding axis 135 by 180°. At times $t_2$ and $t_3$ the retaining section 132 is partially unfolded. At time $t_4$ the retaining section 132 has reached its final unfolded state. The SMP-based foldable structure 130 may, e.g., metal particles for enhancing the efficiency of radiofrequency induced heating of the SMP-based foldable structure, e.g., using an external radiofrequency coil.

Besides the medical functions demonstrated in the following, with its superiority in local on-demand drug delivery, the robotic device 100 may further avoid the risks and complications of a second systematic exposure to tPA. Clinically, after a first intravenous thrombolytic attempt does not achieve the desired recanalization, it is unsafe to expose the whole vascular system to tPA again immediately. This is due to the risks of internal hemorrhage from the repetitive, systematic exposure of the medicine. Thus, the proposed robotic device 100 described herein may function as a solution for such a scenario. Furthermore, the customized concentrated drug may be developed and loaded into the robotic device 100 to improve its therapeutic efficiency. For example, it is demonstrated that the local application of 0.02 mg tPA may enable successful thrombolysis for thrombus with a volume of 3.5 mm$^3$. However, the concentration of the active gradients, alteplase, is only 2.1% in the commercial Actilyse®. 467 mg tPA may contains 10 mg alteplase. Thus, an even smaller amount of drug may be needed with a more concentrated alteplase. In addition, other functional medical tools, e.g., miniature flow pumps realized by cilia-like structures, may also be appropriately integrated into the robotic device 100 to enhance drug delivery efficiency further.

The structure 130 may, e.g., be fabricated with SMP materials using a molding technique. The structure 130 as illustrated for time step $t_4$ may, e.g., firstly be 3D-printed and then used to make the negative molds of PDMS, e.g., with a base and crosslinker mass ratio 10:1. For 3D-printing a positive model of the structure 130, a 3D digital model defining the structure 130 may be used as a template. The template may be provided to a computational unit configured for controlling a 3D-printing device for 3D printing a physical copy of the template. The materials used for the synthesis of the SMP are, e.g., Poly(Bisphenol A-coepichlorohydrin) glycidyl end-capped (PBGD), Poly(propylene glycol) bis(2-aminopropyl ether) (Jeffamine D230), and Neopentyl glycol diglycidyl ether (NGDE). For example, 1 g PBGD is melted by heating in an oven at 70 °C for 20 min. Next, 360 $\mu$L Jeffamine D230 and 300 $\mu$L NGDE are added to the melted PBGD and stirred for 5 minutes. The resulting solution may, e.g., be mixed with the iron oxide particles (Sigma-Aldrich), e.g., with a mass ratio of 1:4. The composite may be cast into the prepared PDMS molds. After curing at 100 °C for 1.5 h in an oven and post-curing at 130 °C for 1h, the SMP-based structure may be demolded from the PDMS mold.

Fig. 2 illustrates an exemplary radiofrequency-based heating and release of a composite cargo. The exemplary composite cargo released in Fig. 2 consists of silk fibroin and fluorescence dye. At time t = 0 min, a radiofrequency-based heating of the robotic device 100 is started. Shown is the releasing of the cargo and the spreading of the cargo within a blood vessel 200 at times after the initiating of the heating.

Given its promising locomotion capability in distal artery lumen environments under pulsatile flow conditions, the robotic device 100 may function as a wireless medical device. Particularly, two proof-of-concept functions demonstrate that the robotic device may, e.g., used to realize local on-demand drug delivery, potentially enhancing current state-of-the-art catheter-based therapies for AIS, aneurysm, and AVM in the distal arteries.

For AIS in the middle cerebral artery, large vessel occlusions in the proximal M1 and M2 may, e.g., be successfully treated with intra-arterial mechanical thrombectomy. However, given the tortuosity, narrower lumen diameters, and deeper territory in the distal M3 and M4 regions, therapies for the occlusions there are not standardized with mechanical thrombectomy, where the intravenous or intra-arterial thrombolytics may be applied. Intravenous thrombolytics may fail to recanalize one-half to two-thirds of these distal occlusions. Meanwhile, catheter-based intra-arterial thrombolytics are still limited by accessibility. To enhance the thrombolysis in these distal vascular regions, using the wireless robotic device 100 to locally deliver a tissue plasminogen activator (tPA). This method may be promising for improving the effectiveness of such medicine, since the releasing location and quantity may be actively controlled. Moreover, the targeted application of a drug may improve the local concentration, thus minimizing the systematic exposure and reducing undesired complications.

To achieve this goal, shape-memory polymer (SMP)-based foldable structures 130 are suggested as an example, which may hold the tPA inside and release it through remote radiofrequency (RF) heating. The demonstration of the working principle is indicated in Fig. 2 using a fluorescence dye. The RF coil is, e.g., placed 20 mm away from the robotic device 100, which also satisfies $I_s$. The robotic device 100 comprises magnetic material, e.g., iron oxide ($Fe_3O_4$) nanoparticles, to enhance the efficiency of RF heating. For example, the may ratio $PDMS:NdFeB:Fe_3O_4$ may be 1:3:1. The maximum power used for RF heating may, e.g., be 752 A with 337 kHz, e.g., using EASYHEAT, Ambrell Corporation.

After heating for around 5 mins, the SMP may reach its predefined shape-memory transition temperature of 30 °C $\pm$ 5 °C and the structures open. Note that the transition temperature may be tuned precisely up to 90 °C using the method adopted in this work, promising for actual clinical usage, where the average human body temperature is 37 °C. After the SMP structures opened, e.g., a capsulated solid pill, made of silk fibroin and fluorescence dye, may be dissolved into the deionized water, which may be visualized clearly via the diffusion of the yellow color.

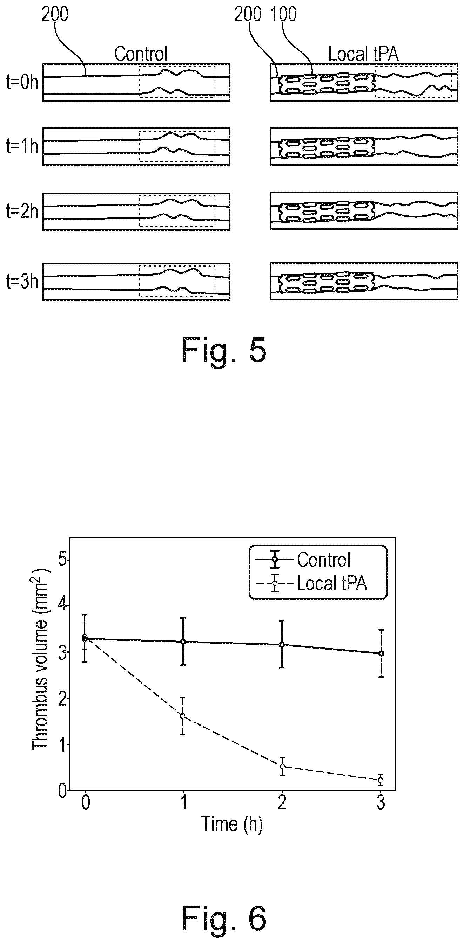

For the visualization demonstration of cargo releasing, a pill made of silk fibroin aqueous solution (15 wt%) doped with fluorescence dye solution (50 wt%) may be used (e.g., volume ratio 1:3). The compound solution may be cast into PDMS molds with a desired pill shape. After the water is evaporated, solid pills may be acquired and assembled into the slots 132 of the SMP-based structures 130 by using, e.g., Ecoflex 00-10 (Smooth-On, Inc.), as the connection agent. The overall structure may be heated to 50 °C and manually closed, as shown in step $t_1$ in Fig. 1B. Then the shape may be held until the temperature is decreased to room temperature, i.e., 23°C, to fix the shape of SMP. For the final step, the structures 130 with the incorporated pills may be assembled to an inner beam of the robotic device 100 using Ecoflex 00-10. For thrombolysis tests, e.g., according to Fig. 2 and 5, the tPA powder (Actilyse®, Boehringer Ingelheim International GmbH) may be directly loaded into the slots 132 of the SMP-based foldable structures 130.

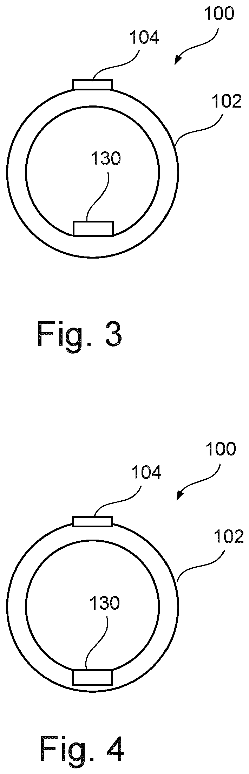

Fig. 3 shows a cross-sectional view of an exemplary robotic device 100 with a foldable structure 130, e.g., a foldable shape-memory structure. The robotic device 100 comprises a hollow cylindrical elastic base structure 102. The robotic device 100 comprises an outer anisotropic surface structure 104 implemented in form of one or more protrusions added, e.g., coated onto the elastic base structure 102. The foldable shape-memory structure 130 is arranged on an inner surface of the base structure 102 and configured to open the closed retaining section in radial direction towards a central longitudinal body axis of the robotic device 100. The foldable structure 130 may, e.g., comprise a shape-memory material, like a shape-memory polymer. The foldable structure 130 may, e.g., comprise a photo-, an electro-, a magnetic- and an ultrasound-responsive material.

Fig. 4 shows a cross-sectional view of another exemplary robotic device 100 with a foldable structure 130, e.g., a foldable shape-memory structure. The robotic device 100 comprises a hollow cylindrical elastic base structure 102. The robotic device 100 comprises an outer anisotropic surface structure 104 implemented in form of one or more protrusions added, e.g., coated onto the elastic base structure 102. The foldable shape-memory structure 130 is integrated into the base structure 102 and configured to open the closed retaining section in radial direction towards a central longitudinal body axis of the robotic device 100. The foldable structure 130 may, e.g., comprise a shape-memory material, like a shape-memory polymer. The foldable structure 130 may, e.g., comprise a photo-, an electro-, a magnetic- and an ultrasound-responsive material.

Fig. 5 shows an exemplary effect of thrombolysis using tPA carried, e.g., using the robotic device 100 of Fig. 1A. The thrombi are labeled by black dotted lines. To confirm the efficacy of the local on-demand drug delivery, is illustrated for an exemplary release of 0.02 mg tPA (Actilyse®, Boehringer Ingelheim International GmbH) in the vicinity of fabricated blood thrombi in blood vessels 200. The blood thrombi have volumes of around 3.5 mm$^3$. The samples were kept at a constant temperature of 37°C. A comparison of thrombolysis results with a control group without delivery of tPA is shows for illustrating the effectiveness of the local application of the tPA. Clinically, the robotic device 100 may, e.g., be immediately retrieved after the drug is released to the thrombus. The comparison of the results is shown at the time 0 h of the release of the tPA, at 1 h, 2 h, and 3 h after the release.

Fig. 6 shows a comparison of the exemplary quantitative results on the effect of thrombolysis for the setup of Fig. 5. As shown, the size of the thrombus volume may be significantly reduced by the local releasee of tPA compared to the control group.

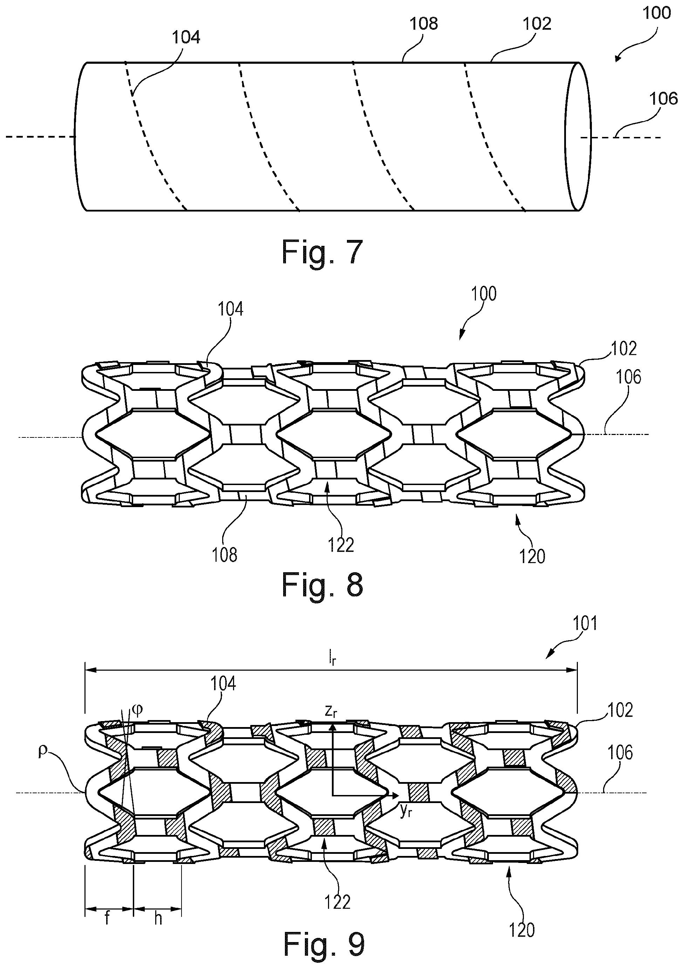

Fig. 7 shows a schematic drawing of an exemplary tube-shaped robotic device 100, i.e., a robotic device provided in form of a tube. The robotic device 100 comprises a hollow cylindrical elastic base structure 102. The robotic device 100 is configured for an insertion into a blood vessel of a vascular system. The robotic device 100 comprises an outer anisotropic surface structure 104. The anisotropic surface structure 104 comprises a geometrical anisotropy between a direction circumferential to the robotic device 100 and a direction longitudinal to the robotic device 100. The elastic base structure 102 is configured for providing a radial elastic force configured for establishing a contact between the anisotropic surface structure 104 of the robotic device 100 and an inner surface of the blood vessel. The anisotropic surface structure 104 is configured for establishing by the contact an anisotropic friction between the anisotropic surface structure 104 and the inner surface of the blood vessel. The anisotropic friction results in a surface propulsion of the robotic device 100, when the robotic device is 100 rotated around a central longitudinal body axis 106 of the robotic device 100. The robotic device 100 further comprises a magnetic material 108 distributed circumferentially around the robotic device 100 and configured for establishing within an external magnetic field a rotation of the robotic device 100 around the central longitudinal body axis 106 of the robotic device 100. In the example of Fig. 7, the magnetic material 108 are comprised by the elastic base structure 102 and thus distributed circumferentially around the robotic device 100 as well as the central longitudinal body axis 106 of the robotic device 100. The magnetic material 108 may, e.g., be distributed in form of ferromagnetic microparticles or nanoparticles. Alternatively, the magnetic material 108 may, e.g., be distributed in form of a magnetic coating film on a surface of the elastic base structure 102. The magnetic particles or the magnetic coating film may be distributed circumferentially around the robotic device 100 following a predefined pattern. For example, the magnetic material 108 may, e.g., be comprised by the anisotropic surface structure 104. The magnetic coating film may, e.g., be a coating film continuously covering an outer or inner surface of the robotic device 100.

For example, the elastic base structure 102 is configured for passively adapting a diameter of the robotic device 100 to altering diameters of the blood vessel for diameters of the blood vessel altering within a predefined range of diameters.

Given the limited accessibility of catheterization into the distal vascular routes, a magnetically-driven wireless robotic device 100 may provide advantages. First, in respect of accessibility, the controlled retrievable locomotion may improve maneuverability into distal regions with self-anchoring assurance given flow or no flow in the lumen. Second, safe interaction forces may be enabled, where lower forces may be applied to the vessel walls during its intervention (max. on the order of $10^{-2}$ N) compared to standard neuro-interventional catheterization (max. on the order of $10^0$ N). Thus, potential safe mechanical forces may be applied to the blood vessel walls to lower the risk of endothelial cell ruptures. Third, controlled local drug delivery capability may, e.g., improve medicine concentration while minimizing the systematic side effects. Finally, a position may be adjusted actively, if misplacement or dislodgement during the endovascular operations or migrations after the surgeries occurs, e.g., as flow diverters to treat wide-neck aneurysms.

The design of the robotic device 100 may, e.g., be potentially extended to the proximal arteries, given the enlarged magnetic moment of the robotic device 100 in the larger lumen diameters and the low coefficients of friction in the arteries. Combining intervention and therapeutics, the robotic device 100 may have enhanced performance over the existing intra-arterial approaches in the proximal regions. For example, the robotic device 100 may be wirelessly and precisely adjusted when the robotic device 100 is misplaced or a dislodged during the endovascular treatment, e.g., a local on-demand drug release. For the follow-up adjustments of the possible migration, further invasive catheterization may not even be needed since the robotic device's 100 location may be tuned wirelessly using external magnetic control and, e.g., proper X-ray imaging.

The overall concept of the mobile robotic device 100 may be combined with state-of-the-art microcatheter techniques in distal vascular systems for a systematic improvement of clinical effectiveness. However, most of the tests are conducted in realistic phantoms to understand the detailed mechanics of soft-bodied adaptive locomotion systematically. Furthermore, the range of shape adaptations may be potentially boosted utilizing active programmable magnetic materials to translate the application of the robotic device from minimally invasive to almost non-invasive surgeries.

For example, silica, which is known to be biocompatible and non-cytotoxic, may be coated on the NdFeB microparticles. Then, the silica-coated NdFeB may, e.g., be compounded with PDMS to cast the robotic device 100. Next, the surface of the robotic device 100 may, e.g., be modified by hemocompatible materials, such as siloxane-bound poly(ethylene glycol) (PEG), Parylene-C, and hyaluronic acid (HA) and/or polydopamine (PDA) composite (HA/PDA). These surface modifications have been shown to reduce fibrinogen adsorption and platelet adhesion in blood-contacting applications. Another promising method may, e.g., be to fabricate the whole device with biocompatible and hemocompatible material, e.g., ferromagnetic FePt nanoparticles and the PDMS-based polyurethane-urea bearing zwitterion sulfobetaine (PDMS-SB-UU). FePt nanoparticles have been shown to be biocompatible and hemocompatible. PDMS-SB-UU may be against lipase and 30% $H_2O_2$ for eight weeks. It also demonstrates significantly higher resistance to fibrinogen adsorption and platelet deposition compared with control PDMS and a lack of hemolysis while maintaining the noncytotoxicity.

To improve biocompatibility and hemocompatibility, e.g., the NdFeB microparticles may fisrt be coated with silica to provide an extra protective shell preventing corrosion. Then, the two composite materials, e.g., PDMS + NdFeB@SiO2 (for the robot body) and SMP + Fe3O4 (for the foldable structure), may be coated with Parylene C (, e.g., SCS Labcoter® 2 (PDS 2010), Specialty Coating Systems). Parylene C, as an FDA approved biocompatible and hemocompatible material, conforms to the stringent USP Class VI, ISO 10993, and RoHS standards.

Fig. 8 shows an exemplary implementation of a robotic device 100 with a hollow cylindrical elastic base structure 102 provided in from of a mesh structure 120. The mesh structure 120 providing a hollow cylindrical base structure 102 of the robotic device may enable a high radial deformability of the robotic device 100, while ensuring low fluidic drag properties. The mesh structure 120 is formed by a plurality of cells 122. In case of Fig. 8, the mesh structure 120 is formed by a plurality of identical cells 122, each with a rhombic shape. The anisotropic surface structure 104 is arranged on the mesh structure 120 comprising a plurality of protrusions added onto the strands forming the cells 122 and thus the mesh structure 120. The anisotropic surface structure 104 is implemented in form of a right-handed helical structure coated onto the mesh structure 120, i.e., the strands of the mesh structure 120. The mesh structure 120 may comprise magnetic material108 in form of magnetic particles, e.g., ferromagnetic microparticles or nanoparticles. The magnetic particles may, e.g., be NdFeB ferromagnetic microparticles. This NdFeB ferromagnetic microparticles inside the body of the robotic device may, e.g., be uniformly magnetized using a homogeneous magnetic field, e.g., a 1.8 T homogeneous magnetic field.

A positive model of the robotic device 100 may be 3D-printed and used to fabricate a negative PDMS mold, e.g., with a base and crosslinker mass ratio 10:1. For 3D-printing the positive model of the robotic device 100, a 3D digital model defining the robotic device 100 may be used as a template. The template may be provided to a computational unit configured for controlling a 3D-printing device for 3D printing a physical copy of the template. The composition for casting may be PDMS with the various mass ratios of 3:1, 7:1, and 12:1, and Neodymium-iron-boron particles (NdFeB, 5 $\mu$m), enabling robotic devices 100 with various Young's modulus $E_r$. The PDMS to NdFeB mass ratio may, e.g., be 1:4. The thoroughly mixed polymer may be cast into the negative PDMS mold and cured in a hot oven, e.g., at 85°C for 7 hours. Then the sample may be put in the vibrating-sample magnetometer (VSM, EZ7, Microsense), e.g., with 1.8 T for uniform magnetization. Finally, the robotic device 100 may be demolded.

Fig. 9 shows an exemplary CAD design drawing, i.e., a 3D digital model 101 of the robotic device, defining the robotic device 100 of Fig. 8. The robotic device 100 of Fig. 8 may, e.g., be manufactured using CAM-methods, like 3D printing, and the 3D digital model 101 of the robotic device as a template. The mesh structure 120 providing a hollow cylindrical base structure 102 of the robotic device may enable a high radial deformability of the robotic device 100, while ensuring low fluidic drag properties. The anisotropic surface structure 104 implemented in form of a helical structure, e.g., a right-handed helical structure, coated onto the mesh structure 120 enable a locomotion of the robotic device 100 utilizing anisotropic frictional forces, when the robotic device 101 is rotated around its body-attached $y_r$-axis, i.e., the central longitudinal body axis 106.

The design of the hollow cylindrical elastic base structure 102 illustrated in Fig. 9 may provide a shape with a desired radial deformability and low fluidic drag. This design may ensure effective operation inside arteries with a high-speed pulsatile flow and changing lumen diameter. Furthermore, to leverage rotational and translational magnetic actuation, magnetic material, e.g., in form of ferromagnetic NdFeB microparticles, may be incorporated, e.g., into the base structure 102. The magnetic material 104 may, e.g., be magnetized uniformly along a $z_r$-axis of the tube-attached coordinate system, e.g., inside a vibrating sample magnetometer (VSM), e.g., with a homogeneous 1.8 T field.

To minimize a radial force applied to the lumen of the blood vessel during locomotion of the robotic device 100, the radial stiffness $k_r$ of the overall robotic device 100 as defined by the 3D digital model 101 of the robotic device may be configured to be as small as possible. Meanwhile, the base structure 102 may be configured to prevent a collapse due to internal magnetization-induced magnetic forces. For this purpose, $k_r$ may be chosen to be high enough to prevent such undesired self-deformation. From the structural aspect, $k_r$ may be decided by three critical design parameters: the strut spacing h, the radius of curvature at the crown junction $\rho$, and the axial amplitude of each segment f as illustrated in Fig. 9. The structure employing larger h, $\rho$, and f may enable smaller $k_r$. The base structure 102 may be provided by a mesh structure 120 composed of identical diamond-shaped cells 122. This arrangement may enable a uniform distribution of the compressive stresses and frictional forces when the base structure 102 deforms, minimizing the possibility of the robotic device as defined by the 3D digital model 101 of the robotic device twisting due to uneven contact force distribution, e.g., at bifurcation branches.

For a locomotion inside the lumen of a blood vessel, the anisotropic surface structure 104 may, e.g., be implemented in form of a helical structure. The anisotropic surface structure 104 may realize an axial propulsion, when it converts rotational motion around a helical axis, i.e., the central longitudinal body axis 106, by magnetic torque to linear motion along the central longitudinal body axis 106. This may be induced by both helical shape and the anisotropic friction, where the coefficient of friction perpendicular is higher than the coefficient of friction parallel to the helix. To leverage such a property for locomotion, e.g., a right-handed helical structure with a helix angle $\phi$ of 8° may be designed on an outer side of the base structure 102, e.g., as mechanical protrusions. Given the identical arrangement of the diamond-shaped cells 122, the helix-coated area 104 along the robotic device as defined by the 3D digital model 101 of the robotic device may be evenly distributed.

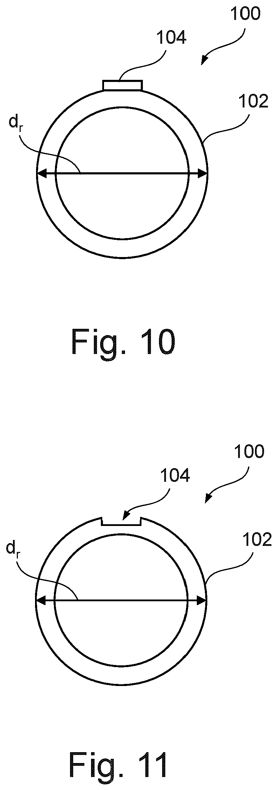

Fig. 10 shows a cross-sectional view of an exemplary robotic device 100. The robotic device 100 comprises a hollow cylindrical elastic base structure 102. The hollow cylindrical elastic base structure 102 may be configured for passively adapting a diameter $d_r$ of the robotic device 100 to altering diameters of the blood vessel. For example, the hollow cylindrical elastic base structure 102 may be configured for passively adapting the diameter $d_r$ to diameters of the blood vessel altering within a predefined range of diameters. The robotic device 100 comprises an outer anisotropic surface structure 104 implemented in form of one or more protrusions added, e.g., coated onto the elastic base structure 102. Furthermore, the robotic device 100 comprises a magnetic material 108. The magnetic material 108 may, e.g., be comprised by the elastic base structure 102. Alternatively, the magnetic material 108 may be arranged on a surface of the robotic device 100 or comprised by the anisotropic surface structure 104.

Fig. 11 shows a cross-sectional view of an exemplary robotic device 100. The robotic device 100 comprises a hollow cylindrical elastic base structure 102. The hollow cylindrical elastic base structure 102 may be configured for

EP 4 507 612 B1

passively adapting a diameter $d_r$ of the robotic device 100 to altering diameters of the blood vessel. For example, the hollow cylindrical elastic base structure 102 may be configured for passively adapting the diameter $d_r$ to diameters of the blood vessel altering within a predefined range of diameters. The robotic device 100 comprises an outer anisotropic surface structure 104 implemented in form of one or more recesses inserted into the elastic base structure 102. The magnetic material 108 may, e.g., be comprised by the elastic base structure 102. Alternatively, the magnetic material 108 may be arranged on a surface of the robotic device 100 or comprised by the anisotropic surface structure 104.

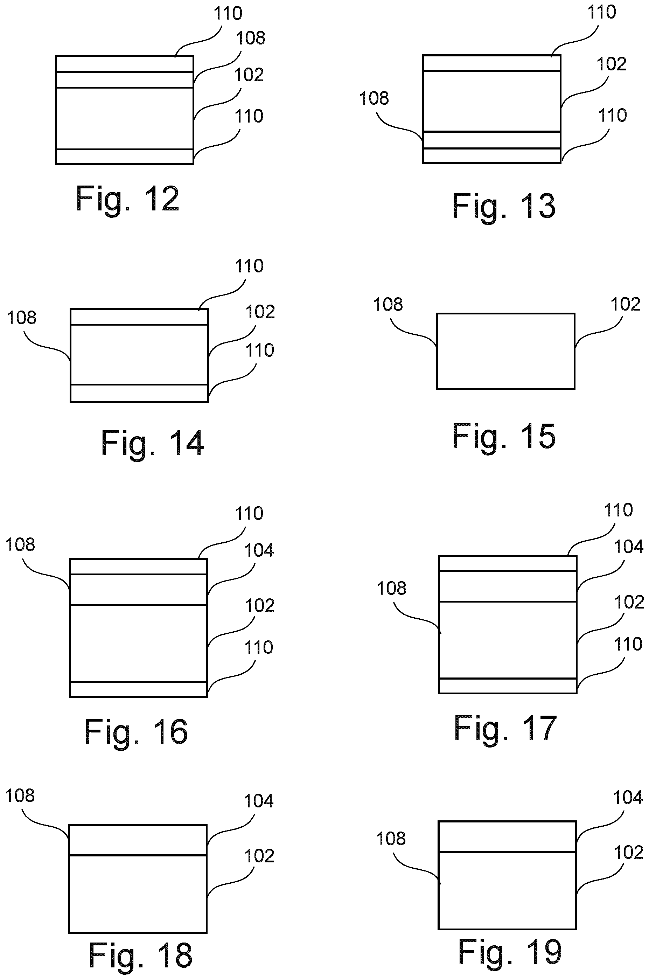

Fig. 12 to Fig. 19 show cross-sections of different implementations of the robotic device. Fig. 12 to Fig 15 show exemplary sections of robotic devices without an anisotropic surface structure extending through the depicted sections. Fig. 12 shows a section of an exemplary embodiment of a robotic device comprising an elastic base structure 102, on which a magnetic coating film 108 is arranged. On an outer surface of the robotic device provided by a top face of the magnetic coating film 108 is a hemocompatible surface coating film 110 arranged. A hemocompatible surface coating film 110 is furthermore arranged on an inner surface of the robotic device provided by a bottom face of the elastic base structure 102. Fig. 13 shows a section of another exemplary embodiment of a robotic device comprising an elastic base structure 102, under which the magnetic coating film 108 is arranged. On an outer surface of the robotic device provided by a top face of the elastic base structure 102 is a hemocompatible surface coating film 110 arranged. A hemocompatible surface coating film 110 is furthermore arranged on an inner surface of the robotic device provided by a bottom face of the magnetic coating film 108. Fig. 14 shows a section of another exemplary embodiment of a robotic device comprising an elastic base structure 102 with magnetic particles 108 distributed therein. On an outer surface of the robotic device provided by a top face of the elastic base structure 102 is a hemocompatible surface coating film 110 arranged.

A hemocompatible surface coating film 110 is furthermore arranged on an inner surface of the robotic device provided by a bottom face of the elastic base structure 102. Fig. 15 shows a section of another exemplary embodiment of a robotic device comprising an elastic base structure 102 with magnetic particles 108 distributed therein. For example, the magnetic particles may be coated with a hemocompatible coating, while the elastic base structure 102 may be made from a hemocompatible material. Thus, no further hemocompatible surface coating film may be required in case of the exemplary embodiment of a robotic device illustrated in Fig. 15.

Fig. 16 to Fig 19 show exemplary sections of robotic devices with an anisotropic surface structure 104 extending through the depicted sections. Fig. 16 shows a section of an exemplary embodiment of a robotic device comprising an elastic base structure 102, on which an anisotropic surface structure 104 is arranged. Magnetic particles 108 are comprised by the anisotropic surface structure 104. On an outer surface of the robotic device provided by a top face of the anisotropic surface structure 104 is a hemocompatible surface coating film 110 arranged. A hemocompatible surface coating film 110 is furthermore arranged on an inner surface of the robotic device provided by a bottom face of the elastic base structure 102. Fig. 17 shows a section of an exemplary embodiment of a robotic device comprising an elastic base structure 102, on which an anisotropic surface structure 104 is arranged. Magnetic particles 108 are comprised by the elastic base structure 102. On an outer surface of the robotic device provided by a top face of the anisotropic surface structure 104 is a hemocompatible surface coating film 110 arranged. A hemocompatible surface coating film 110 is furthermore arranged on an inner surface of the robotic device provided by a bottom face of the elastic base structure 102. Fig. 18 shows a section of another exemplary embodiment of a robotic device comprising an elastic base structure 102, on which an anisotropic surface structure 104 is arranged. Magnetic particles 108 are comprised by the anisotropic surface structure 104. For example, the magnetic particles may be coated with a hemocompatible coating, while the elastic base structure 102 and the anisotropic surface structure 104 may be made from a hemocompatible material. Thus, no further hemocompatible surface coating film may be required in case of the exemplary embodiment of a robotic device illustrated in Fig. 18. Fig. 19 shows a section of another exemplary embodiment of a robotic device comprising an elastic base structure 102 with magnetic particles 108 distributed therein. On the elastic base structure 102 an anisotropic surface structure 104 is arranged. For example, the magnetic particles may be coated with a hemocompatible coating, while the elastic base structure 102 and the anisotropic surface structure 104 may be made from a hemocompatible material. Thus, no further hemocompatible surface coating film may be required in case of the exemplary embodiment of a robotic device illustrated in Fig. 19.

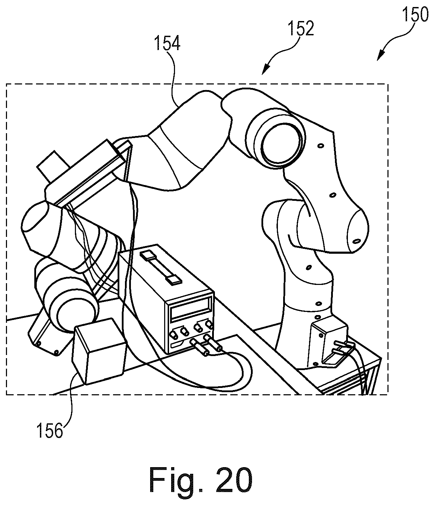

Fig. 20 shows an exemplary actuation and control device 150 configured for actuating and controlling a robotic device as described herein. The actuation and control device comprises a moving unit 152 configured for moving a magnet 156. The magnet 156 is configured for generating a magnetic field. The magnetic field is configured for inducing a rotation of the robotic device around a central longitudinal body axis of the robotic device. The moving unit 152 is configured for moving the magnet 156 along a blood vessel of a vascular system, in which the robotic device is inserted, thereby magnetically forcing a movement of the robotic device through the blood vessel using a surface propulsion of the robotic device within the blood vessel.

The moving unit 152 comprises a robotic arm 154 with the magnet 156 mounted on a distal end of the robotic arm 154. The robotic arm 154 may, e.g., be a 7-DoF robotic arm. The robotic arm 154 with the magnet 156 are configured to realize spatial three-dimension (3D) magnetic actuation of the robotic device using the magnet 156. The communication framework may be realized by a Robot Operating System (ROS). The magnet 156 may be a permanent magnet, e.g., a 50 mm cubic NdFeB permanent magnet, which is rotated for generating the magnetic field. The magnet 156 may, e.g., be rotated using a step motor.

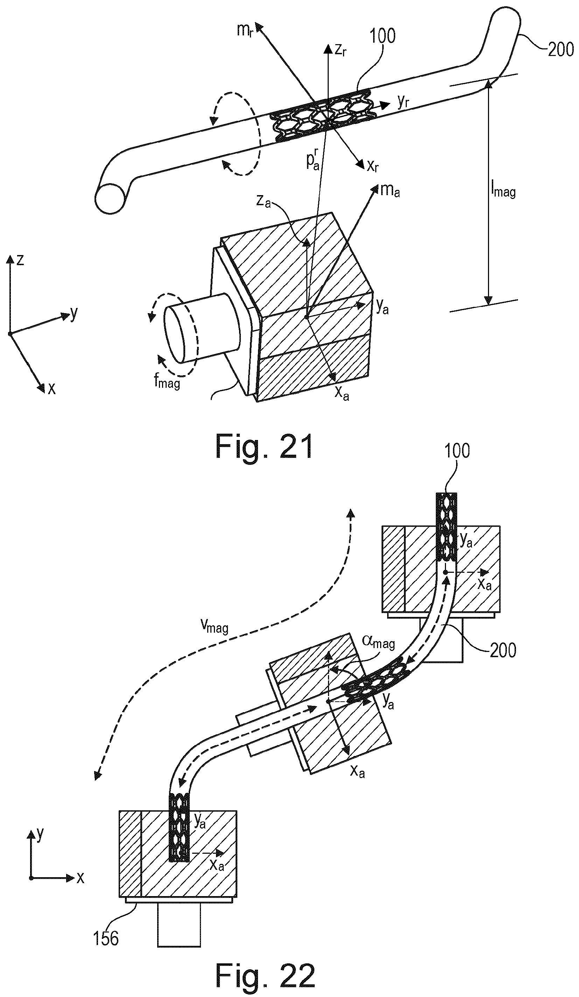

Fig. 21 shows an exemplary actuation of the robotic device 100. Fig. 21 illustrates the rotation of the robotic device 100 and a magnet 156 with respect to local coordinates. The robotic device 100 is inserted into a blood vessel 200. For actuation the robotic device 100, both magnetic torques and forces may be used. The magnet 156 with moment $m_a$ may be rotated around the $y_a$-axis of the magnet-attached local coordinates $x_a$-$y_a$-$z_a$ with frequency $f_{mag}$, such that the robotic device 100 with moment $m_r$ is rotated around the $y_r$-axis of the robot-attached local coordinates $x_r$-$y_r$-$z_r$ by magnetic torques. The orientation of the magnet 156 may be aligned with the direction of extension of the blood vessel 200 and thus with the robotic device 100. The two local coordinate frames, i.e., the magnet-attached local coordinates $x_a$-$y_a$-$z_a$ and the robot-attached local coordinates $x_r$-$y_r$-$z_r$, are separated by a translation defined by translation vector $p_a^r$, i.e., the vector pointing from the actuation magnet 156 to the robotic device 100. The direction of the rotation of the robotic device 100 may, e.g., be reverse of the magnet 156 due to the specific magnetic field generated by the permanent magnet. 156. The $y_r$-axis is the central longitudinal body axis of the robotic device 100. The magnet 156 is may further be translated and reoriented with respect to the global coordinate x-y-z. Given, e.g., a cortex-to-scalp distance $l_s$ = 15 mm, the magnet 156 may be placed at least 50 mm away from the robotic device along the $z_r$-axis, i.e., $l_{mag} \geq 50$ mm > $l_s$.

Fig. 22 shows an exemplary controlling of a movement of the robotic device 100 along the blood vessel 200 using the magnet 156. Fig. 2 illustrates a translation and reorientation of the magnet 156 along the blood vessel 200 with respect to global coordinates, in order move the robotic device 100 through the blood vessel. The magnet 156 is rotated around the $y_a$-axis with frequency $f_{mag}$, as illustrated in Fig. 21. It is translated ahead of the robotic device 100 with a speed of $v_{mag}$ along the blood vessel 200 and reorientated by an angle of $\alpha_{mag}$ to coincide the $y_a$-$z_a$ plane to the current route along the blood vessel 200. The induced magnetic field may drag the robotic device 100 along through the blood vessel with the robotic device 100 being propelled by the surface propulsion resulting from the rotation of the robotic device 100.

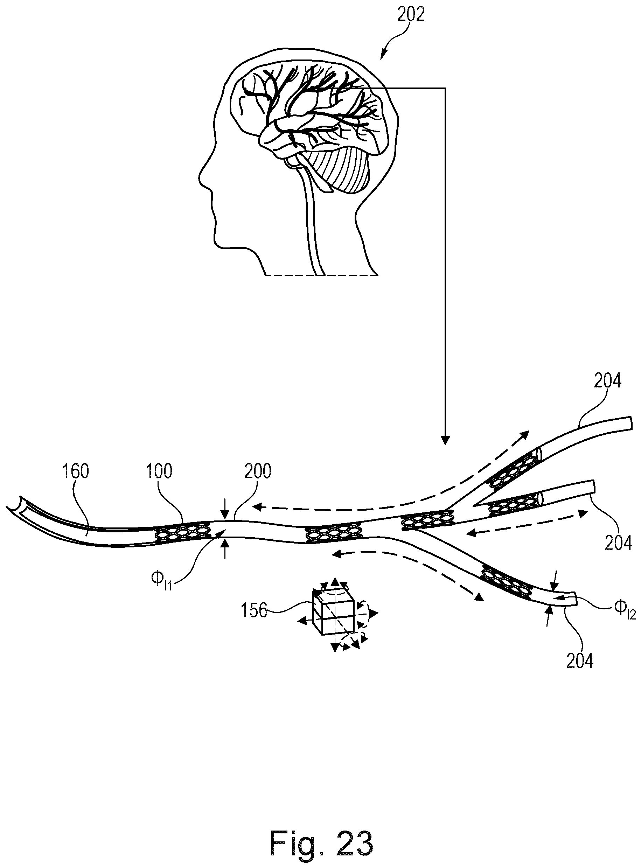

Fig. 23 shows an exemplary concept of an application scenario of a robotic device 100 in a distal vasculature. The M4 segment of the middle cerebral artery (MCA) case is shown here, where catheterization may be challenging. The M4 segment refers to the blood vessels the Sylvian fissure and spread out over the convex surface of the cerebral hemispheres. The robotic device 100 may be delivered to the M4 segment using a microcatheter 160. Using the magnet 156, the robotic device 100 may be moved into the M4 segment. For example, the branches 204 of the blood vessel 200 may have diameters $\Phi_{l2}$ smaller than a diameter $\Phi_{l1}$ of the blood vessel 200. The robotic device 100 may be configured to adapt to different diameters $\Phi_l$ of the blood vessel 200, when advancing into the branches 200. For example, the robotic device 100 may have the following major locomotion capabilities: forward and backward shape adaptation in lumens with varying diameters; flow withstanding when no magnetic field is applied; traversing among curved routes and branches of the blood vessel 200. The robotic device 100 may function as a mobile carrier for other functional tools, e.g., to treat acute ischemic stroke, aneurysm, and arteriovenous malformation.

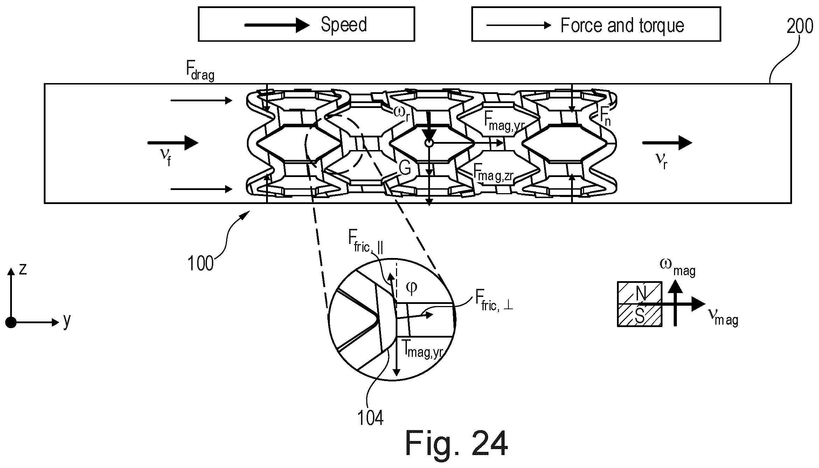

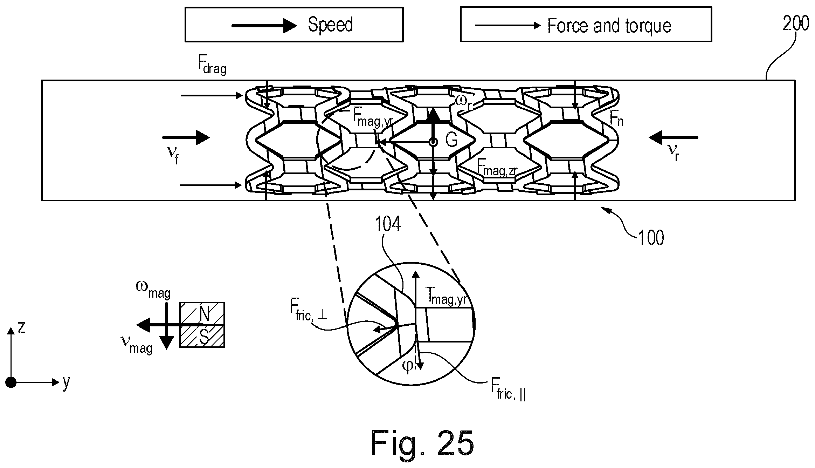

Fig. 24 illustrates a relation between forces, when the robotic device 100 moves along with the flow of blood, while the magnetic actuation is on. Fig. 25 illustrates a relation of forces, when the robotic device 100 moves against the flow, while the magnetic actuation is on.

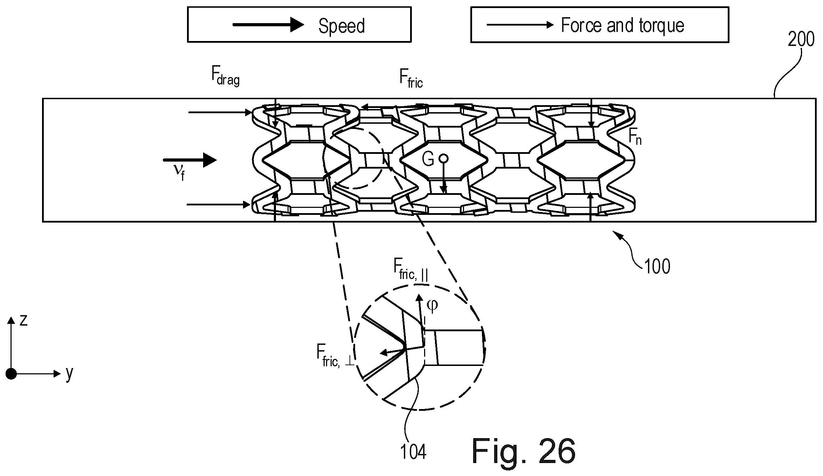

Fig. 26 illustrates a relation of forces, when the robotic device halts at a targeted location such that a self-anchoring is ensured, even if the magnetic actuation is off. There are three major groups of forces contributing to the robotic device locomotion: 1) the magnetic forces applied on the robotic device 100 along the $y_r$-axis, $F_{mag,yr}$, the magnet torque applied on the robotic device around the $y_r$-axis, $T_{mag,yr}$, 2) the fluidic drag force, $F_{drag}$, and 3) the frictional forces parallel and perpendicular to the anisotropic surface structure, e.g., a helical structure, $F_{fric,\parallel}$ and $F_{fric,\perp}$, respectively.

$F_{mag,yr}$, and $T_{mag,yr}$ may be modeled using the dipole approximation as

$$F_{mag}\left(p_a^r, m_r\right) = (m_r \cdot \nabla) B\left(p_a^r\right), \qquad\qquad (1)$$

$$T_{mag} = m_r \times B\left(p_a^r\right), \qquad\qquad (2)$$

where $m_r$ is the magnetic moment of the robotic device 100, and $B\left(p_a^r\right)$ is the magnetic flux density generated by the actuation magnet 156 with the magnetic moment of $m_a$, and $p_a^r$ is the vector pointing from the actuation magnet 156 to the robotic device 100. $F_{drag}$ may be modeled by fluid-structure interaction, e.g., in COMSOL Multiphysics 5.4. $F_{fric,\parallel}$ and $F_{fric,\perp}$ are described by the Coulomb model of friction, where the radial force $F_n$ along with the deformation are modeled, e.g., using Abaqus 2019, using the measured Young's modulus $E_r$ of the robotic device, and the coefficient of friction (CoF) may be quantified by friction tests.

The robotic device 100 as the composite of NdFeB particles and PDMS may be modeled as a linear elastic material, enabling the linear relations between $F_n$ and the radial deformation.

For the robotic device 100 to move along with the flow of blood within the blood vessel 200 as shown in Fig. 24, the force relations as shown in equations (3) and (4) need to be satisfied. The sum of the magnetic forces applied on the robotic device 100 along the $y_r$-axis, $F_{mag,yr}$, the fluidic drag force, $F_{drag}$, and the contribution of the frictional forces perpendicular to the anisotropic surface structure, $F_{fric,\perp} \cos(\varphi)$, need to be equal to or larger than the contribution of the frictional forces parallel to the anisotropic surface structure, $F_{fric,\parallel} \sin(\phi)$. Furthermore, the magnet torque applied on the robotic device around the $y_r$-axis, $T_{mag,yr}$, needs to be equal to or larger than the contributions of the frictional forces parallel and perpendicular to the anisotropic surface structure, $F_{fric,\parallel}$ and $F_{fric,\perp}$, to the torque. Similarly, for the robotic device 100 to move against the flow of blood within the blood vessel 200 as shown in Fig. 25, the force relations as shown in the relations (4) and (5) need to be satisfied. The magnet torque applied on the robotic device around the $y_r$-axis, $T_{mag,yr}$, needs to be equal to or larger than the contributions of the frictional forces parallel and perpendicular to the anisotropic surface structure, $F_{fric,\parallel}$ and $F_{fric,\perp}$, to the torque. Furthermore, the sum of the magnetic forces applied on the robotic device 100 along the $y_r$-axis, $F_{mag,yr}$, and the contribution of the frictional forces perpendicular to the anisotropic surface structure,

$F_{fric,\perp} \cos(\varphi)$, need to be equal to or larger than the sum of the fluidic drag force, $F_{drag}$, and the contribution of the frictional forces parallel to the anisotropic surface structure, $F_{fric,\parallel}\sin(\varphi)$. For a self-anchoring of the robotic device 100 at the target location within the blood vessel 200 as shown in Fig. 26, the force relation as shown in the relation (6) needs to be satisfied. The contribution of the frictional forces parallel to the anisotropic surface structure, $F_{fric,\parallel}\sin(\varphi)$, and the contribution of the frictional forces perpendicular to the anisotropic surface structure, $F_{fric,\perp} \cos(\varphi)$, need to be equal to or larger than fluidic drag force, $F_{drag}$, of the blood.

$$F_{mag,yr} + F_{drag} + F_{fric,\perp} \cos(\varphi) \geq F_{fric,\parallel} \sin(\varphi), \tag{3}$$

$$T_{mag,yr} \geq \left( F_{fric,\parallel} \cos(\varphi) + F_{fric,\perp} \sin(\varphi) \right) R_d, \tag{4}$$

$$F_{mag,yr} + F_{fric,\perp} \cos(\varphi) \geq F_{drag} + F_{fric,\parallel} \sin(\varphi), \tag{5}$$

$$F_{drag} \leq F_{fric,\parallel} \sin(\varphi) + F_{fric,\perp} \cos(\varphi), \tag{6}$$

where $F_{fric,\perp} = \mu_{\perp 0} F_n$, $F_{fric,\parallel} = \mu_{\parallel} F_n$, $F_n$ is the radial force, $\varphi$ is the helix angle, which is 8°, $R_d$ is the robotic device radius after radial deformation, $\mu_\perp$ and $\mu_\parallel$ are the coefficient of friction (CoF) perpendicular to and parallel to the helix, respectively. $F_{mag,yr}$ here refers to the maximum achievable value, and $T_{mag,yr}$ refers to the torque value, when $F_{mag,yr}$ is acquired.

Concerning the dynamics of the robotic device 100 during locomotion, it may be modeled as:

$$F_{mag,yr} + F_{fric,\perp} \cos(\varphi) - F_{fric,\parallel} \sin(\varphi) + a F_{drag} = m_r \ddot{w}_{yr}, \tag{7}$$

$$T_{mag,yr} - \left( F_{fric,\parallel} \cos(\varphi) + F_{fric,\perp} \sin(\varphi) \right) R_d = J_{yr} \ddot{\theta}_{yr}, \tag{8}$$

where a is a parameter equal to 1 or -1, when the robotic device 100 moves along and against the flow, respectively. $w_{yr}$ is the displacement of the robotic device 100 along the $y_r$-axis, $m_r$ is the mass of the robotic device 100, $\theta_{yr}$ is the rotation angle of the robotic device 100 around the $y_r$-axis, and $J_{yr}$ is the moment of inertia around the $y_r$-axis. The dynamic equation may, e.g., be solved by the ODE23 solver in MATLAB R2018a.

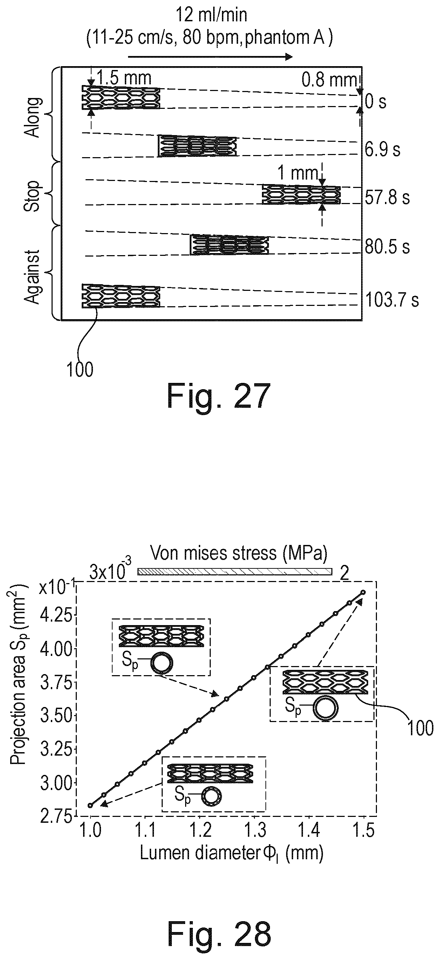

Fig. 27 shows an exemplary radial shape adaptation of the robotic device 100. Snapshots for the retrievable radial shape adaptation of the robotic device 100 in the lumen with diameter $\Phi_l$ changing from 1 mm to 1.5 mm for phantom A is shown. The shape adaptation may be quantified by the maximum allowed distance of the magnet away from the robotic device 100 along the $z_r$-axis enabling the locomotion, $l_{mag\_max}$. $l_{mag\_max}$ is decided by the projection area $S_p$, completeness of helix $\lambda_h$, diameter of the lumen $\Phi_i$, and Young's modulus $E_r$.

Radial shape adaptation of the robotic device as a functional requirement may demand a forward and backward retrievable surface locomotion capability in the lumen with a diameter $\Phi_l$ of a blood vessel. In a M4 region, the lumen diameter may, e.g., change from 1.5 mm to 1 mm. This $\Phi_l$ range may correspond to an average flow speed $v_f$ estimated to be, e.g., from 11.3 cm/s to 25.5 cm/s in a single route without branches. This requirement may, e.g., need to be fulfilled in two dynamic conditions, i.e., the magnet may be actuated to move the robotic device 100 along with and against the flow, for entering the smaller $\Phi_l$ and moving back to the larger $\Phi_l$, respectively.

To experimentally explore which design could fulfill such requirement, a class of prototypes of robotic devices with various Young's moduli of $E_r$ ranging from 6.4 MPa to 13.6 MPa in phantom A made of poly(dimethylsiloxane) (PDMS) elastomer has been tested. The magnet was rotated with a frequency of $f_{mag}$ = 0.5 Hz and led the robotic device 100 by half the size of the magnet. For radial shape adaptation to narrower $\Phi_l$, the magnetic forces and torques may need to be increased by decreasing the distance between the magnet and the robotic device along the $z_r$-axis, $l_{mag}$. Meanwhile, $l_{mag} \geq$ 50 mm may need to be assured for satisfying $l_s$. Thus, a maximum allowed $l_{mag}$ for adapting to various $\Phi_l$, $l_{mag\_max}$, may dictate the capability of radial shape adaptation. The design with larger $l_{mag\_max}$ may be easier to achieve the shape-adaptive locomotion. Particularly, the design with $E_r$ = 6.4 MPa may, e.g., fulfilled the requirement with all $l_{mag\_max}$ greater than 50 mm.

Fig. 28 shows a further exemplary radial shape adaptation of the robotic device 100. The exemplary effect of $S_p$ for $E_r$ = 6.44 MPa is illustrated, with $S_p$ being defined as the projected area of the robotic device 100 to the $x_r$-$z_r$ plane. Higher $S_p$ in smaller diameter of the lumen $\Phi_i$ tends to induce much increased fluidic drag $F_{drag}$ on the robotic device 100. The radial deformability of the robotic device 100 enables the shrinking of $S_p$ into smaller $\Phi_l$, i.e., the low fluidic drag nature.

Four essential variables, the projection area $S_p$, completeness of helix $\lambda_h$, $\Phi_l$, and Young's modulus $E_r$, may affect the relative relations among the magnetic forces and torques, fluidic drag, and frictional forces, which further dictates $l_{mag\_max}$.

To effectively withstand the pulsatile flow, the projection area $S_p$ of the robotic device 100 may be configured to not increase, when the robotic device 100 enters a smaller $\Phi_l$, since increased $S_p$ may increase the fluidic drag $F_{drag}$ on the robotic device 100 notably. Given the low fluidic drag nature of the hollow cylindrical base structure of the robotic device 100, this requirement may be satisfied without further effort. Actually, $S_p$ may, e.g., decrease from 0.44 mm$^2$ for $\Phi_l$ = 1.5 mm to 0.28 mm$^2$ for $\Phi_l$ = 1. Due to the increased flow speed in smaller $\Phi_l$, $F_{drag}$ may still be increased from 0.1 mN to 0.3 mN. However, this increase may be considered to be minor.

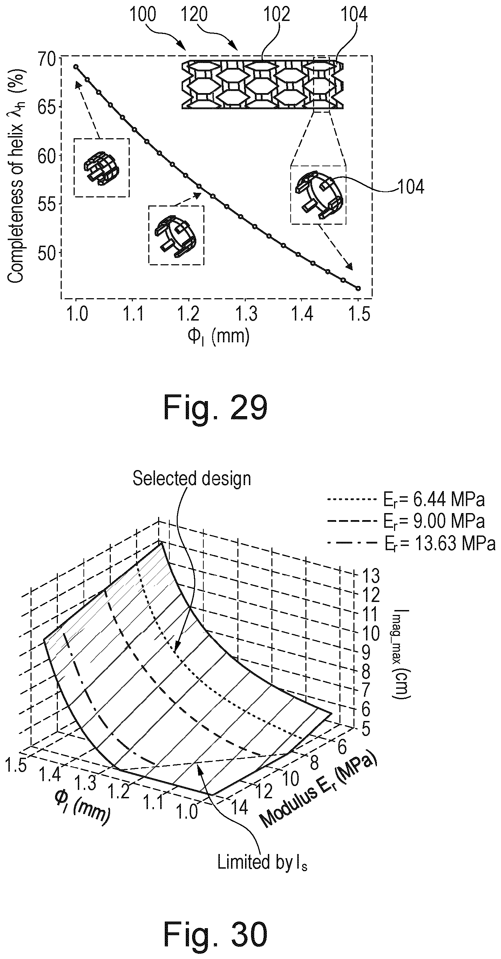

Fig. 29 shows an exemplary diagram of the completeness $\lambda_h$ of an anisotropic surface structure 104 formed on an elastic base structure 102 of a robotic device 100. In the example of Fig. 29, the anisotropic surface structure 104 is provided in form of a helical structure extending over a mesh structure 120 of the elastic base structure 102 with a plurality of cells. The helical structure is interrupted by the voids of the cells of the mesh structure. With decreasing lumen diameter $\Phi_l$ resulting in a decreasing diameter of the robotic device 100, the completeness $\lambda_h$ of the discontinuous helix increases along with radial deformation of the robotic device 100. For one pitch, the completeness may be defined as a ratio between coated helix length, i.e., constant and discontinuous, and the complete helix length, i.e., varying and continuous. The complete helix length is proportional to the lumen diameter $\Phi_l$. For smaller $\Phi_l$, the completeness $\lambda_h$ is increased, and the effect of anisotropic frictional forces for axial locomotion may be enhanced. More precisely, the effect of symmetry breaking from the helical shape and the anisotropic frictions for the axial locomotion may be enhanced. Although both left-handed and right-handed rotations enabled axial propulsion using the actuation method describe herein with the magnetic pulling force resulting in a surface propulsion, the right-handed one improved the robotic device's speed, especially significant when the robotic device moved from smaller $\Phi_l$ with higher $\lambda_h$ to the larger $\Phi_l$ with lower $\lambda_h$.

Fig. 30 shows an exemplary modeling of the maximum distance between the magnet and the robotic device $l_{mag\_max}$. Given the design with low fluidic drag and anisotropic frictional forces, Young's modulus $E_r$ and lumen diameter $\Phi_l$ decide $l_{mag\_max}$. Choosing, e.g., a design with $E_r$ = 6.44 MPa, $l_{mag\_max}$ may be larger than 50 mm for all lumen diameter $\Phi_l$ from 1.5 mm down to 1.0 mm. Either decreasing $\Phi_l$ or increasing $E_r$ increases the necessity of increasing the magnetic force and torques, i.e., the necessity of decreasing $l_{mag\_max}$ for the radial shape-adaptative locomotion.

Particularly, the robotic device with $E_r$ = 6.4 MPa may fulfilled the requirement with all $l_{mag\_max}$ more than 50 mm, satisfying the distance requirement on cortex-to-scalp distance $l_s$, which matched well with the experimental results. Furthermore, modeling based on the ex vivo measurements of the coefficient of friction (CoF) for porcine arteries indicated that $l_{mag\_max}$ could be as large as 10 cm for the robotic device with $E_r$ = 6.4 MPa, suggesting the feasibility of the current system design.

Based on the modeling for arteries, the maximum radial forces $F_n$ and frictional force $F_{fric}$ for $E_r$ = 6.4 MPa, when the robotic device enters the lumen with $\Phi_l$ = 1 mm, are estimated to be around 0.05 N and 0.004 N, respectively in porcine arteries. $F_n$ here may induce a compressive stress of around 6.1 kPa, smaller than a quantified threshold to rupture the endothelial cell at around 12.4 kPa. This design may potentially enable safer interactions with the lumen.

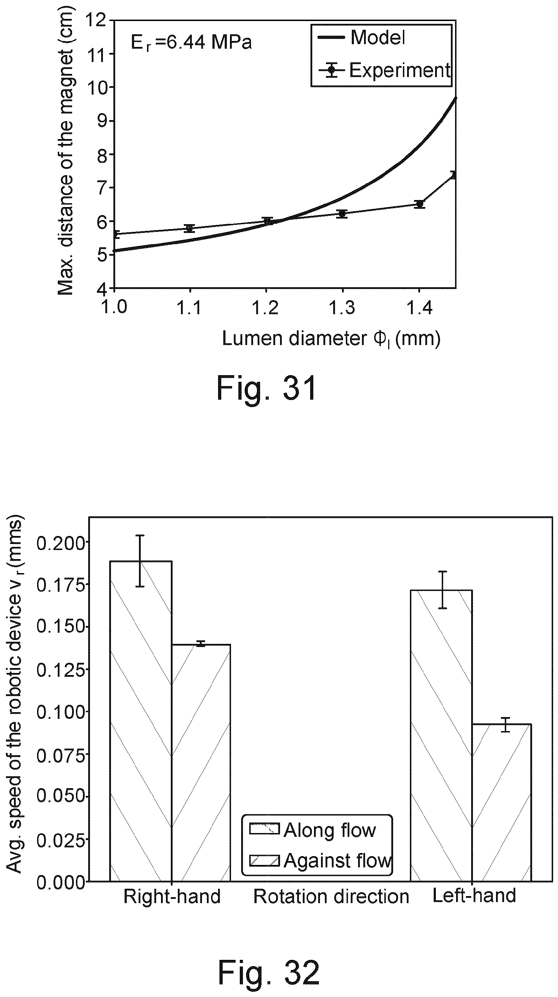

Fig. 31 shows an exemplary comparison of the dependence of the maximum distance between the magnet and the robotic device $l_{mag\_max}$ on the lumen diameter $\Phi_l$ for a Young's modulus $E_r$ = 6.44 MPa. Fig. 31 shows the dependence calculated for a model as well as experimentally measured. Particularly, the robotic device 100 with $E_r$ = 6.4 MPa fulfills the requirement with all $l_{mag\_max}$ larger than 50 mm, satisfying the distance requirement on cortex-to-scalp distance $l_s$ of around 15 mm, which matches well with the experimental results.

Fig. 32 shows a comparison of a left-hand and a right-hand rotation for an anisotropic surface structure in form of a right-handed helix coated onto an elastic base structure in form of a mesh structure. The right-handed helix assists the axial locomotion of the robotic device from a rotation around the $y_r$-axis, i.e., the central longitudinal body axis of the robotic device. Experimentally, left-hand and right-hand rotations both enable axial propulsion using the actuation method described herein. However, a right-hand rotation significantly increases the robotic device's speed compared to a left-handed rotation, especially when the robotic device moves from lumen diameter $\Phi_l$ with higher completeness $\lambda_h$ to larger

$\Phi_l$ with lower $\lambda_h$.

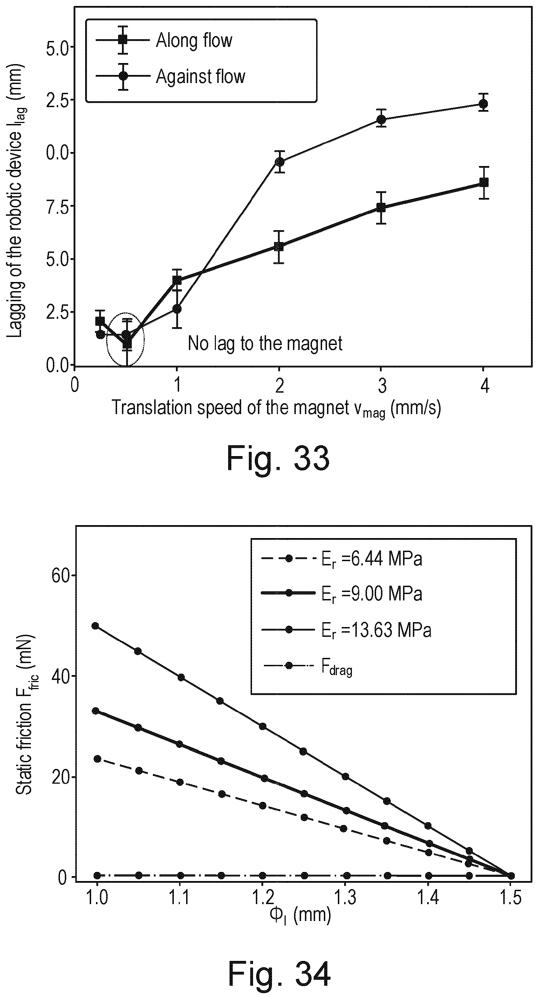

Fig. 33 illustrates the effect of the magnet translation speed $v_{mag}$ on the robotic device displacement in phantom A for $f_{mag}$ = 0.5 Hz and $l_{mag}$ = 55 mm. The lagging distance of the robotic device $l_{lag}$ being moved along the phantom A indicates how well the robotic device follows the magnet. $v_{mag}$ at around 0.5 mm/s is suitable for actuation without lagging. To investigate whether or not the robotic device may achieve the radial shape adaptation, the magnet was, e.g., controlled by a manual operation command from a joystick. To further study, how fast the adaptation could be achieved, $l_{mag}$ = 55 mm was fixed, since this value could enable the radial shape adaptation for all $\Phi_l$. Then the effect of various translation speeds of the magnet $v_{mag}$ and frequencies $f_{mag}$ was investigated. The studies in a phantom B and the corresponding modeling indicated that $f_{mag}$ did not contribute to $v_r$ as much as $v_{mag}$. Therefore, $f_{mag}$ was fixed as 0.5 Hz. The robotic device was, e.g., further tested in the phantom A with different $v_{mag}$ from 0.25 mm/s to 4 mm/s in automatic mode with a pulsatile flow rate was set 12 ml/min. The experimental results are summarized in Fig. 33. When $v_{mag}$ is set to be 0.5 - 1 mm/s, the robotic device followed the magnet translation with no lag, with the average locomotion speed $v_r$ of around 0.18 mm/s along and against the flow on average. Note that due to the variation of the magnetic forces and torques, $v_r$ was not constant.

Fig. 34 illustrates the self-anchoring of the robotic device in a phantom A, i.e., without an external magnetic field. The frictional forces for robots with various $E_r$ are higher than $F_{drag}$, ensuring flow withstanding even when the external magnetic actuation is turned off. Besides the radial adaptation, the robotic device is may further be required to be self-anchoring to withstand pulsatile flow even when the magnetic field is turned off, which may be essential for safe operations. To enable such a property, the frictional forces $F_{fric}$ may have to be larger than the fluidic drag $F_{drag}$. The experiments and modeling results indicated that this requirement may be satisfied for all designs indicated in Fig. 34. The relation may also hold for arteries, given the quantified CoF.

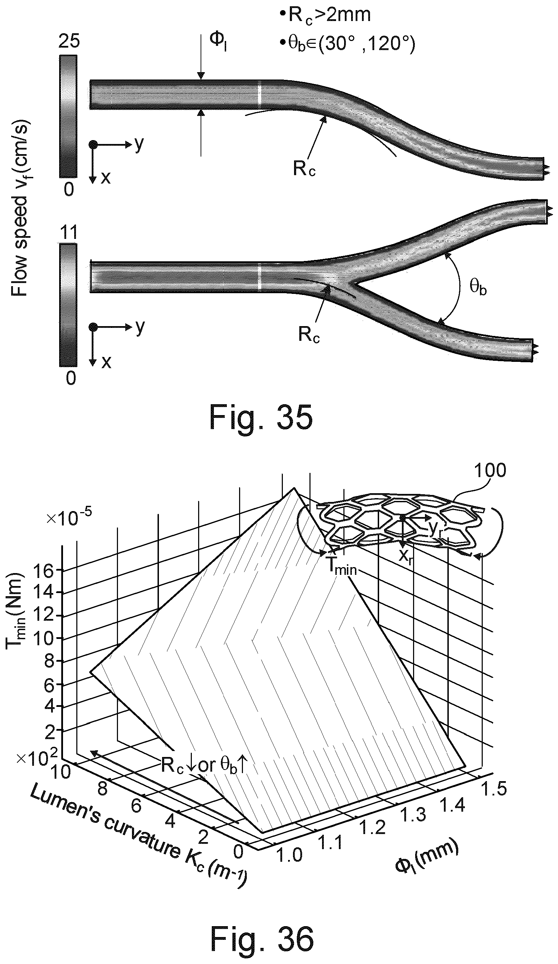

Fig. 35 illustrates physiological requirements on curved lumens with flow speed visualization including curved routes and bifurcations. Exemplary values of radius of curvature $R_c$ > 2mm and bifurcation angles $\theta_b \in (30°, 120°)$ are indicated in the figure.

Fig. 36 illustrates the effect of lumen's curvature $\kappa_c$ and lumen diameter $\Phi_l$ on the minimum required torque $T_{min}$ to bend the robotic device into the curved lumens, e.g., for $E_r$ = 6.44 MPa. For traversing among highly-curved lumens, i.e., the curved routes and bifurcations as illustrated in Fig. 35, the robotic device needs to be bent around the $z_r$-axis. The minimum required torque $T_{min}$ for such bending is correlated to the lumen's curvature $\kappa_c$ and $\Phi_l$, i.e., $T_{min} = E_r \cdot l_r(\Phi_l) \cdot \kappa_c$, where $l_r$ is the second moment of area for the cross-section along the $x_r$-$z_r$ plane. Increasing $\kappa_c$, i.e., for more curved lumens with smaller radii of curvature $R_c$ or larger $\theta_b$, and lumen diameter $\Phi_l$ increases the curved lumens $T_{min}$. Either increasing $\kappa_c$, e.g., by decreasing the radius of curvature $R_c$ or increasing the bifurcation angle $\theta_b$, or increasing $\Phi_l$ may increases $T_{min}$. In the following, it is exemplarily focused on large $\Phi_l$ = 1.45 mm to experimentally find effective curved routes traversing strategies in physiologically relevant $R_c$ (> 2 mm) and $\theta_b$ (30° - 120°) value ranges.

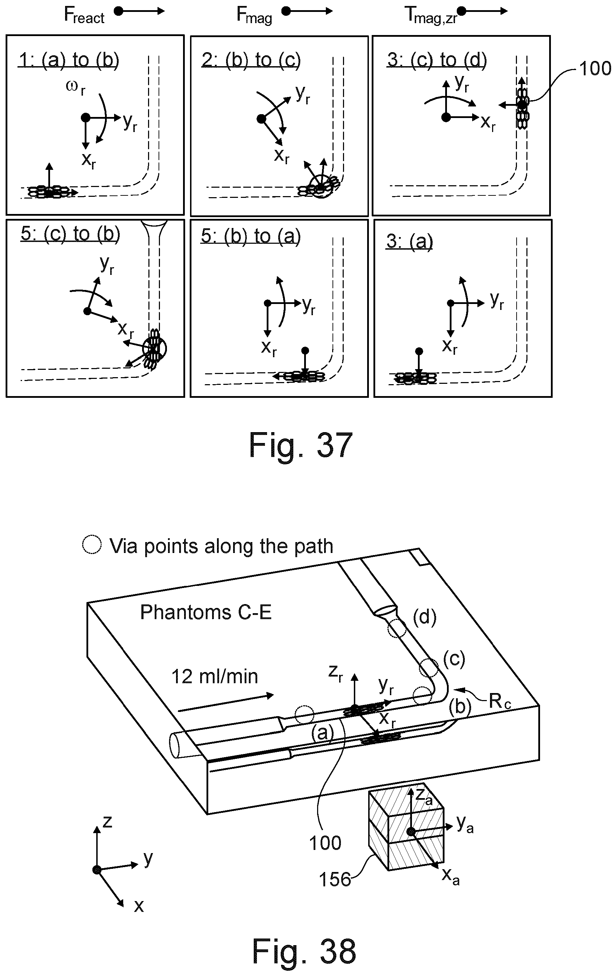

Fig. 37 shows exemplary snapshots for retrievable curved routes traversing a curvature of a blood vessel 100 with a radius of curvature $R_c$ of 5 mm. The bending torques from the magnetic force $F_{mag}$ due to the leading position of the magnet, magnetic torque $T_{mag,z}$ due to the reorientation of the magnet, and the reaction force $F_{react}$ from the lumen wall due to the rotation of the magnet, overcome $T_{min}$, realizing the curved route traversing. In Fig. 37, only the forces and torques enabling the traversing are labeled.

The total torques applied to the robotic device 100 may need to be greater than $T_{min}$ to bend the robotic device 100 into the curved route defined by the blood vessel 200. These contributing torques may include: 1) the torque from the magnetic pulling force, $F_{mag}$, due to the leading position of the magnet, 2) the magnetic toque around the $z_r$-axis, $T_{mag,zr}$, due to the reorientation of the magnet, and 3) the reaction force from the lumen wall of the blood vessel 200 due to the rotation, $F_{react}$, of the robotic device 100.

For illustrating the actuation strategy, the locomotion path is discretized into via-points (a) to (d), as shown in Fig. 37 and 38. When the robotic device enters the curved route and moves along the path defined by the blood vessel 200 to (c) - (d), the magnet is oriented such that the $y_a$-axis of the magnet is parallel to (c) - (d), leading the robotic device 100 and simultaneously rotating the robotic device 100 around the positive $y_r$-axis of the robotic device 100. Thus, the torque from the magnetic pulling force $F_{mag}$ may bend the robotic device towards (c) - (d). Meanwhile, $T_{mag,zr}$ tends to align $x_r$ to $x_a$, which bends the robotic device towards the same direction. Lastly, the rotation tends to roll the robotic device to the positive side of the $x_r$-axis. When the robotic device is in contact with the lumen wall of the blood vessel 200, the reaction force $F_{react}$ bends the robotic device towards (c) - (d). The consequent sum of the bending torques may overcome $T_{min}$ such that the robotic device may enter (c) - (d). This procedure outlined above is indicated in step 2 in Fig. 32.

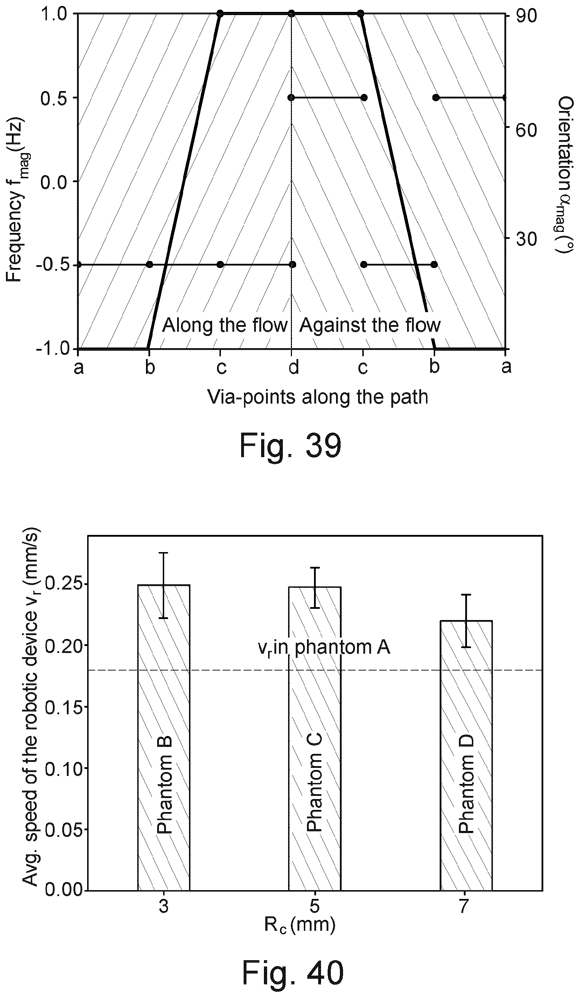

When the robotic device 100 returns to (a) - (b) from (c) - (d), the magnet may be oriented such that the $y_a$-axis is parallel to (a) - (b), leading the robotic device 100 and rotating the robotic device 100 around the positive $y_r$-axis. Thus, the torque from pulling force $F_{mag}$ may bends the robotic device towards (a) - (b). Meanwhile, $T_{mag,zr}$ may tend to align $x_r$ to $x_a$, which may also bend the robotic device 100 towards (a) - (b). Lastly, the rotation may tend to roll the robotic device to the positive side of $x_r$-axis. When the robotic device is in contact with the lumen wall of blood vessel 200, the reaction force $F_{react}$ may point to and bend the robotic device towards (a) - (b). The procedures outlined above are indicated in step 4 in Fig. 37. The complete strategy for the whole path is summarized in Fig. 39 below.

Fig. 38 shows an exemplary illustration of a coordinate system and via-points discretization for a curved route traversing.

Fig. 39 shows exemplary strategies for a curved route traversing along the flow as well as against the flow for the via-points discretization illustrated in Fig. 38. The signs + and - of the frequency of the magnet $F_{mag}$ represent the rotation direction of the magnet around the $y_a$-axis as illustrated in Fig. 38.

Fig. 40 shows exemplary experimental results of robotic device locomotion speed $v_r$ among various radii of curvature $R_c$ in phantoms C, D, and E for $f_{mag} = 0.5$ Hz and $l_{mag} = 55$ mm. The average speed $v_r$ of the robotic device is around 0.2 to 0.25 mm/s, indicating the robustness of the strategies among different curved routes. Thus, the results are consistently around $v_r = 0.18$ mm/s, i.e., the results for the phantoms C to E correspond to the result for the robotic device locomotion speed $v_r$ acquired for phantom A. Thus, traversing among curved routes may not sacrifice the locomotion speed.

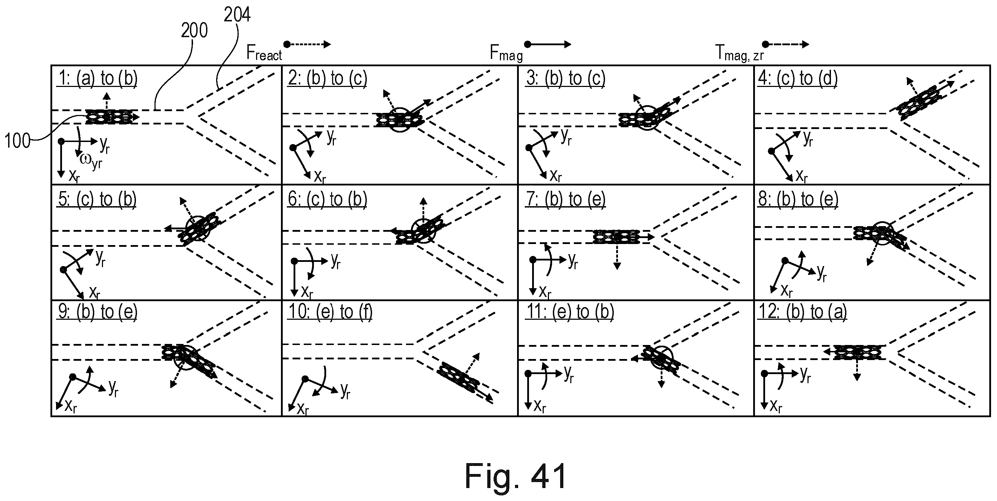

Fig. 41 shows exemplary snapshots for a retrievable branch traversing among a bifurcation with an exemplary branching angle $\theta_b$ of 60°. In Fig. 41, only the leading forces and torques enabling the traversing are indicated on each time step. The composition of the magnetic force-induced torque from $F_{mag}$, magnetic toque $T_{mag,zr}$, and the reaction force-induced torque from $F_{react}$ bends the robotic device and leads it into the desired branch 204.

The strategies for bifurcating branch traversing may be similar to the ones for curved routes as illustrated in Fig. 37 to 39. For a bifurcation angle $\theta_b$ from 30° to 120°, the contributing torques applied on the robotic device 100 may be greater than $T_{min}$. To illustrate the strategies, the path may be discretized into via-points (a) to (f) as shown in Figs. 36 and 37. The procedures for the robotic device 100 entering into the branch (c) - (d) may be indicated in steps 2 and 3 in Fig. 41. The magnet is oriented such that the $y_a$-axis is parallel to the route (c) - (d), leading the robotic device 100 and simultaneously rotating the robotic device 100 around the positive $y_r$-axis. Thus, the torque from pulling force $F_{mag}$ may bend the robotic device 100 towards (c) - (d). Meanwhile, $T_{mag,zr}$ tends to align $x_r$ to $x_a$, which may bend the robotic device 100 towards the same branch. Lastly, the rotation may tend to roll the robotic device 100 to the positive side of the $x_r$-axis. When the robotic device 100 is in contact with the lumen junctions, the reaction force $F_{react}$ may point to the negative $x_r$-axis, bending the robotic device 100 towards (c) - (d). The consequent sum of the bending torques may be greater than $T_{min}$ so that the robotic device 100 is enabled to enter (c) - (d).

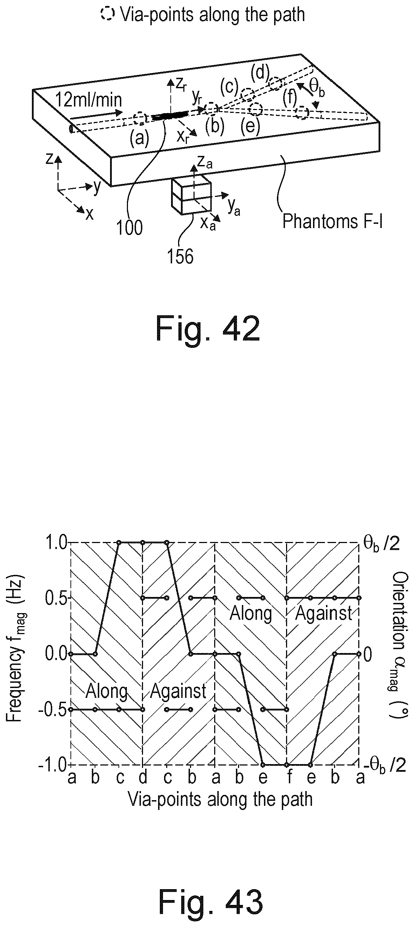

The procedures for robotic device 100 returning to main trunk (a) - (b) from (c) - (d) may be indicated in steps 5 and 6 in Fig. 41. The magnet is oriented such that $y_a$-axis is parallel to the route (a) - (b), leading the robotic device 100 and simultaneously rotating the robotic device around the positive $y_r$-axis. Thus, the torque from pulling force $F_{mag}$ may bend the robotic device towards (a) - (b). Meanwhile, $T_{mag,zr}$ may tend to align $x_r$ to $x_a$, which may also bend the robotic device towards (a) - (b). Lastly, the rotation of the robotic device 100 may tend to roll the robotic device 100 to the positive side of $x_r$-axis. When the robotic device is in contact with the lumen wall of the blood vessel 200, the reaction force $F_{react}$ may point to the negative $x_r$-axis, bending the robotic device towards (a) - (b). Similar strategies may be extrapolated, when the robotic device 100 enters the branch (e) - (f) and returns. The complete strategy for the whole path is summarized in Fig. 43 below.

Fig. 42 shows an exemplary coordinate system and via-points discretization a branch traversing among a bifurcation with an exemplary branching angle $\theta_b$ of 60° as illustrated in Fig. 41.

Fig. 43 shows exemplary strategies for a branch traversing long the flow as well as against the flow for the via-points discretization illustrated in Fig. 42. The signs + and - of the frequency of the magnet $F_{mag}$ represent the rotation direction of the magnet around the $y_a$-axis as illustrated in Fig. 42.

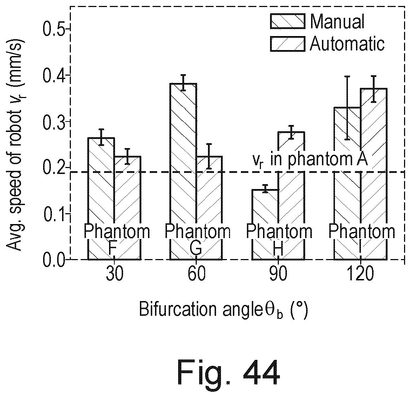

Fig. 44 shows exemplary results of locomotion speed $v_r$ of the robotic device among various bifurcation angles in phantoms F, G, H, and I for a manual control as well as an automatic trajectory with $f_{mag} = 0.5$ Hz and $l_{mag} = 55$ mm. To investigate the consistency of the proposed strategies, the strategies are evaluated in phantoms F-I by manual trajectories, i.e., manipulation by joystick, and automatic ones, i.e., manipulation by via-points from the predefined trajectories. The automatic ones are used to rule out randomness in manual control. The average locomotion speed $v_r$ are 0.15 - 0.35 mm/s, which are relatively consistent among the different phantoms with various $\theta_b$ and different modes, i.e., manual or automatic. Thus, the results are around $v_r = 0.18$ mm/s, i.e., the results for the phantoms F to I correspond to the result for the robotic device locomotion speed $v_r$ acquired for phantom A. Thus, traversing among bifurcations may not sacrifice the locomotion speed. However, the values of $v_r$ may not exactly the same due to the fabrication differences on various robotic devices 100 and phantoms. As shown above, the curved lumen traversing is enabled by the magnetic forces, magnetic torques, and interaction with the lumen walls of the blood vessel. Given the same magnetic actuation configuration and similar elastic properties of phantoms and arteries, the strategies may be expected to be transferred to real artery cases.

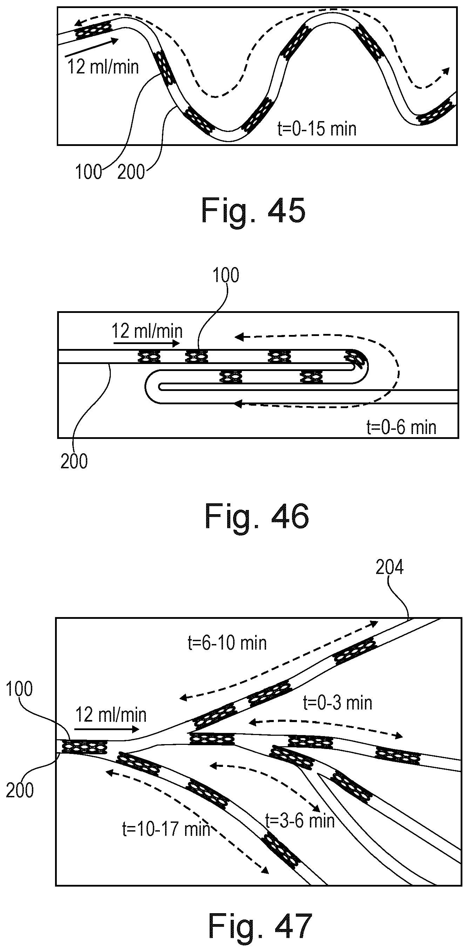

Fig. 45 shows an exemplary locomotion along a tortuous route provided by phantom J of a blood vessel 200. The robot's locomotion capability is demonstrated in the phantoms with combined physiological features. The radius of curvature $R_c$ of phantom J ranges from 2.5 mm to 5 mm. The lumen diameter $\Phi_l$ is, e.g., set to be 1.45 mm The pulsatile flow rate into the inlet of phantom J is 12 ml/min. Note that the robotic arm configuration may enable the magnet's orientation up to ± 90° around the global z-axis. For a rather tortuous route, reorientation of the phantoms may be performed to assist the robotic device's locomotion, if the robotic arm manipulation comes to a singular configuration.

Fig. 46 shows an exemplary locomotion of a robotic device 100 in a strongly curved route provided by phantom K of a blood vessel 200. The angle of corner is 0°, while the radius of curvature $R_c$ is 1 mm. The pulsatile flow rate into the inlet of phantom K is 12 ml/min. Here the robotic device 100 with a mesh structure comprising two cells along the central longitudinal body axis of the robotic device 100 shows flexible navigation in this specific condition.

Fig. 47 shows an exemplary locomotion among branches 204 of phantom L in 2D. The pulsatile flow rate into the inlet of phantom K is 12 ml/min. Phantom L comprises six exemplary branches 204, where $\Phi_l$ is, e.g., designed to be from 1 mm to 1.5 mm. The successful locomotion of the robotic device 100 among these phantoms confirms proper understanding of mechanics and effective system development towards the shape-adaptive locomotion in complex lumens of various $\Phi_l$ with the pulsatile flow.

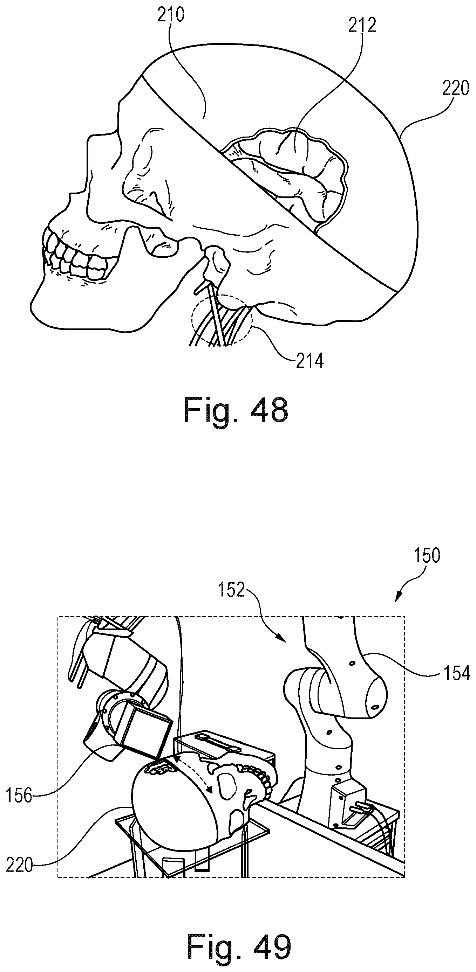

Fig. 48 shows an exemplary locomotion among 3D branches in a cranium simulant 210 provided by phantom M. Phantom M is a 3D version of phantom L, fixed on a cerebral hemisphere and brain stimulant. The successful locomotion of the robotic device 100 among these phantoms confirms proper understanding of mechanics and effective system development towards the shape-adaptive locomotion in complex lumens of various $\Phi_l$ with the pulsatile flow.

Fig. 49 shows an exemplary locomotion among 3D branches in a cranium simulant 210 provided by phantom M. To evaluate the robotic device 100 in more medically relevant settings and demonstrate the scenario of its advantage against catheters, the robotic device 100 is delivered via medical tubing 214 and its locomotion is tested in fresh porcine arteries with $\Phi_l$ from, e.g., 1 mm to 1.5 mm. The pulsatile flow is pumped inside. The blood vessels 200 used are coronary arteries freshly cut from porcine hearts. The blood analog with 12 ml/min is pumped into the arteries during the experiment. The robotic device 100 is first delivered to the target region's vicinity by a catheter, e.g., with inner diameter $d_c = 0.072$ in, i.e., $d_c = 1.8288$ mm, polyurethane medical tubing from Nordson Medical. Then, the robotic device is released to locomote to the region that the catheter could not access. For example, ultrasound imaging may be utilized to visualize the locomotion of the robotic device in the artery, e.g., using B-mode, Vevo 3100, FUJIFILM Visualsonics, Inc. The pulsatile flow may be visualized by a contrast agent added to the blood analog, e.g., Vevo MicroMarker Non-Targeted Contrast Agent, FUJIFILM Visualsonics, Inc. The proof-of-concept demonstration indicates how the robotic device 100 may be compatible with existing endovascular tools and improve their accessibility. Thinner microcatheters may be chosen for practical usage in distal segments, e.g., with a diameter at the tip of $d_c = 0.027$ in, i.e., $d_c = 0.6858$ mm. The robotic devices 100 with different initial diameters may be fabricated and delivered using these microcatheters based on application needs. On the other hand, the robotic devices 100 may need to carry other functional tools when delivering therapeutic agents. Therefore, a robotic device 100 may not be collapsed too much, and the initial diameter may need to fit the diameter $d_c$ of the microcatheter. Based on the size of the microcatheter mentioned above, a compatible robotic device 100 may be fabricated with an outer diameter of, e.g., $d_r = 0.68$ mm and an inner diameter of, e.g., $d_i = 0.58$ mm using the same fabrication technique. The theoretical assurance of retrievably shape-adaptation and self-anchoring may indicate a promising use in the clinics.

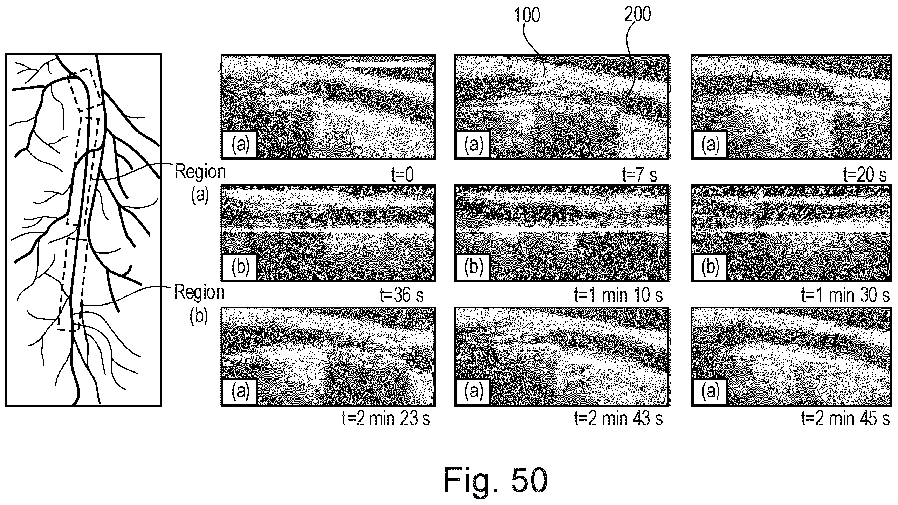

Fig. 50 shows an exemplary delivery of a robotic device 100 to region (a) of a vascular system using a medical catheter. Starting at region (a) locomotion of the robotic device 100 in blood vessels 200, i.e., porcine arteries, is visualized by ultrasound imaging. Robotic device 100 moves into region (b). At t = 1 min 10 s, the magnet was moved away, but the robotic device 100 could withstand the flow safely. After that the robotic device 100 moves back into region (a).

The robotic device may also be tested under X-ray imaging, e.g., used in minimally invasive endovascular surgeries. The robotic device's 100 detection under the X-ray imaging, e.g., XPERT® 80, Cabinet X-ray System, KUBTEC® Scientific, is managed under various conditions, e.g., 1) the robotic device 100 inside a PDMS phantom, 2) the robotic device 100 inside the PDMS covered by cranium-simulant, 3) the robotic device 100 in porcine vessels, and 4) the robotic device 100 in vessels covered by cranium-simulant. A contrast agent, e.g., Iomeron 400, solution for injection, Bracco UK Limited, in PBS may be injected into the lumens, e.g., mass ratio 1:1. Two critical imaging parameters, voltage and current, may be swept to find the best imaging results. The clear identification of the robotic device from the background materials may support its efficacy, e.g., in potential future clinical usage for human inspection-based intervention or robotic surgeries.

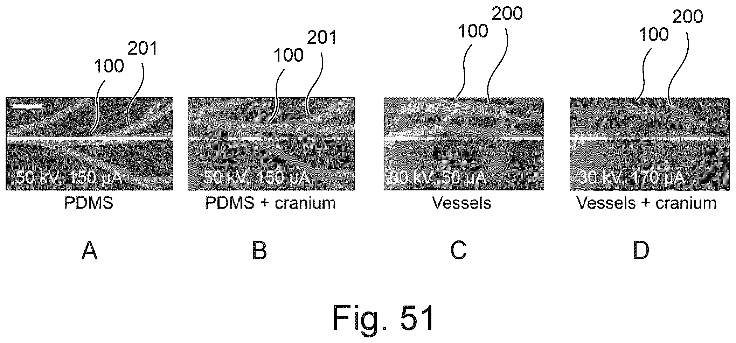

Figs. 51 show exemplary X-ray imaging parameters for detecting the robotic device 100. Fig. 51A shows exemplary X-ray imaging parameters for detecting the robotic device 100 in an PDMS phantom 201 with contrast agent, but without a cranium simulant as cover. Fig. 51B shows exemplary X-ray imaging parameters for detecting the robotic device 100 in an PDMS phantom 201 with contrast agent and with a cranium simulant as cover. Fig. 51C shows exemplary X-ray imaging parameters for detecting the robotic device 100 in a blood vessel 200 with contrast agent, but without a cranium simulant as cover. Fig. 51D shows exemplary X-ray imaging parameters for detecting the robotic device 100 in a blood vessel 200 with contrast agent and with a cranium simulant as cover.

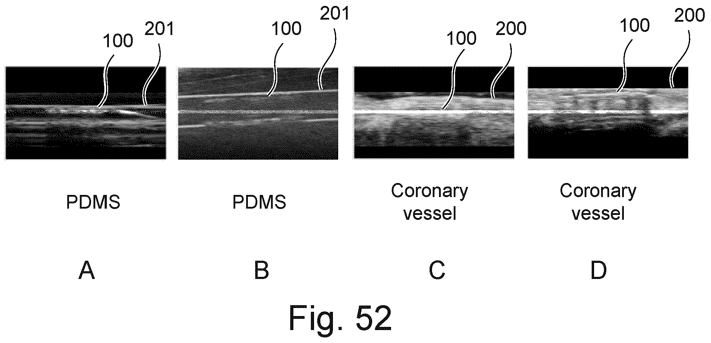

Figs. 52 show exemplary properties of sonographic image acquisition. Fig. 52A shows an exemplary sonographic image acquisition of the robotic device 100 in an PDMS phantom 201. The sonographic image acquisition parameters used are MX 201, a transmit frequency of 15 MHz, an acquisition of 120-199 fps, and a gain of 26-57 dB.

Fig. 52B shows an exemplary sonographic image acquisition of the robotic device 100 in the PDMS phantom 201. The sonographic image acquisition parameters used are MX 550 D, a transmit frequency of 40 MHz, an acquisition of 70-247 fps, and a gain of 33-70 dB.

Fig. 52C shows an exemplary sonographic image acquisition of the robotic device 100 in a blood vessel 200. The sonographic image acquisition parameters used are MX 201, a transmit frequency of 15 MHz, an acquisition of 120-199 fps, and a gain of 26-57 dB.

Fig. 52D shows an exemplary sonographic image acquisition of the robotic device 100 in the blood vessel 200. The sonographic image acquisition parameters used are MX 550 D, a transmit frequency of 40 MHz, an acquisition of 70-247 fps, and a gain of 33-70 dB.

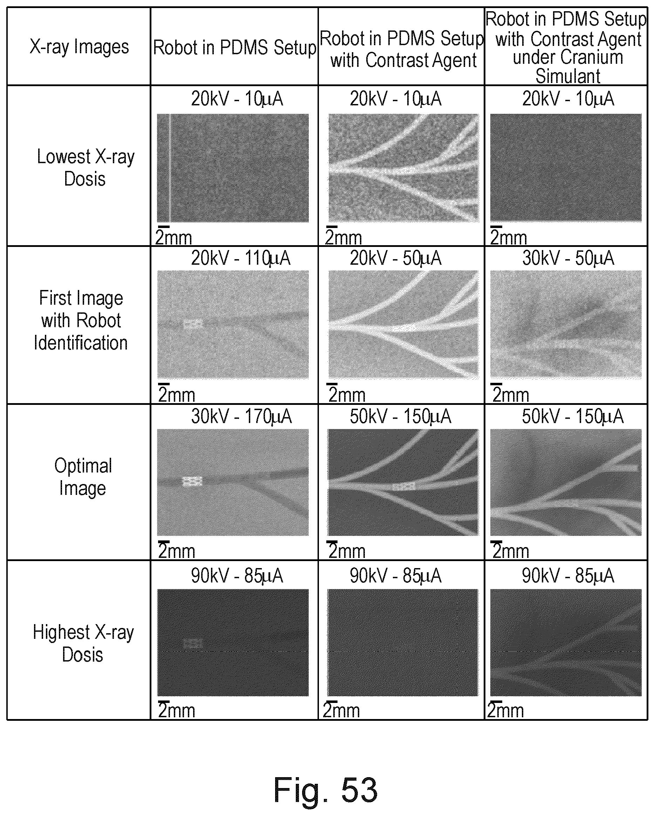

Fig. 53 shows exemplary X-ray imaging tests. The robotic device detection under X-ray imaging (e.g., XPERT® 80, Cabinet X-ray System, KUBTEC® Scientific) is managed under various conditions. The first column of images shows X-ray images of the robotic device inside a PDMS phantom. The second column of images shows X-ray images of the robotic device inside the PDMS with contrast agent. The third column of images shows X-ray images of the robotic device inside the PDMS with contrast agent covered by a cranium-simulant.

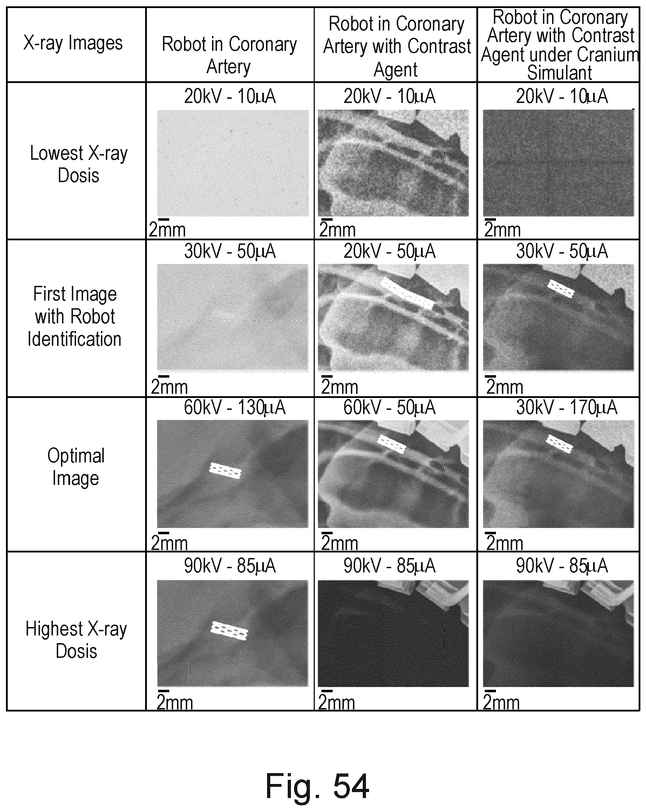

Fig. 54 shows further exemplary X-ray imaging tests (e.g., XPERT® 80, Cabinet X-ray System, KUBTEC® Scientific) of the robotic device detection under various conditions. The first column of images shows X-ray images of the robotic device inside a coronary artery. The second column of images shows X-ray images of the robotic device inside the coronary artery with contrast agent. The third column of images shows X-ray images of the robotic device inside the coronary artery with contrast agent covered by a cranium-simulant.

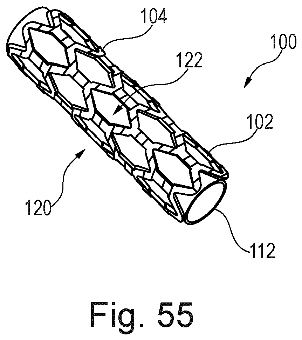

Fig. 55 shows an exemplary robotic device 100. The robotic device 100 of Fig. 55 corresponds to the robotic device 100 of Fig. 8. The only difference may be, that the robotic device 100 of Fig. 55 in addition comprises an inner film, i.e., continuous inner layer 112, arranged on an inner surface of the mesh structure 120. The continuous inner layer 112 may be configured for channeling a blood flow through the robotic device 100. Thus, this robotic device 100 may, e.g., be used as an actively controlled, flexible flow diverter, e.g., for treating an aneurysm and/or an arteriovenous malformation (AVM). The additional continuous inner layer 112 may, e.g., stopped any blood from passing through the mesh structure 120. For example, a porosity of the continuous inner layer 112 is less than 70%, preferably less than 50%, more preferably less than 1%, e.g., equal 0%.

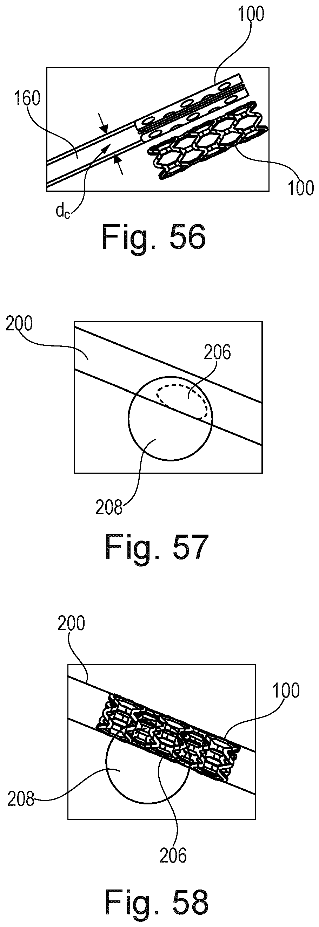

Fig. 56 shows an exemplary robotic device 100 arranged in a microcatheter 160. The microcatheter 160 has an exemplary diameter of $d_c = 0.03$ in, i.e., $d_c = 0.762$ mm. The robotic device 100 may be inserted into the vascular system using the microcatheter 160. The combination of the robotic device 100 with the microcatheter 160 may be used for a local on-demand drug delivery. In order to fit into the microcatheter 160, the robotic device is squeezed together reducing the diameter of the robotic device 100. In addition, the robotic device 100 is shown with its unreduced diameter for purposes of comparison.

For aneurysm and AVM in distal neurovascular routes, current minimally invasive therapies depending on catheters may be limited by the accessibility into the vicinity of the lesioned regions and the flexibility of the delivered therapeutic agents. The robotic device 100 is demonstrated to function as an actively controlled transport vehicle for an on-demand drug delivery. To achieve so, the robotic device is collapse enough to fit into a microcatheter 160 compatible with the distal M3 segment. The diameter $d_c = 0.03$ in, i.e., $d_c = 0.762$ mm.

Fig. 57 shows an exemplary blood vessel 200 with a saccular-shape aneurysm 208 and a neck 206 connecting the blood vessel 200 and the saccular-shape aneurysm 208. Fig. 58 shows the robotic device 100 arranged at the saccular-shape aneurysm 208, in order to prevent blood from flowing into the saccular-shape aneurysm 208 by blocking the neck 206. The porosity mesh structure of the of the robotic device 100 in the example depicted in Fig. 58, is 46% for the neck size of 0.44 mm². The porosity, measured on the patch covering the neck 206 as the ratio between the area of voids and the total area, indicates flow diversion efficacy. When the porosity is less than 70%, the flow condition may be more favorite to initialize the thrombus formation and promote the aneurysm occlusion.

Given, e.g., a Young's module $E_r$ of 6.44 MPa, compared with a Nitinol conventional flow diverter of 28 - 83 GPa, the collapsed robotic device 100 may be flexible enough to be delivered up to the end of the M3 segment. Next, the robotic device 100 may be released and magnetically controlled to the even distally tortuous site to divert the flow. Particularly, for a saccular aneurysm 208 with neck 206 size of, e.g., 0.44 mm², the robotic device may achieve a porosity of 46%, which is promising to trigger the effective occlusion. The porosity, measured on the patch covering the neck 206 as the ratio between the area of voids and the total area, may indicate flow diversion efficacy. Quantified investigations have shown that, when the porosity is less than 70%, the flow condition is more favorite to initialize a thrombus formation and promote an aneurysm occlusion.

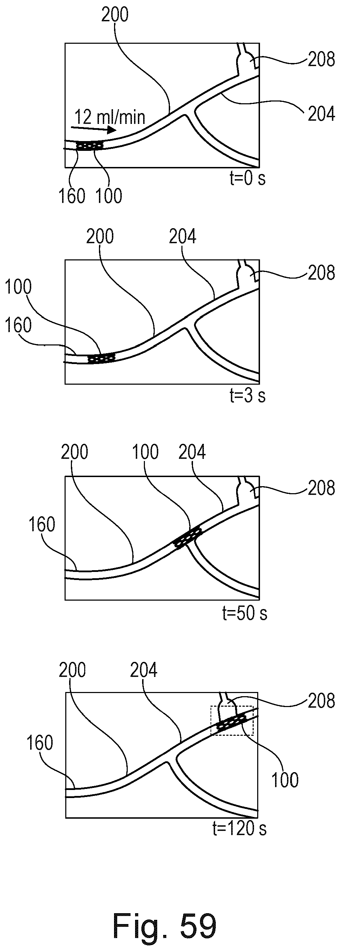

Fig. 59 shows an exemplary flow diversion using the robotic device 100 delivered by a microcatheter 160 and magnetically moved using surface propulsion to a desired lesion site with an aneurysm 208 of a phantom. The aneurysm is located at a branch 204 of the blood vessel 200. The microcatheter 160 has an exemplary diameter of $d_c = 0.03$ in, i.e., $d_c = 0.762$ mm. At t = 0s, the robotic device 100 is delivered using the microcatheter 160. A flow rate of blood through the blood vessel 200 is, e.g., 12 ml/min. The releasing of the robotic device 100 from the microcatheter 160 at t = 3s is shown. The movement of the released robotic device 100 through the blood vessel 200 into the branch 204 towards the desired lesion site with an aneurysm 208 at t = 50s is shown. At t = 120s, the robotic device 100 arrives at the desired lesion site with an aneurysm 208.

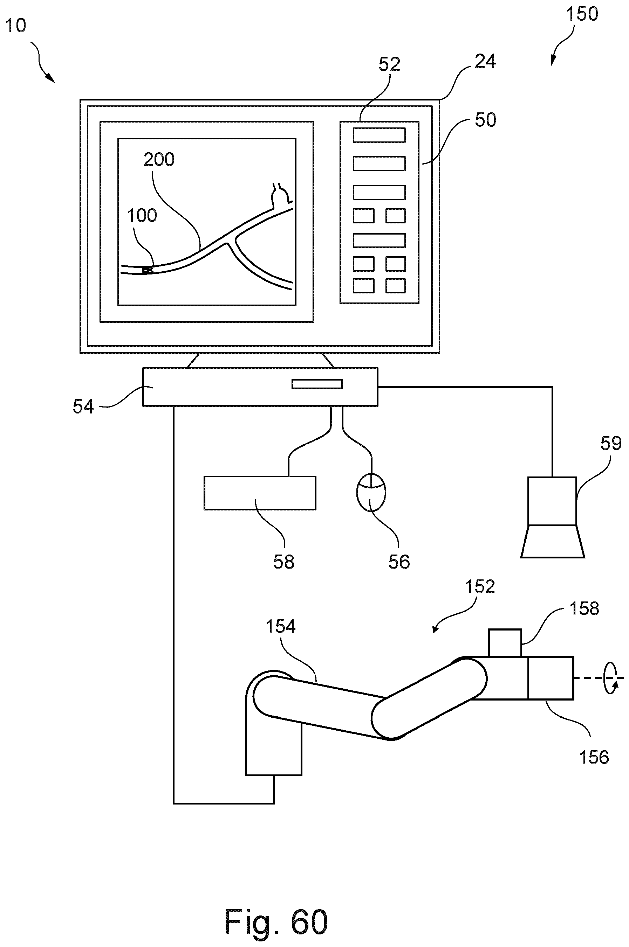

Fig. 60 shows an exemplary actuation and control device 150 configured for actuating and controlling an energy source 158, e.g., a radiofrequency coil, in order to actuate and control a foldable structure of a robotic device 100 inserted into a vascular system. The actuation and control device 150 comprises a computational unit 10. The computational unit 10 may, e.g., comprise a hardware component 54 comprising one or more processors as well as a memory storing machine-executable program instructions. Execution of the program instructions by the one or more processors may cause the one or more processors to control the computational unit 10 to control movements of a moving unit 152. The moving unit 152 comprises a robotic arm 154 with the energy source. The energy source 158 is mounted on a distal end of the robotic arm 154. The foldable structure provides a closed retaining section configured for retaining an agent for medical use, which opens and releases the agent upon reaching a predefined internal energy level, e.g., a shape-memory transition temperature induced by the radiofrequency heating. The computational unit 10 may, e.g., be configured for controlling the energy source 158 and thus the increasing of the internal energy level, e.g., by radiofrequency heating. For example, energy source 158 is mounted on the distal end of the robotic arm 154.

Furthermore, the moving unit 152 may comprise a magnet 156 in order to actuate and control a robotic device inserted into a vascular system. The magnet 156 is mounted on a distal end of the robotic arm 154. The computational unit 10 may further comprise one or more input devices, like a keyboard 58 and a mouse 56, enabling a user to interact with the computational unit 10. Furthermore, the computational unit 10 may comprise one or more output devices, like a display 24 providing a graphical user interface 50 with control elements 52, e.g., GUI elements, enabling the user to control the movement of the magnet 156.

The magnet 156 may, e.g., be a permanent magnet, which is rotated for generating the magnetic field. The magnet 156 may, e.g., be rotated using a step motor. The magnetic field is configured for inducing a rotation of the robotic device around a central longitudinal body axis of the robotic device. The moving unit 152 is configured to be controlled by the computer device for moving the magnet 156 along the blood vessel of the vascular system a long a predefined trajectory extending parallel to the blood vessel in a predefined distance. Thereby a movement of the robotic device is magnetically forced through the blood vessel using a surface propulsion of the robotic device within the blood vessel.

Furthermore, the computational unit 10 may for example comprise a scanner 59, e.g., an X-ray scanner, for determining a position of the robotic device within the vascular system and/or a form of the blood vessel along which the robotic device is to be moved. The form of the blood vessel may be used to determine the trajectory of the magnet.

The hardware components may, e.g., include a 50 mm cubic permanent magnet 156 (N45, IMPLOTEX GmbH), a step motor (NEMA 17) to rotate the magnet 156, and a linked 7-DoF robotic arm 154 (Panda, Franka Emika GmbH). The communication software may be built upon the framework of Robot Operating System (ROS Melodic). There may be three major groups of nodes used in the communication frameworks, i.e., the motion command generator node, the step motor controller node, and the arm controller node. Particularly, the motion command may either be from the manual input or the automatically-generated via-points of the predefined trajectory. During manual manipulation mode, the command published by the joy is subscribed by the arm controller node (arm joy receiver) and the step motor controller mode (step motor joy receiver). During automatic manipulation mode, the predefined via-points are published by the motion command generator node and subscribed by the two controllers (arm command receiver and step motor command receiver). The overall system may enable the 6-DoF spatial manipulation around the end-effector, i.e., the cubic magnet. Note that the rotation direction of the robotic device may be the reverse of the magnet 156 due to the specific magnetic field generated by the permanent magnet 156.

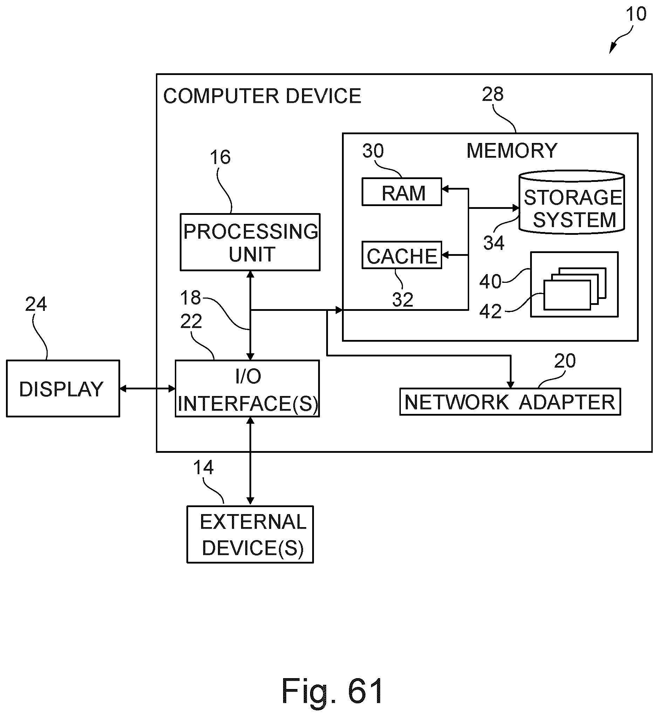

Fig. 61 shows a schematic diagram of an exemplary computational unit 10 for actuating and controlling a radiofrequency coil in order to actuate and control a foldable structure of a robotic device inserted into a vascular system. The computational unit 10 may further be configured for actuating and controlling a magnet configured for moving the robotic device through a blood vessel. The computational unit 10 may be operational with numerous other general-purpose or special-purpose computing system environments or configurations. Computational unit 10 may be described in the general context of computer device executable instructions, such as program modules comprising executable program instructions, being executable by the computational unit 10. Generally, program modules may include routines, programs, objects, components, logic, data structures, and so on that perform particular tasks or implement particular abstract data types. Computational unit 10 may be practiced in distributed computing environments where tasks are performed by remote processing devices that are linked through a communications network. In a distributed computing environment, program modules may be located in both local and remote computer device storage media including memory storage devices.

In Fig. 61, computational unit 10 is shown in the form of a general-purpose computing device. The components of computational unit 10 may include, but are not limited to, one or more processors or processing units 16, a system memory 28, and a bus 18 that couples various system components including system memory 28 to processor 16. Bus 18 represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus.

Computational unit 10 may comprise a variety of computer device readable storage media. Such media may be any available storage media accessible by computational unit 10, and include both volatile and non-volatile storage media, removable and non-removable storage media.

A system memory 28 may include computer device readable storage media in the form of volatile memory, such as random-access memory (RAM) 30 and/or cache memory 32. Computational unit 10 may further include other removable/non-removable, volatile/non-volatile computer device storage media. For example, storage system 34 may be provided for reading from and writing to a non-removable, non-volatile magnetic media also referred to as a hard drive. For example, a magnetic disk drive for reading from and writing to a removable, non-volatile magnetic disk, e.g., a floppy disk, and an optical disk drive for reading from or writing to a removable, non-volatile optical disk such as a CD-ROM, DVD-ROM or other optical storage media may be provided. In such instances, each storage medium may be connected to bus 18 by one or more data media interfaces. Memory 28 may, e.g., include control commands for controlling the radiofrequency coil. Memory 28 may, e.g., further include control commands for controlling movements of the magnet.

Program 40 may have a set of one or more program modules 42 and by way of example be stored in memory 28. The program modules 42 may comprise an operating system, one or more application programs, other program modules, and/or program data. Each of the operating system, one or more application programs, other program modules, and program data or some combination thereof, may include an implementation of a networking environment. One or more of the program modules 42 may be configured for controlling the radiofrequency coil. One or more of the program modules 42 may further be configured for controlling movements of the magnet.

Computational unit 10 may further communicate with one or more external devices 14 such as a keyboard, a pointing device, like a mouse, and a display 24 enabling a user to interact with computational unit 10. Such communication can occur via input/output (I/O) interfaces 22. Computational unit 10 may further communicate with one or more networks such as a local area network (LAN), a general wide area network (WAN), and/or a public network, like the Internet, via network adapter 20. Network adapter 20 may communicate with other components of computational unit 10 via bus 18. It should be understood that although not shown, other hardware and/or software components could be used in conjunction with computational unit 10.

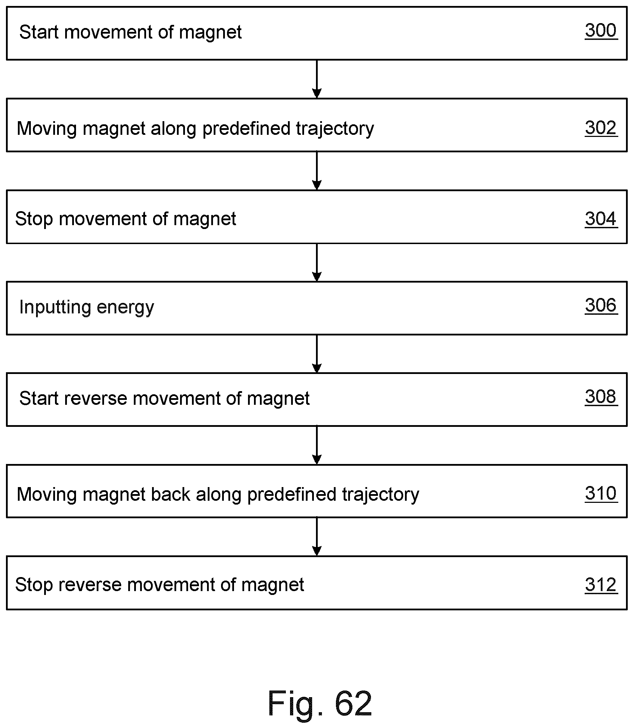

Fig. 62 shows an exemplary method for controlling an actuation and control device, like the actuation and control device shown in Fig. 60. The actuating and controlling device comprises a radiofrequency coil in order to actuate and control a foldable structure of a robotic device inserted into a vascular system. The actuating and controlling device may further comprise a magnet configured for controlling a movement of the robotic device. In block 300, the movement of the magnet is started at a starting position. The magnet generates a magnetic field, which induces a rotation of the robotic device around its central longitudinal body axis resulting in a surface propulsion of the robotic device. For example, the generated magnetic field is a rotating magnetic field. For example, a permanent magnet may be used for generating the rotating magnetic field by rotating the permanent magnet. For example, an electromagnet may be used for generating the magnetic field, e.g., the rotating magnetic field. In block 302, the magnet is moved along a predefined trajectory. The trajectory follows the blood vessel, such that the magnet is moved along the blood vessel magnetically forcing movement of the robotic device through the blood vessel to a predefined destination within the vascular system. For example, the magnet may be moved in front of the robotic device along the trajectory dragging the robotic device. The starting position of the magnet along the blood vessel may correspond to a position, at which the robotic device is released from a catheter, e.g., a microcatheter, within the blood vessel. In block 304, the movement of the magnet is stopped upon reaching a target position at the end of the predefined trajectory. The target position of the magnet at the end of the predefined trajectory may correspond to a position along the blood vessel corresponding to a target position of the robotic device within the blood vessel. In addition, the rotating magnetic field may be switched off. In case of a permanent magnet, e.g., the rotation of the magnet may be stopped. In case of an electromagnet, e.g., a rotation of the electromagnet may be stopped and/or the electromagnet may be switched off.

In block 306, an energy input into a foldable structure is executed, e.g., a radiofrequency heating is executed. For this purpose, an energy source, e.g., a radiofrequency coil is actuated. The radiofrequency coil may induce a heating of the foldable structure, e.g., a foldable shape-memory structure, comprised by the robotic device. The foldable shape-memory structure is heated to a predefined shape-memory transition temperature at which a closed retaining section of the foldable shape-memory structure is opened for releasing an agent for medical use transported by the robotic device. After the energy input, e.g., radiofrequency heating, the magnet may be moved back. In block 308, a reverse movement of the magnet may be started. The magnet generates a magnetic field, which induces am reversed rotation of the robotic device around its central longitudinal body axis resulting in a reversed surface propulsion of the robotic device. For example, the generated magnetic field is a rotating magnetic field rotating in a reversed direction. For example, a permanent magnet may be used for generating the rotating magnetic field by rotating the permanent magnet with a reversed direction of rotation. For example, an electromagnet may be used for generating the magnetic field, e.g., the reversed rotating magnetic field. In block 310, the magnet is moved back along a predefined trajectory. The trajectory follows the blood vessel, such that the magnet is moved back along the blood vessel magnetically forcing movement of the robotic device back through the blood vessel to its starting position within the vascular system. For example, the magnet may be moved in front of the robotic device back along the trajectory dragging the robotic device. In block 312, the movement of the magnet is stopped upon reaching its starting position at the beginning of the predefined trajectory. In addition, the rotating magnetic field may be switched off. In case of a permanent magnet, e.g., the rotation of the magnet may be stopped. In case of an electromagnet, e.g., a rotation of the electromagnet may be stopped and/or the electromagnet may be switched off.

The middle cerebral artery (MCA) originates at the internal carotid artery (ICA) bifurcation. The M4 segment, with a lumen diameter $\Phi_l$ of around 1- 1.5 mm, is regarded as the medium vessel category and the distal segments of MCA. It starts when the vessels exit the Sylvian fissure and spread out over the convex surface of the cerebral hemispheres. The angle for bifurcation $\theta_b$ in this segment varies, and a representative range from 30° to 120° may, e.g., be selected to cover data reported in the literature. The radius of curvature $R_c$ for the vessels here is reported to be larger than 2 mm. Without the loss of generality, the reported data on the average scalp-to-cortex distance may be used to describe the distance between M4 segment and the outer side of the scalp $I_s$, around 15 mm on average. Based on the above geometrical features, two groups of phantoms have been prepared, i.e., the quantitative phantoms for evaluating performances of the robotic device and the ones for demonstrations. The quantitative ones include the tapering-lumen phantom with $\Phi_l$ from 1.5 mm to 0.75 mm (phantom A), uniform-lumen phantom with $\Phi_l$ of 1.45 mm (phantom B), the curved route phantoms with $R_c$ from 3 to 7 mm (phantoms C, D, E with 2 mm as the step, $\Phi_l$ = 1.45 mm), and the bifurcation phantoms with $\theta_b$ ranging from 30° to 120° (phantoms F, G, H, I with 30° as the step, $\Phi_l$ = 1.45 mm). The ones for demonstrations include the 2D and 3D phantoms. The 2D phantoms include a single tortuous route (phantom J), the one with an extremely curved route, i.e., 0° for the corner ($R_c$ = 1 mm, phantom K), the one with six branches (phantom L), and the one with an aneurysm-like structure (phantom N). The 3D one (phantom M) is a spatial version of phantom L, which is bent and fixed to comply with the surface of the cerebral hemisphere model, i.e., a human skull model with the brain.

All phantoms are made by PDMS elastomer (Sylgard™ 184, Dow Inc.). PDMS is chosen for its mechanical stability and wide acceptance in biomedical applications. Injection molding technique was used to fabricate the desired lumen geometries. The negative molds (two parts) for the desired shape of the lumens were firstly 3D-printed (Clear V4 resin, FormLab Form 3). Commercial cast wax (Salmue) was then melted at 120 °C and injected into the molds by syringe. The solidified wax with the desired lumen shapes was taken out. Next, PDMS with a mass ratio 10:1 was poured into and cured at room temperature, i.e., 23°C, for 48 hours. The phantoms were then placed in the hot oven at 120°C, and the molding wax was melted away. Finally, the cured PDMS phantoms with the hollow lumen structures were placed in the ultrasound cleaner and cleaned with ethanol for 5 hours, which removed the wax residuals. Young's modulus of PDMS phantoms is around 2.6 MPa and achieves similar values as arteries at around 1 - 3 MPa. However, the friction properties, e.g., the coefficients of friction (CoF), may not generally match. Therefore, a customized model was developed to show the robotic device may realize desired locomotion functions in arteries in each relevant section. The successful locomotion demonstration in the porcine artery, as shown in Fig. 54, also justifies the practical usage of the design of the robotic device.

The blood analog for all the quantitative analyses in PDMS phantoms is the glycerol/deionized water mixture, and the volume ratio is 44 to 65. The mixture has a dynamic viscosity of 4.4 cP (1cP = 1mPa·s) at room temperature of 23°C, matching human blood for normal control subjects and moderate normal artery shear rates at 37°C, i.e., 4.4 $\pm$ 0.5 cP. For all ex-vivo demonstrations in organs, PBS (Gibco™, Thermo Fisher Scientific) was pumped to the porcine coronary arteries to clean the route first, and then the blood analog was used for the tests.

Concerning the flow rate in brain arteries, the total cerebral blood flow (717 $\pm$ 123 ml/min) distributed to one side of MCA is 21%, with 6% supplied to the distal MCA. This calculates around 4.5 - 7 ml/min of flow rate going into each part of the M4 segment. We currently set the flow rate of the M4 segment to be around 10 - 12 ml/min for quantitative analysis and modeling. The average resting heart rate for adults ranges from 60 to 100 bpm, and the stroke rate was unified to 80 bpm. A commercial pulsatile blood pump was used to pump 10 - 12 ml/min blood analog with 80 bpm in all the experimental conditions with the flow.

The coefficient of friction (CoF) between the robotic device and the porcine arteries was settled using the fresh porcine aorta and the friction tests by a mechanical tester (Instron 5942). The aorta samples are cut and immersed into the PBS in the customized holders. The pulling direction for the measurement is parallel and perpendicular to the outer helical structure of the robotic device, respectively. The tests gave the average maximum static CoF along the helix $\mu_{sa,||}$ = 0.12, the average maximum static CoF perpendicular to the helix $\mu_{sa,\perp}$ = 0.18, the average kinetic CoF along the helix $\mu_{ka,||}$ = 0.07, the average kinetic CoF perpendicular to the helix $\mu_{ka,\perp}$= 0.08.

The same tests were managed between the helical structure and the phantom material PDMS. PDMS is known for its hydrophobicity, increasing the friction between the robotic device and the phantom lumen wall of the blood vessel and hindering locomotion. Therefore, plasma treatment was used to render the PDMS surface hydrophilic before the experiments. The PDMS phantoms were put into the plasma cleaner and treated with the power of 75 W for 3 mins by air (Tergeo, PIE Scientific LLC). The sample was then immediately immersed in the blood analog for experiments and tests. Due to the phenomenon of stick-slip friction of PDMS, the maximum static CoFs for the samples were quantified and used for modeling and analyses, where the average maximum static CoF along the helix $\mu_{sp,||}$ = 0.38, the average maximum static CoF perpendicular to the helix $\mu_{sp,\perp}$ = 0.47.

The coronary arteries for locomotion experiments and aorta for friction tests were cut from the fresh porcine hearts slaughtered within 48 hours and stored under 4 °C. The cut tissues were firstly cleaned by PBS and then prepared for the tests.

There were two sources of thrombi. The first source was directly sampled from the fresh porcine aorta and cut into desired sizes for tests. The second one was manually fabricated from blood, obtained from freshly slaughtered porcine within 48 hours, and stored under 4 °C. The liquid with the volume ratio of 50 to 1 for blood to calcium chloride aqueous solution (0.5 mol/L) was prepared. After 15 mins at room temperature, i.e., 23 °C, the thrombi were formulated. For both methods, the thrombi with volumes of around 3.5 $mm^3$ were prepared and immersed in PBS for thrombolysis tests.

All quantitative values from the experiments presented herein are presented as means plus/minus the standard deviation. Each individual test was managed on more than two robotic device samples. The two-sample t-test was used for the statistical analysis. The statistical significance was set at 95% confidence level ($P < 0.05$).

While the invention has been illustrated and described in detail in the drawings and foregoing description, such illustration and description are to be considered illustrative or exemplary and not restrictive; the invention is not limited to the disclosed embodiments but by the appended claims.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

As will be appreciated by one skilled in the art, aspects of the present invention may be embodied as an apparatus, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

Any combination of one or more computer readable medium(s) may be utilized. The computer readable medium may be a computer readable signal medium or a computer readable storage medium. A "computer-readable storage medium" as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. A further example of an optical disk may be a Blu-ray disk. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

"Computer memory" or "memory" is an example of a computer-readable storage medium. Computer memory is any memory which is directly accessible to a processor. "Computer storage" or "storage" is a further example of a computer-readable storage medium. Computer storage is any non-volatile computer-readable storage medium. In some embodiments, computer storage may also be computer memory or vice versa.

A "processor" as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer device or distributed amongst multiple computer devices. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Generally, the program instructions can be executed on one processor or on several processors. In the case of multiple processors, they can be distributed over several different entities like clients, servers etc. Each processor could execute a portion of the instructions intended for that entity. Thus, when referring to a system or process involving multiple entities, the computer program or program instructions are understood to be adapted to be executed by a processor associated or related to the respective entity.

A "user interface" as used herein is an interface which allows a user or operator to interact with a computer or computer device. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, one or more switches, one or more buttons, and accelerometer are all examples of user interface components which enable the receiving of information or data from an operator.

A GUI element is a data object some of which's attributes specify the shape, layout and/or behavior of an area displayed on a graphical user interface, e.g., a screen. A GUI element can be a standard GUI element such as a button, a text box, a tab, an icon, a text field, a pane, a check-box item or item group or the like. A GUI element can likewise be an image, an alphanumeric character or any combination thereof. At least some of the properties of the displayed GUI elements depend on the data value aggregated on the group of data object said GUI element represents.

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special-purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer-implemented process such that the instructions which execute on the computer or other programmable apparatus provide processes for implementing the functions/acts specified in the flowchart and/or block diagram block or blocks.

List of reference numerals

| 10 | computer unit |
|---|---|
| 14 | external device |
| 16 | processing unit |
| 18 | bus |
| 20 | network adapter |
| 22 | I/O interface |
| 24 | display |
| 28 | memory |
| 30 | RAM |
| 32 | cache |
| 34 | storage system |
| 40 | program |
| 42 | program module |
| 50 | user interface |
| 52 | control elements |
| 54 | hardware device |
| 56 | keyboard |
| 58 | mouse |
| 59 | scanner |
| 100 | robotic device |
| 101 | 3D digital device model |
| 102 | hollow cylindric elastic base structure |
| 104 | anisotropic surface structure |
| 106 | central longitudinal body axis |
| 108 | magnetic material |
| 110 | hemocompatible surface coating |
| 112 | continuous inner layer |
| 120 | mesh structure |
| 122 | cell |
| 130 | foldable structures |
| 132 | retaining section |
| 134 | reception |
| 135 | folding axis |
| 136 | agent |
| 150 | actuation and control device |
| 152 | moving unit |
| 154 | robotic arm |
| 156 | magnet |
| 158 | energy source |
| 160 | microcatheter |
| 200 | blood vessel |
| 202 | vascular system |
| 204 | branch |
| 206 | neck |
| 208 | saccular-shape aneurysm |
| 210 | cranium simulant |
| 212 | M4 section |
| 214 | inlet/outlet |
| 220 | phantom |
| t | time |
| $d_r$ | diameter of robotic device |
| $l_r$ | length of robotic device |

| $x_r$ | x-coordinate of robotic device coordinate frame |
|---|---|
| $y_r$ | y-coordinate of robotic device coordinate frame |
| $z_r$ | z-coordinate of robotic device coordinate frame |
| $m_r$ | moment of robotic device |
| $f$ | axial amplitude of segment |
| $h$ | strut spacing |
| $\rho$ | radius of curvature at the crown junction |
| $\phi$ | helix angle |
| $x_a$ | x-coordinate of magnet coordinate frame |
| $y_a$ | y-coordinate of magnet coordinate frame |
| $z_a$ | z-coordinate of magnet coordinate frame $p_a^r$ vector pointing from magnet to robotic device |
| $f_{mag}$ | frequency |
| $\alpha_{mag}$ | angle |
| $I_{mag}$ | distance between magnet and robotic device |
| $v_{mag}$ | velocity of magnet |
| $F_{drag}$ | dragging force |
| $F_{mag}$ | magnetic force |
| $F_{mag,yr}$ | magnetic force in direction $y_r$ |
| $F_{mag,zr}$ | magnetic force in direction $z_r$ |
| $F_n$ | radial force |
| $G$ | gravitational force |
| $\omega_r$ | angular frequency of robotic device |
| $v_f$ | velocity of blood flow |
| $v_r$ | velocity of robotic device |
| $\omega_{mag}$ | angular frequency of magnet |
| $F_{fric,\parallel}$ | frictional force parallel to helix |
| $F_{fric,\perp}$ | frictional force perpendicular to helix |
| $T_{mag,yr}$ | magnet torque in direction $y_r$ |
| $R_c$ | radius of curvature |
| $\theta_b$ | branching angle |
| $F_{react}$ | reaction force |
| $S_p$ | projection area |
| $E_r$ | Young's modulus |
| $\Phi_l$ | lumen diameter of blood vessel |
| $\lambda_h$ | completeness of helix |
| $I_{lag}$ | lagging of robotic device |
| $K_c$ | curvature of lumen |
| $T_{min}$ | minimum required torque to bend robotic device |
| $T$ | temperature |
| $d_c$ | diameter of catheter |

**Claims**

1.  A robotic device (100) for insertion into a blood vessel (200) of a vascular system (202) with a base structure (102) and a foldable structure (130), the foldable structure (130) comprising a shape of a container, the foldable structure (130) comprising a closed retaining section (132) configured for retaining an agent (136) for medical use, the foldable structure being made from one of the following materials: a photo-responsive material, an electro-responsive material, a magneto-responsive material, an ultrasound-responsive material,

    the foldable structure (130) further being configured for opening the closed retaining section (132) and releasing the agent (136) upon reaching a predefined internal energy level,
    the base structure (102) being a hollow cylindrical elastic base structure (102),
    the elastic base structure (102) being configured for providing a radial elastic force ($F_n$) configured for establishing a contact between an outer anisotropic surface structure (104) of the robotic device (100) and an inner surface of the blood vessel (200) for the diameters of the blood vessel (200),
    the anisotropic surface structure (104) comprising a geometrical anisotropy between a direction circumferential to the robotic device (100) and a direction longitudinal to the robotic device (100), the anisotropic surface structure (104) being configured for establishing by the contact an anisotropic friction between the anisotropic surface structure (104) and the inner surface of the blood vessel (200), the anisotropic friction resulting in a surface propulsion of the robotic device (100) when being rotated around the central longitudinal axis (106) of the robotic device (100),

the robotic device (100) further comprising a magnetic material (108) distributed circumferentially around the robotic device (100) and configured for establishing within an external magnetic field a rotation of the robotic device (100) around the central longitudinal body axis (106) of the robotic device (100).

2. The robotic device (100) of claim 1, the foldable structure (130) being made from a shape-memory material configured for opening the closed retaining section (132) and releasing the agent (136) upon reaching a predefined shape-memory transition temperature.

3. The robotic device (100) of any of the preceding claims, the foldable structure (130) further comprising metal particles for enhancing an efficiency of an external energy input into the foldable structure, for example an external radiofrequency induced heating of the foldable structure (130).

4. The robotic device (100) of any of the preceding claims, the foldable structure (130) having a size in the submillimeter range.

5. The robotic device (100) of any of the preceding claims, the anisotropic surface structure (104) comprising one or more helical structures extending in the longitudinal direction around the elastic base structure (102).

6. The robotic device (100) of any of the preceding claims, the foldable structure (130) being arranged on an inner surface of base structure (102), the foldable structure (130) being integrated into the base structure (102), or the foldable structure (130) being arranged on an external surface of the base structure (102).

7. The robotic device (100) of any of the preceding claims, the robotic device (100) being configured for passively adapting a diameter ($d_r$) of the robotic device (100) to altering diameters ($\Phi_l$) of the blood vessel (200) for diameters of the blood vessel (200) altering within a predefined range of diameters.

8. A system comprising the robotic device (100) of any of the preceding claims and an actuation and control device (150) configured for actuating and controlling the foldable structure (130) of the robotic device (100),

the actuation and control device (150) comprising an energy source (158) configured for increasing the internal energy level of the foldable structure (130) of the robotic device (100) to a predefined internal energy level for an opening of a closed retaining section (132) of the foldable structure (130) upon reaching the predefined internal energy level, the energy source (158) being one of the following sources: photo-, electro-, magnetic- and/or ultrasound-source.

9. The system of claim 8, the energy source (158) being a radiofrequency coil configured for radiofrequency induced heating of the foldable structure (130) made from a shape-memory material to a predefined shape-memory transition temperature for an opening of a closed retaining section (132) of the foldable structure (130) upon reaching the predefined shape-memory transition temperature.

10. The system of any of claims 8 to 9, further comprising a moving unit (152) configured for moving a magnet (156) configured for generating a magnetic field,

the magnetic field being configured for inducing a rotation of the robotic device (100) inserted in a blood vessel (200) of a vascular system (202) around a central longitudinal body axis (106) of the robotic device (100),

the moving unit (152) being configured for moving the magnet (156) along the blood vessel (200) magnetically forcing movement of the robotic device (100) through the blood vessel (200).

**Patentansprüche**

1. Robotische Vorrichtung (100) zum Einbringen in ein Blutgefäß (200) eines vaskulären Systems (202) mit einer Basisstruktur (102) und einer faltbaren Struktur (130), wobei die faltbare Struktur (130) eine Form eines Behälters umfasst, wobei die faltbare Struktur (130) einen geschlossenen Rückhalteabschnitt (132) umfasst, der zum Zurückhalten eines Wirkstoffs (136) zur medizinischen Verwendung eingerichtet ist, wobei die faltbare Struktur aus einem der folgenden Materialien hergestellt ist: einem photoresponsiven Material, einem elektroresponsiven Material, einem magnetoresponsiven Material, einem ultraschallresponsiven Material,

wobei die faltbare Struktur (130) ferner dazu eingerichtet ist, bei Erreichen eines vordefinierten internen Energieniveaus den geschlossenen Rückhalteabschnitt (132) zu öffnen und den Wirkstoff (136) freizusetzen, wobei die Basisstruktur (102) eine hohlzylindrische elastische Basisstruktur (102) ist, wobei die elastische Basisstruktur (102) dazu eingerichtet ist, eine radiale elastische Kraft ($F_n$) bereitzustellen, die dazu eingerichtet ist, für die Durchmesser des Blutgefäßes (200) einen Kontakt zwischen einer äußeren anisotropen Oberflächenstruktur (104) der robotischen Vorrichtung (100) und einer inneren Oberfläche des Blutgefäßes (200) herzustellen, wobei die anisotrope Oberflächenstruktur (104) eine geometrische Anisotropie zwischen einer Umfangsrichtung der robotischen Vorrichtung (100) und einer Längsrichtung der robotischen Vorrichtung (100) umfasst, wobei die anisotrope Oberflächenstruktur (104) dazu eingerichtet ist, durch den Kontakt eine anisotrope Reibung zwischen der anisotropen Oberflächenstruktur (104) und der inneren Oberfläche des Blutgefäßes (200) herzustellen, wobei die anisotrope Reibung zu einem Oberflächenvortrieb der robotischen Vorrichtung (100) führt, wenn diese um die zentrale Längsachse (106) der robotischen Vorrichtung (100) gedreht wird, wobei die robotische Vorrichtung (100) ferner ein magnetisches Material (108) umfasst, das entlang des Umfangs um die robotische Vorrichtung (100) verteilt und dazu eingerichtet ist, innerhalb eines externen Magnetfelds eine Rotation der robotischen Vorrichtung (100) um die zentrale Längskörperachse (106) der robotischen Vorrichtung (100) herzustellen.

2. Robotische Vorrichtung (100) nach Anspruch 1, wobei die faltbare Struktur (130) aus einem Formgedächtnismaterial hergestellt ist, das dazu eingerichtet ist, bei Erreichen einer vordefinierten Formgedächtnis-Übergangstemperatur den geschlossenen Rückhalteabschnitt (132) zu öffnen und den Wirkstoff (136) freizusetzen.

3. Robotische Vorrichtung (100) nach einem der vorhergehenden Ansprüche, wobei die faltbare Struktur (130) ferner Metallpartikel umfasst, um eine Effizienz eines externen Energieeintrags in die faltbare Struktur, beispielsweise einer durch externe Hochfrequenz induzierten Erwärmung der faltbaren Struktur (130), zu erhöhen.

4. Robotische Vorrichtung (100) nach einem der vorhergehenden Ansprüche, wobei die faltbare Struktur (130) eine Größe im Submillimeterbereich aufweist.

5. Robotische Vorrichtung (100) nach einem der vorhergehenden Ansprüche, wobei die anisotrope Oberflächenstruktur (104) eine oder mehrere spiralförmige Strukturen umfasst, die sich in Längsrichtung um die elastische Basisstruktur (102) herum erstrecken.

6. Robotische Vorrichtung (100) nach einem der vorhergehenden Ansprüche, wobei die faltbare Struktur (130) an einer inneren Oberfläche der Basisstruktur (102) angeordnet ist, wobei die faltbare Struktur (130) in die Basisstruktur (102) integriert ist oder wobei die faltbare Struktur (130) an einer äußeren Oberfläche der Basisstruktur (102) angeordnet ist.

7. Robotische Vorrichtung (100) nach einem der vorhergehenden Ansprüche, wobei die robotische Vorrichtung (100) dazu eingerichtet ist, einen Durchmesser ($d_r$) der robotischen Vorrichtung (100) passiv an sich ändernde Durchmesser ($\Phi_l$) des Blutgefäßes (200) anzupassen, für Durchmesser des Blutgefäßes (200), die sich innerhalb eines vordefinierten Durchmesserbereichs ändern.

8. System, umfassend die robotische Vorrichtung (100) nach einem der vorhergehenden Ansprüche und eine Betätigungs- und Steuervorrichtung (150), die dazu eingerichtet ist, die faltbare Struktur (130) der robotischen Vorrichtung (100) zu betätigen und zu steuern, wobei die Betätigungs- und Steuervorrichtung (150) eine Energiequelle (158) umfasst, die dazu eingerichtet ist, das interne Energieniveau der faltbaren Struktur (130) der robotischen Vorrichtung (100) auf ein vordefiniertes internes Energieniveau zu erhöhen, um bei Erreichen des vordefinierten internen Energieniveaus einen geschlossenen Rückhalteabschnitt (132) der faltbaren Struktur (130) zu öffnen, wobei die Energiequelle (158) eine der folgenden Quellen ist: eine Photo-, Elektro-, Magnet- und/oder Ultraschallquelle.

9. System nach Anspruch 8, wobei die Energiequelle (158) eine Hochfrequenzspule ist, die zu einer durch Hochfrequenz induzierten Erwärmung der aus einem Formgedächtnismaterial hergestellten faltbaren Struktur (130) auf eine vordefinierte Formgedächtnis-Übergangstemperatur eingerichtet ist, um bei Erreichen der vordefinierten Formgedächtnis-Übergangstemperatur einen geschlossenen Rückhalteabschnitt (132) der faltbaren Struktur (130) zu öffnen.

**10.** System nach einem der Ansprüche 8 bis 9, ferner umfassend eine Bewegungseinheit (152), die dazu eingerichtet ist, einen Magneten (156) zu bewegen, der dazu eingerichtet ist, ein Magnetfeld zu erzeugen,
wobei das Magnetfeld dazu eingerichtet ist, eine Rotation der in ein Blutgefäß (200) eines vaskulären Systems (202) eingebrachten robotischen Vorrichtung (100) um eine zentrale Längskörperachse (106) der robotischen Vorrichtung (100) zu induzieren,
wobei die Bewegungseinheit (152) dazu eingerichtet ist, den Magneten (156) entlang des Blutgefäßes (200) zu bewegen und dadurch eine Bewegung der robotischen Vorrichtung (100) durch das Blutgefäß (200) magnetisch zu bewirken.

**Revendications**

**1.** Dispositif robotique (100) destiné à être inséré dans un vaisseau sanguin (200) d'un système vasculaire (202) avec une structure de base (102) et une structure pliable (130), la structure pliable (130) comprenant une forme d'un récipient, la structure pliable (130) comprenant une section de retenue fermée (132) configurée pour retenir un agent (136) à usage médical, la structure pliable étant réalisée à partir de l'un des matériaux suivants : un matériau photosensible, un matériau électrosensible, un matériau magnétosensible, un matériau sensible aux ultrasons,

la structure pliable (130) étant en outre configurée pour ouvrir la section de retenue fermée (132) et libérer l'agent (136) lors de l'atteinte d'un niveau d'énergie interne prédéfini,
la structure de base (102) étant une structure de base élastique cylindrique creuse (102),
la structure de base élastique (102) étant configurée pour fournir une force élastique radiale ($F_n$) configurée pour établir un contact entre une structure de surface anisotrope externe (104) du dispositif robotique (100) et une surface interne du vaisseau sanguin (200) pour des diamètres du vaisseau sanguin (200),
la structure de surface anisotrope (104) comprenant une anisotropie géométrique entre une direction circonférentielle par rapport au dispositif robotique (100) et une direction longitudinale par rapport au dispositif robotique (100), la structure de surface anisotrope (104) étant configurée pour établir par le contact un frottement anisotrope entre la structure de surface anisotrope (104) et la surface interne du vaisseau sanguin (200), le frottement anisotrope résultant en une propulsion de surface du dispositif robotique (100) lorsqu'il est mis en rotation autour de l'axe longitudinal central (106) du dispositif robotique (100),
le dispositif robotique (100) comprenant en outre un matériau magnétique (108) réparti circonférentiellement autour du dispositif robotique (100) et configuré pour établir dans un champ magnétique externe une rotation du dispositif robotique (100) autour de l'axe de corps longitudinal central (106) du dispositif robotique (100).

**2.** Dispositif robotique (100) selon la revendication 1, la structure pliable (130) étant réalisée à partir d'un matériau à mémoire de forme configuré pour ouvrir la section de retenue fermée (132) et libérer l'agent (136) lors de l'atteinte d'une température de transition à mémoire de forme prédéfinie.

**3.** Dispositif robotique (100) selon l'une quelconque des revendications précédentes, la structure pliable (130) comprenant en outre des particules métalliques pour améliorer une efficacité d'une entrée d'énergie externe dans la structure pliable, par exemple un chauffage de la structure pliable (130) induit par radiofréquence externe.

**4.** Dispositif robotique (100) selon l'une quelconque des revendications précédentes, la structure pliable (130) ayant une taille dans la plage submillimétrique.

**5.** Dispositif robotique (100) selon l'une quelconque des revendications précédentes, la structure de surface anisotrope (104) comprenant une ou plusieurs structures hélicoïdales s'étendant dans la direction longitudinale autour de la structure de base élastique (102).

**6.** Dispositif robotique (100) selon l'une quelconque des revendications précédentes, la structure pliable (130) étant agencée sur une surface interne de la structure de base (102), la structure pliable (130) étant intégrée dans la structure de base (102), ou la structure pliable (130) étant agencée sur une surface externe de la structure de base (102).

**7.** Dispositif robotique (100) selon l'une quelconque des revendications précédentes, le dispositif robotique (100) étant configuré pour adapter passivement un diamètre ($d_r$) du dispositif robotique (100) à des diamètres variables ($\Phi l$) du vaisseau sanguin (200) pour des diamètres du vaisseau sanguin (200) variant dans une plage prédéfinie de diamètres.

8.  Système comprenant le dispositif robotique (100) selon l'une quelconque des revendications précédentes et un dispositif d'actionnement et de commande (150) configuré pour actionner et commander la structure pliable (130) du dispositif robotique (100),

le dispositif d'actionnement et de commande (150) comprenant une source d'énergie (158) configurée pour augmenter le niveau d'énergie interne de la structure pliable (130) du dispositif robotique (100) à un niveau d'énergie interne prédéfini pour une ouverture d'une section de retenue fermée (132) de la structure pliable (130) lors de l'atteinte du niveau d'énergie interne prédéfini, la source d'énergie (158) étant l'une des sources suivantes : une source lumineuse, une source électrique, une source magnétique et/ou une source ultrasonore.

9.  Système selon la revendication 8, la source d'énergie (158) étant une bobine radiofréquence configurée pour un chauffage de la structure pliable (130), réalisée à partir d'un matériau à mémoire de forme, induit par radiofréquence à une température de transition à mémoire de forme prédéfinie pour une ouverture d'une section de retenue fermée (132) de la structure pliable (130) lors de l'atteinte de la température de transition à mémoire de forme prédéfinie.

10. Système selon l'une quelconque des revendications 8 à 9, comprenant en outre une unité de déplacement (152) configurée pour déplacer un aimant (156) configuré pour générer un champ magnétique,

le champ magnétique étant configuré pour induire une rotation du dispositif robotique (100) inséré dans un vaisseau sanguin (200) d'un système vasculaire (202) autour d'un axe de corps longitudinal central (106) du dispositif robotique (100),

l'unité de déplacement (152) étant configurée pour déplacer l'aimant (156) le long du vaisseau sanguin (200) forçant magnétiquement un mouvement du dispositif robotique (100) à travers le vaisseau sanguin (200).

**Fig. 1**

**Fig. 2**

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

12 ml/min
(11-25 cm/s, 80 bpm, phantom A)

1.5 mm                                    0.8 mm

Along {                                            0 s

                                                  6.9 s

Stop {                            1 mm           57.8 s

                                                  80.5 s

Against {                                        103.7 s

100

## Fig. 27

Von mises stress (MPa)

$3 \times 10^3$                                    2

$S_p$

$S_p$

100

$S_p$

Projection area $S_p$ (mm$^2$)

$\times 10^{-1}$

4.25
4.00
3.75
3.50
3.25
3.00
2.75

1.0   1.1   1.2   1.3   1.4   1.5

Lumen diameter $\Phi_l$ (mm)

## Fig. 28

Fig. 29

Fig. 30

Fig. 31

Fig. 32

Fig. 33

Fig. 34

Fig. 35

Fig. 36

Fig. 37

Fig. 38

Fig. 39

Fig. 40

Fig. 41

⚪ Via-points along the path

Fig. 42

Fig. 43

Fig. 44

12 ml/min

t=0-15 min

100  200

Fig. 45

100

12 ml/min

t=0-6 min

200

Fig. 46

204

t=6-10 min

100

12 ml/min

t=0-3 min

200

t=10-17 min

t=3-6 min

Fig. 47

Fig. 48

Fig. 49

Fig. 50

Fig. 51

Fig. 52

| X-ray Images | Robot in PDMS Setup | Robot in PDMS Setup with Contrast Agent | Robot in PDMS Setup with Contrast Agent under Cranium Simulant |
|---|---|---|---|
| Lowest X-ray Dosis | 20kV - 10µA 2mm | 20kV - 10µA 2mm | 20kV - 10µA 2mm |
| First Image with Robot Identification | 20kV - 110µA 2mm | 20kV - 50µA 2mm | 30kV - 50µA 2mm |
| Optimal Image | 30kV - 170µA 2mm | 50kV - 150µA 2mm | 50kV - 150µA 2mm |
| Highest X-ray Dosis | 90kV - 85µA 2mm | 90kV - 85µA 2mm | 90kV - 85µA 2mm |

Fig. 53

| X-ray Images | Robot in Coronary Artery | Robot in Coronary Artery with Contrast Agent | Robot in Coronary Artery with Contrast Agent under Cranium Simulant |
|---|---|---|---|
| Lowest X-ray Dosis | 20kV - 10μA<br><br>2mm | 20kV - 10μA<br><br>2mm | 20kV - 10μA<br><br>2mm |
| First Image with Robot Identification | 30kV - 50μA<br><br>2mm | 20kV - 50μA<br><br>2mm | 30kV - 50μA<br><br>2mm |
| Optimal Image | 60kV - 130μA<br><br>2mm | 60kV - 50μA<br><br>2mm | 30kV - 170μA<br><br>2mm |
| Highest X-ray Dosis | 90kV - 85μA<br><br>2mm | 90kV - 85μA<br><br>2mm | 90kV - 85μA<br><br>2mm |

Fig. 54

Fig. 55

Fig. 56

Fig. 57

Fig. 58

Fig. 59

Fig. 60

Fig. 61

| Start movement of magnet | 300 |
|---|---|

↓

| Moving magnet along predefined trajectory | 302 |
|---|---|

↓

| Stop movement of magnet | 304 |
|---|---|

↓

| Inputting energy | 306 |
|---|---|

↓

| Start reverse movement of magnet | 308 |
|---|---|

↓

| Moving magnet back along predefined trajectory | 310 |
|---|---|

↓

| Stop reverse movement of magnet | 312 |
|---|---|

Fig. 62

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108553300 A
- US 2020046633 A1
- EP 1591057 A1
- US 2012035440 A

**Non-patent literature cited in the description**

- **AARUSHI BHARGAVA et al.** Ultrasound actuated shape-memory polymer based drug delivery containers. *PROCEEDINGS OF SPIE*, 2018, vol. 10595
- **BRIAN P. TIMKO et al.** Remotely Triggerable Drug Delivery Systems. *ADVANCED MATERIALS*, 2010, vol. 22 (44)